# EUROPEAN PATENT APPLICATION

(11) **EP 1 197 225 A1**
(43) Date of publication of application: **17.04.2002**
(21) Application number: 00942455.7
(22) Date of filing: 03.07.2000
(51) Int. Cl.: A61K 45/00, A61K 39/395, A61P 3/14, A61P 29/00, A61P 37/02

(54) **REMEDIES FOR DISEASES CAUSED BY PTH OR PTHrP**

(30) Priority: 02.07.1999 JP 18979399
(71) Applicant: CHUGAI SEIYAKU KABUSHIKI KAISHA, Tokyo, 115-8543 (JP)
(72) Inventor: OGATA, Etsuro, Setagaya-ku, Tokyo 157-0076 (JP); SATO, Koh, Chugai Seiyaku Kabushiki Kaisha, Chuo-ku, Tokyo 104-8301 (JP); ONUMA, Etsuro, Chugai Seiyaku Kabushiki Kaisha, Gotenba-shi, Shizuoka 412-8513 (JP); TSUNEMARI, Toshiaki, Chugai Seiyaku K. Kaisha, Gotenba-shi, Shizuoka 412-8513 (JP); SAITO, Hidemi, Chugai Seiyaku Kabushiki Kaisha, Gotenba-shi, Shizuoka 412-8513 (JP); AZUMA, Yumiko, Chugai Seiyaku Kabushiki Kaisha, Gotenba-shi, Shizuoka 412-8513 (JP)
(74) Representative: Maschio, Antonio
(86) International application number: JP0004414
(87) International publication number: WO0102011

(57) **Abstract**

The present invention is directed to providing a therapeutic agent for diseases caused by PTH or PTHrP. The present invention provides a therapeutic agent for diseases caused by PTH or PHTrP, which comprises, as an active ingredient, an agonist or antagonist binding to a PTH receptor or PTHrP receptor, or a substance which binds to a ligand of such a receptor to thereby promote or inhibit binding of the ligand to the receptor.

## Description

### TECHNICAL FIELD

The present invention relates to a therapeutic agent for diseases caused by parathyroid hormone (PTH) or parathyroid hormone-related protein (PTHrP).

### BACKGROUND ART

A parathyroid hormone-related protein (PTHrP) is a protein, which was identified in 1987 by investigation of humoral factors causing humoral hypercalcemia of malignancy. It is known that the N-terminus of the protein expresses functions thereof by binding to a receptor (PTH/PTHrP receptor) which is common to parathyroid hormone (PTH).

It has been reported that PTHrP is generated from various tumor tissues, but it is also generated from a wide range of normal tissues such as skin, mammary gland, uterus, placenta, bone, smooth muscle, heart, lung, kidney, liver and brain, and exhibits various functions locally via autocrine/paracrine secretory mechanism.

A PTH/PTHrP receptor strongly expresses in kidney and bone, a target organ of PTH and PTHrP. However, it has been clarified that, apart from these organs, this receptor expresses also in aorta, adrenal, brain, mammary gland, heart, digestive tract, liver, lung, skeletal muscle, ovary, placenta, skin, stomach and uterus etc., and that the receptor shows an extremely similar distribution to PTHrP.

As functions of PTHrP, there are known not only a bone resorption promoting function by activation of osteoclasts in bone and a calcium reabsorption promoting function by acting to distal convoluted tubules of nephros, but also the following (1) to (3):
(1) Involvement in calcium transportation system (e.g. mammary gland epithelium, placenta etc.) in epidelial cells,
(2) A strong smooth muscle relaxing activity (e.g. uterus, urinary tract, blood vessel and digestive tract etc.), and
(3) Involvement in growth, differentiation and development,
but the physiological role of PTHrP in many tissues other than those stated above is still unknown.

For example, PTHrP and a PTH/PTHrP receptor express in the central nervous system (CNS), but there has been hardly any clarification of the functions thereof. When localization of PTHrP mRNA in rat brain was analyzed by in situ hybridization, it was found that the mRNA existed in the hippocampus, the granular cell layer of cerebellum, cerebral cortex and hypothalamus (Weaver et al., Mol Brain Res 28:296-301, 1995; Weir et al., Proc Natl Acad Sci USA 87:108-112, 1990).

Moreover, the distribution of PTH/PTHrP receptor in rat brain matches with the distribution of PTHrP, and so it is assumed that PTHrP acts as a local autocrine/paracrine factor in central nervous system (CNS). An experiment regarding binding of PTH to a cell membrane fraction prepared from each site of rat brain teaches that the order of binding strength is as hypothalamus, cerebellum and cerebral cortex (Harvey et al., Peptides 14:1187-1191, 1993). Furthermore, it has been reported that arginine vasopressin (AVP) is released by PTHrP (1-34) stimulation of rat supraoptic nucleus (SON) slices, and that there is a possibility that PTHrP involves in homeostasis of water or electrolyte in an organism (Yamamoto et al., Endocrinology 139:383-388, 1998, Yamamoto et al., Endocrinology 138:2066-2072, 1997). Thus, although it is clear that PTHrP and a PTH/PTHrP receptor are widely distributed over the brain, physiological role thereof in central nervous system is still unknown.

Recently, induction of various types of cytokines by PTHrP or induction of PTHrP by cytokine has been reported, and a new possibility of involvement of PTHrP in various types of diseases caused by cytokine, as well as its own activity, has been clarified. The following reports which suggest the possibility of crosstalk between PTH or PTHrP and cytokine are known:
1) The values of IL-6 and TNF-α are high in a patient of primary hyperparathyroidism caused by high value of PTH (Grey A. et al., J Clin Endocrinol Metab 81:3450-5, 1996)
2) When osteoblasts are stimulated by PTH or PTHrP in an in vitro system, expression of IL-6 and LIF is promoted (Pollock JH. et al., J Bone Miner Res 11:754-9, 1996)
3) A series of experiments with synovial cells showed that production of IL-6 is accentuated by stimulation with PTHrP, and that TNF-α and IL-1β promote expression of PTHrP, and that therefore PTHrP is a member of pro-inflammatory cytokine cascade (Funk JL. et al., Endocrinology 138:2665-73, 1997; Funk JL. et al., J Clin Invest 101:1362-71, 1998)
4) In cultured human vascular endothelial cells also, TNF-α and IL-1β promote expression of PTHrP (Biochem Biophys ResCommun 249:339-343, 1998)

Thus, there has been reported induction of cytokine by PTHrP or induction of PTHrP by cytokine etc., and a possibility of involvement of PTHrP in various types of diseases caused by cytokines, especially those such as IL-1 β, IL-6 and TNF- α, as well as its own activity, has been clarified.

Humoral hypercalcemia of malignancy and cachexia caused by PTHrP and cachexy bring about clinical properties such as body weight loss, weight loss of fat and muscular tissues, anorexia and anemia, and serious damage is given to quality of life (QOL) of a patient. To improve QOL of a cancer patient, application of treatment methods for improving significantly reduced QOL can be considered in addition to normal treatments for the patient with anticancer agents, but at present, there is no treatment method for improving QOL of a patient at a sufficient level. At the moment, treatment methods having recognized QOL improving effect include forced nutrition and pharmacotherapy. With regard to forced nutrition, since the effect is not long-lasting although body weight is maintained, negative opinions have begun to be voiced in the field of palliative care (Tatsuji Kataoka, Blood/Tumor (Ketsueki/Syuyo-ka), 36, 500-506, 1998). With regard to pharmacotherapy, mainly medroxyprogesterone acetate (MPA) is used, but originally MPA is used to treat emmeniopathy and breast cancer. MPA shows effects such as stimulation of appetite and fat accumulation, but this is an application of the side effects in treatment of the diseases for which MPA was originally adapted for the purpose of improving QOL. Nevertheless, MPA is not a sufficient therapeutic agent in that this compound is a progestational (gestagenic) formulation, and so it shows original hormone actions, and effects thereof are insufficient.

### DISCLOSURE OF THE INVENTION

The object of the present invention is to provide a therapeutic agent for diseases caused by PTH or PTHrP.

The present inventors have performed various studies regarding possibility of alleviating symptoms of diseases (treatment for central nervous system diseases, and treatment for diseases caused by PTHrP-cytokine cascade) caused by PTH or PTHrP other than humoral hypercalcemia of malignancy caused by excessive production of PTHrP. As a result, the inventors have found that an anti-PTHrP antibody is effective in alleviating and treating the above symptoms, thereby completing the present invention.

As shown in Examples described later, it was confirmed that an anti-PTHrP antibody has a high improving effect against symptoms on which no or low improving effects were confirmed using the existing therapeutic agents for hypercalcemia. At the same time, it was also found that the above improvement and treatment effects are not only caused by effects brought by decrease of blood calcium level.

That is to say, the present invention provides a therapeutic agent for a disease caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor. Mainly, the disease may be one caused by PTH or PTHrP other than hypercalcemia.

Moreover, the present invention provides a QOL improving agent alleviating symptom of a disease caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor.

Examples of diseases to which the above therapeutic agent and QOL improving agent are applied may include a syndromes associated with malignancy caused by PTHrP (e.g. digestive system disorders such as diarrhea, vomiturition and nausea), proteometabolism abnormality (e.g. hypoalbuminemia), saccharometabolism abnormality (e.g. reduction of glucose tolerance and reduction of insulin secretion), lipid metabolism abnormality (e.g. hyperlipidemia and reduction of serum lipoprotein lipase activity), anorexia, hematological abnormality (e.g. anemia, thrombosis and DIC syndrome), electrolyte abnormality (e.g. hyponatremia, hypokalemia and hypercalcemia), immunodeficiency (e.g. infection disease), pain, secondary hyperparathyroidism and primary hyperparathyroidism which are caused by PTH, etc.

Furthermore, the present invention provides an improving agent for a central nervous system disease caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor. Examples of central nervous system diseases may include dyssomnia, neuropathy (e.g. schizophrenia, manic-depressive psychosis, neurosis and psychophysiologic disorder), nervous symptom (e.g. vomitation, nausea, mouth dryness, anorexia and vertigo), brain metabolism abnormality, cerebral circulation abnormality, autonomic imbalance, and endocrine system abnormality with which central nervous system is associated, etc.

Still further, the present invention provides an improving agent for a disease caused by PTH or PTHrP-cytokine cascade, which comprises, as an active ingredient, an agonist or antagonist binding to a PTH receptor or PTHrP receptor, or a substance binding to a ligand of the receptor to promote or inhibit binding between the ligand and the receptor. Examples of cytokines may include IL-1, IL-2, IL-3, IL-4, IL-5, IL-6, IL-7, IL-8, IL-9, IL-10, IL-11, IL-12, IL-13, IL-15, G-CSF, GM-CSF, M-CSF, EPO, LIF, TPO, EGF, TGF- α, TGF- β, FGF, IGF, HGF, VEGF, NGF, activin, inhibin, a BMP family, TNF and IFN, etc. Examples of diseases caused by PTH or PTHrP-cytokine cascade may include septicemia, cachexia, inflammation, hemopathy such as hematopoietic system abnormality and leukemia, calcium metabolism abnormality, and autoimmune disease such as rheumatism.

The present invention further provides a central nervous system regulator, which comprises, as an active ingredient, an agonist or antagonist binding to a PTH receptor or PTHrP receptor, or a substance binding to a ligand of the receptor to promote or inhibit binding between the ligand and the receptor.

Moreover, the present invention provides a cytokine network regulator, which comprises, as an active ingredient, an agonist or antagonist binding to a PTH receptor or PTHrP receptor, or a substance binding to a ligand of the receptor to promote or inhibit binding between the ligand and the receptor.

The PTH receptor or PTHrP receptor may be a PTH/PTHrP type I receptor.

The substance binding to a ligand of PTH receptor or PTHrP receptor to inhibit binding between the ligand and the receptor may be selected from the group consisting of an anti-PTHrP antibody and an anti-PTH antibody, and among them, an anti-PTHrP antibody, especially a humanized anti-PTHrP antibody is effective.

The present invention is a therapeutic agent, which comprises, as an active ingredient, an agonist or antagonist binding to a PTH receptor or PTHrP receptor, or a substance binding to a ligand of the receptor to promote or inhibit binding between the ligand and the receptor.

In the present specification, the term "a PTH receptor or PTHrP receptor" is used to mean a receptor binding to PTH or PTHrP, and examples include a PTH/PTHrP type I receptor (described in Japanese Patent Application Laying-Open (kohyo) No. 6-506598).

Examples of "an agonist binding to a PTH receptor or PTHrP receptor" include PTH (1-34), PTH (3-34), PTHrP (1-34), PTHrP (3-34) and an amide form thereof.

The term "an antagonist binding to a PTH receptor or PTHrP receptor" is used to mean a substance which inhibits binding of PTHrP to a PTH receptor or PTHrP receptor by binding to the PTH receptor or PTHrP receptor (e.g. an antagonist against the PTH receptor or PTHrP receptor (which is also referred to as a PTH or PTHrP antagonist), and specific examples include a PTH or PTHrP peptide comprising a substitution or deletion of at least one amino acid, and a partial sequence of a PTH or PTHrP peptide, etc.) Examples of a PTH or PTHrP antagonist include a polypeptide and a low molecule, and specifically examples of substances antagonistically binding to the receptors against PTH or PTHrP include PTH (7-34), PTH (8-34), PTH (9-34), PTH (10-34), PTHrP (7-34), PTHrP (8-34), PTHrP (9-34), PTHrP (10-34), mutants thereof (e.g. [Nle8, 18, D-Trp12, Tyr34] bovine PTH (7-34) NH2, [Nle8, 18, Tyr34] bovine PTH- (7-34)-amide), and an amide form thereof, etc. Examples of substances antagonistically binding to a PTHrP receptor against PTHrP include polypeptides having a PTHrP antagonist activity described in Japanese Patent Application Laying-Open (kokai) No. 7-165790, Japanese Patent Application Laying-Open (kohyo) No. 5-509098, and Peptides (The United States) 1995, 16 (6) 1031-1037, Biochemistry (The United States) Apr. 28, 1992, 31 (16) 4026-4033. Moreover, among the above-stated polypeptides, polypeptides which comprise a deletion, substitution, addition or insertion of at least one amino acid and have an equivalent amount of PTH or PTHrP antagonist activity, are also included in the PTH or PTHrP antagonist of the present invention. However, examples are not limited thereto.

The term "a ligand" is used to mean a substance binding to an enzyme or receptor.

The term "a substance binding to a ligand of a PTH receptor or PTHrP receptor to inhibit binding between the ligand and the receptor" is used to mean a substance (e.g. an anti-PTH antibody, an anti-PTHrP antibody, etc.) which inhibits binding of a ligand (e.g. PTH, PTHrP etc.) to a PTH receptor or PTHrP receptor by binding to the ligand of the PTH receptor or PTHrP receptor. Examples of an anti-PTH antibody include an antibody recognizing PTH (1-34) etc. Examples of an anti-PTHrP antibody include antibodies such as a humanized antibody, a human antibody (WO96/33735) and a chimeric antibody (Japanese Patent Application Laying-Open (kokai) 4-228089), and an antibody (a #23-57-137-1 antibody) produced by hybridoma #23-57-137-1, etc. Note that the antibody may be a polyclonal antibody, but a monoclonal antibody is preferable.

The term "QOL" is an abbreviation for "quality of life" and stands for the quality of life. Cancer patients undergo loss of body weight, anorexia, anemia and pain etc. and so their parameters of QOL are significantly damaged.

The term "central nervous system" is used to mean a nervous system consisting of brain and spinal cord.

The term "PTH or PTHrP-cytokine cascade" is described as follows. First, the term "cytokine cascade" stands for a part of cytokine network and means that information of a first cytokine is read by messengers of a second cytokine and a third cytokine. Then, "PTH or PTHrP-cytokine cascade" is used to mean that PTH or PTHrP is included among cytokine members.

The term "cytokine network" is used to mean antigen-nonspecific network control performed by means of factors (cytokines) produced from immunocompetent cells etc.

The term "a humanized antibody" is used to mean an antibody comprising frameworks derived from a human antibody and complementarity determining regions (CDRs) derived from an antibody other than human (e.g. a mouse antibody).

The present specification includes part or all of the contents disclosed in the specification and/or drawings of Japanese Patent Application No. 11-189793, which is a priority document of the present application.

As an example of the use of "a substance binding to a ligand of a PTH receptor or PTHrP receptor to inhibit binding between the ligand and the receptor", an anti-PTHrP antibody is described below.

### 1. Anti-PTHrP antibody

The anti-PTHrP antibody used in the present invention may be any one, regardless of its source, type (monoclonal or polyclonal) and configuration, as long as it can exhibit a desired pharmacological effect.

The anti-PTHrP antibody used in the present invention can be produced by any known method as a polyclonal or monoclonal antibody. Preferably, the anti-PTHrP antibody is a monoclonal antibody derived from a mammal. The mammal-derived monoclonal antibody includes those produced from a hybridoma and those produced by a genetic engineering technique from a host transformed with a recombinant expression vector carrying a gene for the antibody. The antibody can bind to PTHrP to prevent binding of the PTHrP to a PTH/PTHrP receptor, thus blocking the signal transduction of the PTHrP and consequently inhibiting the biological activity of the PTHrP.

A specific example of such antibody is #23-57-137-1 antibody which can be produced with a hybridoma clone #23-57-137-1. The hybridoma done #23-57-137-1 has been designated as "mouse-mouse hybridoma #23-57-137-1" and deposited under the terms of the Budapest Treaty on August 15, 1996 at the National Institute of Bioscience and Human-technology, Agency of Industrial Science and Technology, Japan (1-3, Higashi 1-chome, Tsukuba-shi, Ibaraki, Japan) under the accession No. FERM BP-5631.

### 2. Antibody-producing hybridoma

A monoclonal antibody-producing hybridoma can be produced as follows. That is, PTHrP is used as an antigen for immunization in accordance with a conventional immunization method. The resulting immunocytes are fused to known parent cells by a conventional cell fusion method, and monoclonal antibody-producing cells are screened from the fused cells by a conventional screening method.

First, a human PTHrP, which is used as an sensitizing antigen for producing the antibody, is prepared by expressing the PTHrP gene/amino acid sequence disclosed in Suva, L. J. et al., Science (1987) 237, 893. A nucleotide sequence encoding the PTHrP is inserted into a known expression vector, and a suitable host cell is transformed with the expression vector. The PTHrP protein is then isolated and purified from the transformed host cell or from a culture supernatant of the transformed host cell by any known method.

Then, the purified PTHrP protein is used as a sensitizing antigen. Alternatively, a 34-amino acid peptide of the N-terminal region of the PTHrP may be chemically synthesized as the sensitizing antigen.

The mammal to be immunized with the sensitizing antigen is not particularly limited. However, the mammal is preferably selected taking into consideration of compatibility with the patent cell used for cell fusion. Generally, a rodent (e.g., mouse, rat, hamster), rabbit or monkey may be used.

The immunization of the mammal with the sensitizing antigen can be performed in accordance with any known method, for example, by injecting the sensitizing antigen to a mammal intraperitoneally or subcutaneously. More specifically, the sensitizing antigen is properly diluted with or suspended to phosphate-buffered saline (PBS) or physiological saline, the resulting dilution or suspension is then mixed with an appropriate amount of a conventional adjuvant (e.g., Freund's complete adjuvant) to give an emulsion. The emulsion is injected to a mammal several times at intervals of 4 to 21 days. For the immunization, the sensitizing antigen may be attached to a suitable carrier.

After the immunization, the serum antibody level is checked. When the serum antibody level is confirmed to reach a desired level, immunocytes are isolated from the mammal and then subjected to cell fusion. A preferable immunocyte is a spleen cell.

The parent cell used for the cell fusion (i.e., the counterpart of the cell fusion with the immunocyte) is a myeloma cell derived from a mammal. The myeloma cell is of any known cell line, and, for example, P3 (P3x63Ag8.653) (J. Immnol. (1979) 123, 1548-1550), P3x63Ag8U.1 (Current Topics in Microbiology and Immunology (1978) 81, 1-7), NS-1 (Kohler, G. and Milstein, C. Eur. J. Immunol. (1976) 6, 511-519), MPC-11 (Margulies, D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S., J. Exp. Med. (1978) 148, 313-323) or R210 (Galfre, G. et al., Nature (1979) 277, 131-133).

Cell fusion of the immunocyte to the myeloma cell is basically performed in accordance with any known method, such as the method of Milstein et al. (Kohler, G. and Milstein, C., Methods Enzymol. (1981) 73, 3-46).

More specifically, the cell fusion is performed, for example, in a conventional nutrient culture medium in the presence of a cell fusion promoter. The cell fusion promoter may be polyethylene glycol (PEG) or a Sendai virus (hemagglutinating virus of Japan; HVJ). If desired, for the purpose of improving the fusion efficiency, an additive such as dimethyl sulfoxide may be incorporated.

The ratio between the immunocytes and the myeloma cells for the cell fusion may be any one. For example, the immunocytes are used in the amount 1-10 times larger than the myeloma cells. The culture medium used for the cell fusion is, for example, RPMI 1640 medium or MEM medium suitable for the growth of the above-mentioned myeloma cell lines, or other medium conventionally used for the culture of such cell lines. If desired, a serum supplement, such as feral calf serum (FCS), may be added to the culture medium.

The cell fusion is performed by fully mixing given amounts of the immunocytes and the myeloma cells in the culture medium, adding a PEG solution (e.g., mean molecular weight: about 1000-6000) (which has been previously warmed to about 37° C) to the mixture usually to a concentration of 30-60% (w/v), and then mixing the resulting solution, thereby producing the desired fusion cells (i.e., hybridomas). Subsequently, an appropriate culture medium is added to the culture solution successively, and centrifuged to remove the supernatant. This procedure is repeated several times to remove the cell fusion promoter or the like that are undesirable for the growth of the hybridomas, from the culture medium.

The thus obtained hybridomas can be selected by culturing in a conventional selective medium, such as hypoxanthine-aminopterin-thymidine (HAT) medium. The culturing of the hybridomas in HAT medium is performed for the time of period enough to cause the death of the cells other than the desired hybridomas (i.e., cells that fail to fuse), usually for several days to several weeks. Subsequently, conventional limiting dilution method is performed for screening and mono-cloning of the hybridomas that are secreting the desired antibody.

As a method other than preparing the hybridomas by immunizing a non-human mammal with the antigen as described above, a human lymphocyte may be sensitized with PTHrP in vitro, and then subjected the sensitized lymphocyte to cell fusion to a human-derived myeloma cell capable of infinite growth, thereby producing a human antibody having a binding activity against the PTHrP (Japanese Patent Publication No. 1-59878). Alternatively, a human antibody against PTHrP may be prepared by injecting PTHrP as an antigen to a transgenic animal that has the entire repertories of human antibody genes to produce an anti-PTHrP antibody-producing cell, and then immortalizing the cells, thus producing the human antibody from the immortalized cell (International Patent Publication Nos. WO 94/25585, WO 93/12227, WO 92/03918 and WO 94/02602).

The monoclonal antibody-producing hybridoma prepared as above can be subcultured in a conventional culture medium and stored under liquid nitrogen for a long time of period.

For the production of a monoclonal antibody from the hybridoma, a method may be employed that involves culturing the hybridoma in accordance with a conventional technique and collecting the monoclonal antibody from the culture supernatant, or that involves injecting the hybridoma to a mammal compatible with the hybridoma to grow the hybridoma in the mammal and collecting the hybridoma from the ascites of the mammal. The former method is suitable for producing the antibody in high purity, while the latter method is suitable for producing the antibody in a large amount.

### 3. Recombinant antibody

In the present invention, a recombinant-type monoclonal antibody may be used, which can be produced by cloning an antibody gene from the hybridoma, integrating the antibody gene into a suitable vector, introducing the vector into a host, and then producing the antibody from the host according to a conventional genetic recombination technique (see, for example, Vandamme, A. M. et al., Eur. J. Biochem. (1990) 192, 767-775, 1990)

Specifically, mRNA encoding variable (V) region of an anti-PTHrP antibody is isolated from the anti-PTHrP antibody-producing hybridoma. The isolation of the mRNA is performed by preparing a total RNA by any known method, such as guanidium ultracentrifugation method (Chirgwin, J. M. et al., Biochemistry (1979) 18, 5294-5299) and AGPC method (Chomczynski, P. et al., Anal. Biochem. (1987) 162, 156-159), and then producing the desired mRNA from the total RNA using mRNA Purification Kit (Pharmacia) or the like. Alternatively, the mRNA may also be prepared directly using QuickPrep mRNA Purification Kit (Pharmacia).

Next, cDNA for the antibody V-region is synthesized from the mRNA with a reverse transcriptase. The synthesis of the cDNA is performed using AMV Reverse Transcriptase First-strand cDNA Synthesis Kit (Seikagaku Corporation) or the like. The cDNA may also be synthesized and amplified by 5'-RACE method (Frohman, M.A. et al., Proc. Natl. Acad. Sci. USA (1988) 85, 8998-9002; Belyavsky, A. et al., Nucleic Acids Res. (1989) 17, 2919-2932) using 5'-Ampli FINDER RACE Kit (CLONETECH) in combination with PCR method, or the like.

A DNA fragment of interest is isolated and purified from the resulting PCR product and then ligated to a vector DNA to obtain a recombinant vector. The recombinant vector is introduced into a host such as E. coli, and a colony containing a desired recombinant vector is selected. The nucleotide sequence of the DNA of interest in the recombinant vector is confirmed by, for example, dideoxynucleotide chain termination method.

Once DNA encoding the anti-PTHrP antibody V-region is obtained, the DNA is integrated into an expression vector containing a DNA encoding a desired antibody constant (C) region.

For the production of the anti-PTHrP antibody used in the present invention, the antibody gene is integrated into an expression vector so that the antibody gene can be expressed under the control of expression control regions (e.g., enhancer, promoter). A host cell is transformed with the expression vector to express the antibody.

In the expression of the antibody gene, a DNA encoding heavy (H) chain and a DNA encoding light (L) chain of the antibody may be integrated into separate expression vectors, and then a host cell is co-transformed with the resulting recombinant expression vectors. Alternatively, both the DNA encoding H-chain and the DNA encoding L-chain of the antibody may be integrated together into a single expression vector, and then a host cell may be transformed with the resulting recombinant expression vector (WO 94/11523).

For the production of the recombinant antibody, besides the above-mentioned host cells, a transgenic animal may also be used as a host. For example, the antibody gene is inserted into a predetermined site of a gene encoding a protein inherently produced in the milk of an animal (e.g., goat β -casein) to obtain a fusion gene. A DNA fragment containing the antibody gene-introduced fusion gene is injected into an embryo of a goat, and the embryo is then introduced into a female goat. The female goat having the embryo therein bears a transgenic goat. The antibody of interest is secreted in the milk from the transgenic goat or a progeny thereof. For the purpose of increasing the amount of the antibody-containing milk from the transgenic goat, an appropriate hormone may be administered to the transgenic goat (Ebert, K.M. et al., Bio/Technology (1994) 12, 699-702).

### 4. Modified antibody

In the present invention, for the purpose of reducing the heterogenisity against a human body or the like, an artificially modified recombinant antibody may be used, such as a chimeric antibody and a humanized antibody. These modified antibodies can be prepared by the following known methods.

A chimeric antibody usable in the present invention can be prepared by ligating the DNA encoding the antibody V-region prepared as set forth above to a DNA encoding a human antibody C-region, integrating the ligation product into an expression vector, and introducing the resulting recombinant expression vector into a host to produce the chimeric antibody.

A humanized antibody is also referred to as a "reshaped human antibody", in which the complementarity determining regions (CDRs) of an antibody of a non-human mammal (e.g., a mouse) are grafted to those of a human antibody. The general genetic recombination procedures for producing such humanized antibody are also known (EP 125023; WO 96/02576).

Specifically, a DNA sequence in which mouse antibody CDRs are ligated through framework regions (FRs) of a human antibody is amplified by PCR method using several oligonucleotides as primers which have been designed to have regions overlapping to the terminal regions of the CDRs and the FRs. The resulting DNA is ligated to a DNA encoding a human antibody C-region, and the ligation product is integrated into an expression vector. The resulting recombinant expression vector is introduced into a host, thereby producing the humanized antibody (EP 239044, WO 96/02576).

The FRs of the human antibody ligated through the CDRs are selected so that the CDRs can form a suitable antigen binding site. If necessary, an amino acid(s) in the FRs of the antibody V-region may be replaced so that the CDRs of the reshaped human antibody can form a suitable antigen binding site (Sato, K. et al., Cancer Res. (1993) 53, 851-856).

The C-region of the chimeric or humanized antibody may be any human antibody C-region, such as C γ 1, C γ 2, C γ 3 or C γ 4 for the H-chain, and C κ or C λ for the L-chain. The human antibody C-region may be modified for the purpose of improving the stable production of the antibody.

The chimeric antibody is composed of V-regions derived from a non-human mammalian antibody and C-regions derived from a human antibody. The humanized antibody is composed of CDRs derived from a non-human mammalian antibody and FRs and C-regions derived from a human antibody. The humanized antibody is useful as an active ingredient for the agent of the present invention, because the antigenicity of the antibody against a human body is reduced.

A specific example of the humanized antibody usable in the present invention is humanized #23-57-137-1 antibody; in which the CDRs are derived from mouse-derived #23-57-137-1 antibody; the L-chain is composed of the CDRs ligated through three FRs (FR1, FR2 and FR3) derived from human antibody HSU 03868 (GEN-BANK, Deftos, M. et al., Scand. J. Immunol., 39, 95-103, 1994) and a FR (FR4) derived from human antibody S25755 (NBRF-PDB); and the H-chain is composed of the CDRs ligated through FRs derived from human antibody S31679 (NBRF-PDB, Cuisinier, A. M. et al., Eur. J. Immunol, 23, 110-118, 1993) in which a part of the amino acid residues in the FRs is replaced so that the reshaped humanized antibody can exhibit an antigen-binding activity.

The E. coli strains containing plasmids having DNA encoding the H-chain and the L-chain of the humanized #23-57-137-1 antibody are designated as Escherichia coli JM109 (hMBC1HcDNA/pUC19) (for H-chain) and Escherichia coli JM109 (hMBC1Lq λ /pUC19) (for L-chain), respectively. These strains were deposited under the terms of the Budapest Treaty on August 15, 1996 at the National Institute of Bioscience and Human-technology, Agency of Industrial Science and Technology, Japan (1-3, Higashi 1-chome, Tsukuba-shi, Ibaraki, Japan), under the accession No. FERM BP-5629 for Escherichia coli JM109 (hMBC1HcDNA/pUC19), and under the accession No. FERM BP-5630 for Escherichia coli JM109 (hMBC1Lq λ/pUC19).

### 5. Antibody variants

The antibody used in the present invention may be a fragment thereof or a modified form of the fragment, as long as it binds to PTHrP to inhibit the activity thereof. For example, the fragment of the antibody includes Fab, F(ab')₂, Fv, or a single chain Fv (scFv) composed of a H-chain Fv fragment and a L-chain Fv fragment linked together through a suitable linker. Specifically, such antibody fragments can be produced by cleaving the antibody with an enzyme (e.g., papain, pepsin) into antibody fragments, or by constructing a gene encoding the antibody fragment and inserting the gene into an expression vector and introducing the resulting recombinant expression vector into a suitable host cell, thereby expressing the antibody fragment (see, for example, Co, M. S., et al., J. Immunol. (1994), 152, 2968-2976; Better, M. & Horwitz, A. H., Methods in Enzymology (1989), 178, 476-496, Academic Press, Inc.; Plueckthun, A. & Skerra, A., Methods in Enzymology (1989) 178, 476-496, Academic Press, Inc.; Lamoyi, E., Methods in Enzymology (1989) 121, 652-663; Rousseaux, J. et al., Methods in Enzymology (1989) 121, 663-669; and Bird, R. E. et al., TIBTECH (1991) 9, 132-137).

A scFv can be produced by linking the H-chain V-region to the L-chain V-region through a linker, preferably a peptide linker (Huston, J. S. et al., Proc. Natl. Acad. Sci. USA (1988) 85, 5879-5883). The H-chain V-region and the L-chain V-region in the scFv may be derived from any one of the antibodies described herein. The peptide linker which binds the V-regions may be any single chain peptide, for example, of 12-19 amino acid residues.

The DNA encoding the scFv can be prepared by first amplifying a DNA encoding the H-chain V-region and a DNA encoding the L-chain V-region of the antibody separately using a DNA fragment encoding the entire region or a part of the H-chain that includes the V-region and a DNA fragment encoding the entire region or a part of the L-chain that includes the V-region as templates and primer pairs that define the terminal ends of the DNA fragments; and then amplifying a DNA encoding the peptide linker using a DNA fragment encoding the peptide linker as a template and a primer pair that define the terminal ends of the DNA fragment so that each terminal end of the peptide linker is ligated to the H-chain V-region and the L-chain V-region, respectively.

Once the DNA encoding the scFv is prepared, an expression vector carrying the DNA and a host transformed with the expression vector can be prepared by conventional methods. The scFv can be produced from the transformed host by a conventional method.

The fragments of the antibody may be produced by preparing genes for the fragments and expressing the genes in suitable hosts as described above. The antibody fragments is also encompassed in the "antibody" of the present invention.

As a modified form of the above-mentioned antibodies, for example, anti-PTHrP antibody conjugated to any molecule (e.g., polyethylene glycol) may also be used. Such modified antibodies are also encompassed in the "antibody" of the present invention. The modified antibodies can be prepared by chemical modifications of the antibodies. The chemical modification techniques suitable for this purpose have already been established in the art.

### 6. Expression and production of recombinant antibody or modified antibody

The antibody gene constructed as described above can be produced and expressed by known methods. For the expression in a mammalian cell, a conventional useful promoter, the antibody gene to be expressed and a poly(A) signal (located downstream to the 3' end of the antibody gene) are operably linked. For example, as the useful promoter/enhancer system, a human cytomegalovirus immediate early promoter/enhancer system may be used.

Other promoter/enhancer systems usable in the expression of the antibody used in the present invention include those derived from viruses (e.g., retrovirus, polyoma virus, adenovirus and simian virus 40 (SV40)) and those derived from mammalian cells (e.g., human elongation factor 1 α (HEF1α).

When SV40 promoter/enhancer system is used, the gene expression may be performed readily by the method of Mulligan et al. (Nature (1979) 277, 108). When HEF1 α promoter/enhancer system is used, the gene expression may be performed readily by the method of Mizushima et al. (Nucleic Acids Res. (1990) 18, 5322).

For the expression in E. coli, a conventional useful promoter, a signal sequence for secreting the antibody of interest and the antibody gene may be operably linked. As such a promoter, lacZ promoter or araB promoter may be used. When lacZ promoter is used, the gene expression may be performed by the method of Ward et al. (Nature (1098) 341, 544-546; FASBE J. (1992) 6, 2422-2427). When araB promoter is used, the gene expression may be performed by the method of Better et al. (Better et al., Science (1988) 240, 1041-1043).

Regarding the signal sequence for secretion of the antibody, when the antibody of interest is intended to be secreted in a periplasmic space of the E. coli, pelB signal sequence (Lei, S. P. et al., J. Bacteriol. (1987) 169, 4379) may be used. The antibody secreted into the periplasmic space is isolated and then refolded so that the antibody takes an appropriate configuration for use.

Regarding the replication origin, those derived from viruses (e.g., SV40, polyoma virus, adenovirus, bovine papilloma virus (BPV)) or the like may be used. In order to increase the gene copy number in the host cell system, the expression vector may further contain a selective marker gene, such as an aminoglycoside phosphotranferase (APH) gene, a thymidine kinase (TK) gene, an E. coli xanthine-guanine phosphoribosyltransferase (Ecogpt) gene and a dihydrofolate reductase (dhfr) gene.

For the production of the antibody used in the present invention, any expression system such as eukaryotic and prokaryotic cell systems may be used. The eukaryotic cell includes established cell lines of animals (e.g., mammals, insects, molds and fungi, yeast). The prokaryotic cell includes bacterial cells such as E. coli cells. It is preferable that the antibody used in the present invention be expressed in a mammalian cell, such as a CHO, COS, myeloma, BHK, Vero or HeLa cell.

Next, the transformed host cell is cultured in vitro or in vivo to produce the antibody of interest. The culturing of the host cell may be performed by any known method. The culture medium usable herein may be DMEM, MEM, RPMI 1640 or IMDM medium. The culture medium may contain a serum supplement, such as fetal calf serum (FCS).

### 7. Isolation and purification of antibody

The antibody expressed and produced as described above may be isolated from the cells or the host animal body and purified to uniformity. The isolation and purification of the antibody used in the present invention may be performed on an affinity column. Examples of a protein A column include Hyper D, POROS and Sepharose F.F. (Pharmacia). The method is not particularly limited and other methods conventionally used for the isolation and purification of an antibody may also be employed. For example, various chromatographs using columns other than the above-mentioned affinity column, filtration, ultrafiltration, salting out and dialysis may be used singly or in combination to isolate and purify the antibody of interest (Antibodies A Laboratory Manual. Ed. Harlow, David Lane, Cold Spring Harbor Laboratory, 1988).

### 8. Determination of the activities of the antibody

The determination of the antigen-binding activity (Antibodies A Laboratory Manual, Ed. Harlow, David Lane, Cold Spring Harbor Laboratory, 1988) or the inhibitory activity against a ligand receptor (Harada, A. et al., International Immunology (1993) 5, 681-690) of the antibody used in the present invention may be performed by any known methods.

The method for the determination of the antigen-binding activity of the anti-PTHrP antibody used in the present invention may be ELISA (enzyme-linked immunosorbent assay), EIA (enzyme immunoassay), RIA (radioimmunoassay) or a fluorescent antibody. For example, when enzyme immunoassay is employed, a sample solution containing the anti-PTHrP antibody (e.g., a culture supernatant of anti-PTHrP antibody-producing cells, or the anti-PTHrP antibody in a purified form) is added to a plate on which PTHrP (1-34) is previously coated. A secondary antibody labeled with an enzyme (e.g., alkaline phosphatase) is further added to the plate. The plate is incubated and washed. A substrate for the enzyme (e.g., p-nitrophenylphosphoric acid) is added to the plate, and the absorbance of the solution in the plate is measured to evaluate the antigen-binding activity of the antibody.

To confirm the activity of the antibody used in the present invention, a neutralizing activity of the antibody (e.g., anti-PTHrP antibody) may be determined.

### 9. Routes for administration and pharmaceutical preparations

The agent of the present invention can be used as a therapeutic agent for diseases caused by PTH or PTHrP; a QOL improving agent alleviating symptoms of diseases caused by PTH or PTHrP; an improving agent for central nervous system diseases caused by PTH or PTHrP; an improving agent for diseases caused by PTH or PTHrP-cytokine cascade; a central nervous system regulator; and a cytokine network regulator etc. The agent of the present invention can be administered for any one or a plurality of the above uses.

An agent containing the anti-PTHrP antibody of the present invention as an active ingredient may be administered orally or parenterally, but preferably parenterally. Specifically, the agent may be administered to a body as a whole or regionally, taking any dosage form, such as a transpulmonary agent (e.g., an agent administered with the help of a device such as a nebulizer), a nasogastric agent, a transdermic agent (e.g., ointment, cream) and an injection. Examples of an injection include an intravenous injection such as a drip, an intramuscular injection, an intraperitoneal injection and a subcutaneous injection. The route of administration may be properly selected depending on the age of a patient and the conditions of diseases. An effective single dose may be selected within the range from 0.001 to 1,000 mg per kg of body weight. Alternatively, the dose to a patient may be selected within the range from 0.01 to 100,000 mg/body, preferably 0.1 to 10,000 mg/body, more preferably 0.5 to 1,000 mg/body, and further more preferably 1 to 100mg/body. However, the dose of the agent comprising the anti-PTHrP antibody of the present invention is not particularly limited to these ranges.

The agent may be administered to a patient at any stage, including before or after the development of disease or symptoms, or may also be administered at a stage when body weight reduction is predicted in a patient.

The agent comprising the anti-PTHrP antibody as an active ingredient of the present invention may be formulated by any conventional method (Remington's Pharmaceutical Science, latest edition, Mark Publishing Company, Easton, USA). The preparation may further comprise pharmaceutically acceptable carriers and additives.

Examples of such carriers and additives include water, pharmaceutically acceptable organic solvents, collagen, polyvinyl alcohol, polyvinyl pyrrolidone, carboxyvinyl polymer, sodium carboxymethyl cellulose, poly(sodium acrylate), sodium arginate, water soluble dextran, sodium carboxymethyl starch, pectin, methyl cellulose, ethyl cellulose, xanthane gum, gum arabic, casein, agar, polyethylene glycol, diglycerin, glycerin, propylene glycol, vaseline, paraffin, stearyl alcohol, stearic acid, human serum albumin (HSA), mannitol, sorbitol, lactose, and surfactants acceptable as pharmaceutical additives.

In the practical use, the additive is properly selected from the above members either singly or in combination depending on (without limitation) the dosage form of the agent of the present invention employed. For example, for use as an injectable form, the anti-PTHrP antibody of the purified form is dissolved in a solvent (e.g., physiological saline, a buffer, a grape sugar solution) and then an adsorption-preventing agent (e.g., Tween 80, Tween 20, a gelatin, human serum albumin) is added thereto. The therapeutic agent of the present invention may also be in a re-constitutable freeze-dried form, which is dissolved before use. For the formulation of the freeze-dried dosage form, an excipient such as a sugar alcohol (e.g., mannitol, grape sugar) or a sugar may be incorporated.

### BRIEF DESCRIPTION OF DRAWINGS

Fig. 1 is a figure showing effect of a humanized anti-PTHrP antibody on blood vasopressin level in high PTHrP-related hypercalcemia model rats.
Fig. 2 is a figure showing effect of a humanized anti-PTHrP antibody on urine volume in high PTHrP-related hypercalcemia model rats.
Fig. 3 is a figure showing life-prolonging effect of a humanized anti-PTHrP antibody in septicemia model rats.
Fig. 4 is a figure showing the results of pharmacological study of a humanized anti-PTHrP antibody and alendronate in high PTHrP-related hypercalcemia model rats.
Fig. 5 is a figure showing effect of a humanized anti-PTHrP antibody on amount of autonomic movement in high PTHrP-related hypercalcemia model rats.

### BEST MODE FOR CARRYING OUT THE INVENTION

The present invention is further described in the following examples. However, the examples are provided for illustrative purposes only, and are not intended to limit the scope of the invention.

### [EXAMPLE 1]

### Low vasopressin level improving effect by anti-PTHrP antibody

A living organism has control mechanisms for maintaining various electrolytes in body fluid at their optimum levels in order to control its body water content. Vasopressin (alias: antidiuretic hormone (ADH), one kind of posterior pituitary hormones) is known as a hormone controlling metabolism of electrolyte and water. Further, various types of diseases caused by disorder of this hormone are also known. Examples of such diseases include posterior pituitary gland hypergasia (diabetes insipidus) and vasopressin secretion abnormality etc.

Among these symptoms, polydipsia, polyuria and mouth dryness etc. are particularly characteristic clinical symptoms. PTHrP is known as a causal substance for humoral hypercalcemia of malignancy (HHM). The onset mechanism of HHM, which is caused by PTHrP produced by malignancy, relates to promotion of bone resorption and promotion of calcium resorption in the kidneys. It is known that, once hypercalcemia develops, polyuria caused by vasopressin adiaphoria or dehydration relative to anorexia, vomitation and nausea caused by the disease itself occurs, and thereby hypercalcemia being more promoted.

### (1) Purpose:

Using a human high PTHrP-related hypercalcemia model animal, human tumor inoculated nude rat model, the effect of a humanized anti-PTHrP monoclonal antibody on blood vasopressin level and urine volume in the model animal was examined.

### (2) Methods:

As a model animal, a nude rat implanted with human large cell lung carcinoma LC-6 [purchased from the Central Institute for Experimental Animals] was used. It is known that a nude rat implanted with human large cell lung carcinoma LC-6 shows increased blood calcium level along with production of PTHrP as tumor volume increases, and develops loss of body weight and so on. The blood vasopressin level in this human high PTHrP-related hypercalcemia model animal was measured, and the value was compared with that of a normal rat. Further, the effect of a humanized anti-PTHrP monoclonal antibody on blood vasopressin level was analyzed. Furthermore, urine volume was measured and the effect of a humanized anti-PTHrP monoclonal antibody on urine volume was also analyzed.

Preparation and grouping of human high PTHrP-related hypercalcemia model animals were carried out as follows. Using a BALB/c-nu/nu nude mouse (CLEA Japan, Inc.), in vivo transplanted human large cell lung carcinoma LC-6 was removed from the nude mouse, and then finely cut into 3-mm cubic blocks. The resulting tumor blocks were subcutaneously implanted into each of the rats at the lateral region at a ratio of one piece per mouse. As rats, 5-weeks-old male F344/N Jcl-rnu nude rats (CLEA Japan, Inc.) were purchased and acclimatized for 1 week. The resulting 6-weeks-old rats were implanted with the tumor blocks, and about a month and a half after the implantation, the rats with increased blood calcium levels and reduced body weights were used as human high PTHrP-related hypercalcemia model animals for evaluation of drug efficacy. Using blood calcium level and body weight as indicators, the rats were divided into groups so that blood calcium levels and body weights of the rats in the individual groups were averaged.

In experiments regarding vasopressin level measurement, 3mg/kg humanized anti-PTHrP monoclonal antibody were administered to human high PTHrP-related hypercalcemia model animals prepared and divided into groups by the above method via caudal vein once a week, that is, on the day 0, 7, 14, 21, 28 and 35. Alendronate was administered to the model animals via caudal vein twice a week, that is, on the day 0, 3, 7, 10, 14, 17, 21, 24, 28, 31, 35 and 38 at a dose level of 2.5 mg/kg. As a control, phosphate buffered saline (PBS) was administered to each of another group of the model animals via caudal vein on the day 0, 7, 14, 21, 28 and 35. Where an implanted tumor block was clearly deciduated during the experiment period, it was eliminated from counting of results.

For the measurement of blood vasopressin level, blood plasma was collected from descending aorta and separated with EDTA. Since data of individuals whose implanted tumor was clearly deciduated when the blood was collected was eliminated from counting, the number of each group at the time of blood collection was: 12 rats in a humanized anti-PTHrP monoclonal antibody administration group, 3 rats in an alendronate administration group, 8 rats in phosphate buffered saline (PBS) administration group and 5 rats in a normal rat group. The measurement was carried out by RIA method using blood plasma.

In experiments regarding urine volume measurement, 3mg/kg humanized anti-PTHrP monoclonal antibody or 5mg/kg alendronate were administered to human high PTHrP-related hypercalcemia model animals prepared and divided into groups by the above method via caudal vein. As a control, phosphate buffered saline (PBS) was administered to the model animals via caudal vein. On the morning of the day 13 to the morning of the day 14 after administration, urine was collected over 24 hours, and then weight and specific gravity were measured to calculate urine volume.

### (3) Results:

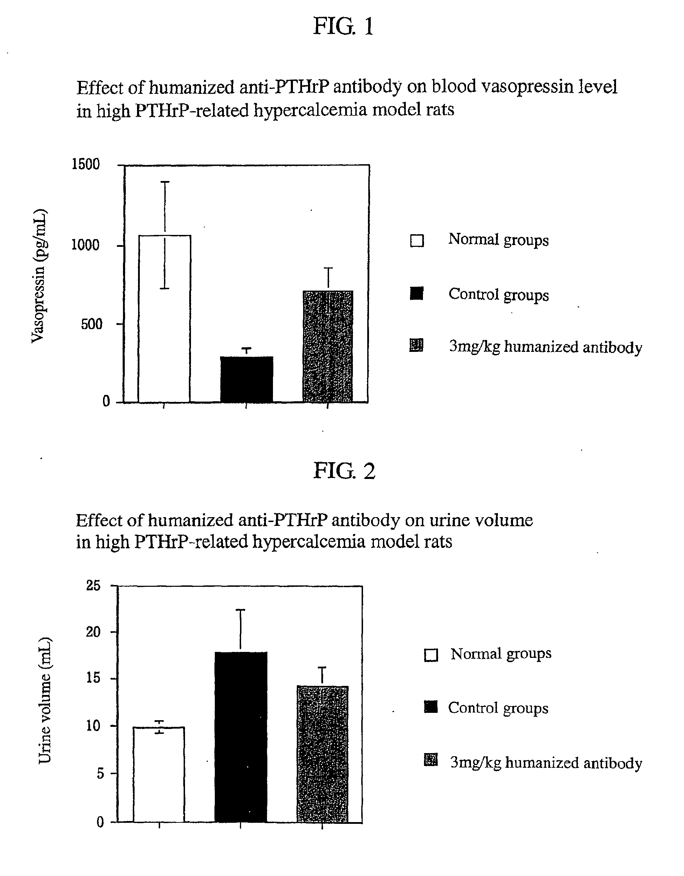

It was found that blood vasopressin level was reduced in human high PTHrP-related hypercalcemia model animals. A humanized monoclonal antibody improved the reduced blood vasopressin level in human high PTHrP-related hypercalcemia model animals (Figure 1). Furthermore, it was also found that the humanized monoclonal antibody has an effect of improving the state of polyuria in human high PTHrP-related hypercalcemia model animals (Figure 2). From these results, it turned out that a humanized anti-PTHrP monoclonal antibody effects recovery from dehydration by means of normalization of blood vasopressin level.

### [EXAMPLE 2] Life-prolonging effect of anti-PTHrP antibody in septicemia model animals Possibility of humanized anti-PTHrP monoclonal antibody as therapeutic agent for septicemia (a therapeutic agent for septicemia)

Septicemia (sepsis) is state wherein microorganisms such as bacteria and fungus, and metabolites thereof continuously spread from an in vivo infectious focus via the circulating blood. Clinical symptoms of this disease are fever, ague, shaking chill, tachycardia and consciousness disorder etc., and after the progression of the disease, it results in septic shock and combines with disorders of various organs such as circulatory failure, disseminated intravascular coagulation syndrome (DIC), adult respiratory distress syndrome (ARDS) and multiple organ failure (MOF) etc., and this disease still shows a high mortality rate.

Triggered by endotoxin derived from bacteria, these various clinical symptoms are developed by progression of a series of reactions, what is known as septicemia cascade. It is known that, above all, the fatty portion of endotoxin constituting cell wall of Gram-negative bacteria (LPS: lipopolysaccharide) has a strong physiological action. In recent years, septicemia has been clarified at the cytokine level, and it has been found that, in septic shock caused by LPS, cytokines such as IL-1, IL-6, IL-8 and IFN γ increase in serum.

Moreover, Funk et al. has reported that, when a large amount of LPS is administered to a mouse, a parathyroid hormone-related peptide (PTHrP) is produced and induced, while various types of cytokines are produced and induced. Still more, it has been reported that life-prolonging effect can be obtained when a goat anti-PTHrP antibody and a rabbit anti-PTHrP antibody are administered to this model (Mol Med 2, 204, 1996).

Establishing life-prolonging effect as an indicator, a retest was performed to examine whether the same effect can be obtained by a humanized anti-PTHrP monoclonal antibody, and the adaptability of the humanized anti-PTHrP monoclonal antibody as a therapeutic agent for septicemia.

### Effect of humanized anti-PTHrP monoclonal antibody against septicemia model animals

### (1) Purpose:

Effect of a humanized anti-PTHrP monoclonal antibody on model animals with septicemia induced by LPS was analyzed to expand adaptability of this antibody.

### (2) Methods:

According to the method of Funk et al., this experiment was carried out.
1. As model animals, 6-week-old normal Jcl:ICR mice (CLEA Japan, Inc.) which were acclimatized for 1 week were used , and septicemia-developed mice were prepared by administration of LPS. Each of 700, 800 and 900 µg/mouse LPS (E. coli 055 : B5 (Difco)) was intraperitoneally administered to mice (n=3), and conditions until death were observed over time. 800 µg/mouse LPS was selected as a dose with which 80% or more mice die over 48 hours.
2. Evaluation of drug efficacy of a humanized anti-PTHrP monoclonal antibody (an antibody comprising, as an L-chain, version q described later) was performed by the following method. Concurrently with (concurrent administration group : n=12) or at 1 hour after (prior administration group : n=13) the administration of 1,000µg/mouse humanized anti-PTHrP monoclonal antibody to mice via caudal vein, 800 µg/mouse LPS was intraperitoneally administered thereto (n=13). The succeeding conditions to death were observed with time (0 to 72 hours), and life-prolonging effect in a humanized anti-PTHrP monoclonal antibody was analyzed by making a comparison between a group to which the antibody was administered and a control group.

### (3) Results:

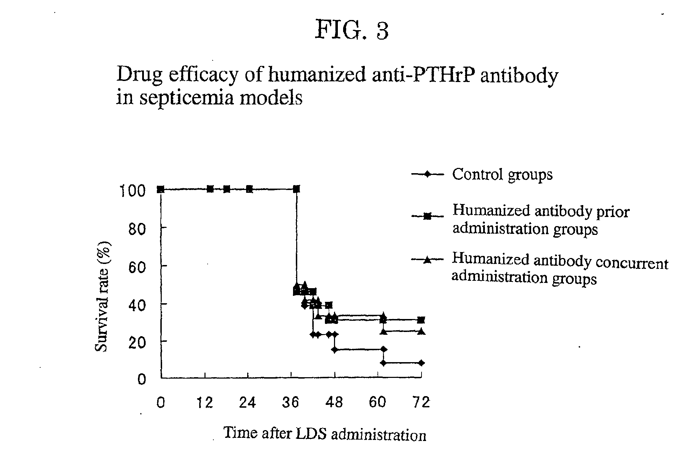

A large number of mice died at 36 hours after administration of LPS in all groups. Survival rate after 48 hours was 15.4% in a control group, 33.3% in a concurrent administration group and 30.8% in a prior administration group, then after 72 hours, 7.7% in a control group, 25.0% in a concurrent administration group and 30.8% in a prior administration group. This result shows that the concurrent or prior administration of a humanized anti-PTHrP monoclonal antibody brought about a life-prolonging effect in LPS-induced septicemia model animals (Figure 3). Accordingly, it was suggested that a humanized anti-PTHrP monoclonal antibody is useful as a therapeutic agent for septicemia.

### [EXAMPLE 3]

### QOL improving effect of anti-PTHrP antibody in human high PTHrP-related hypercalcemia model animals

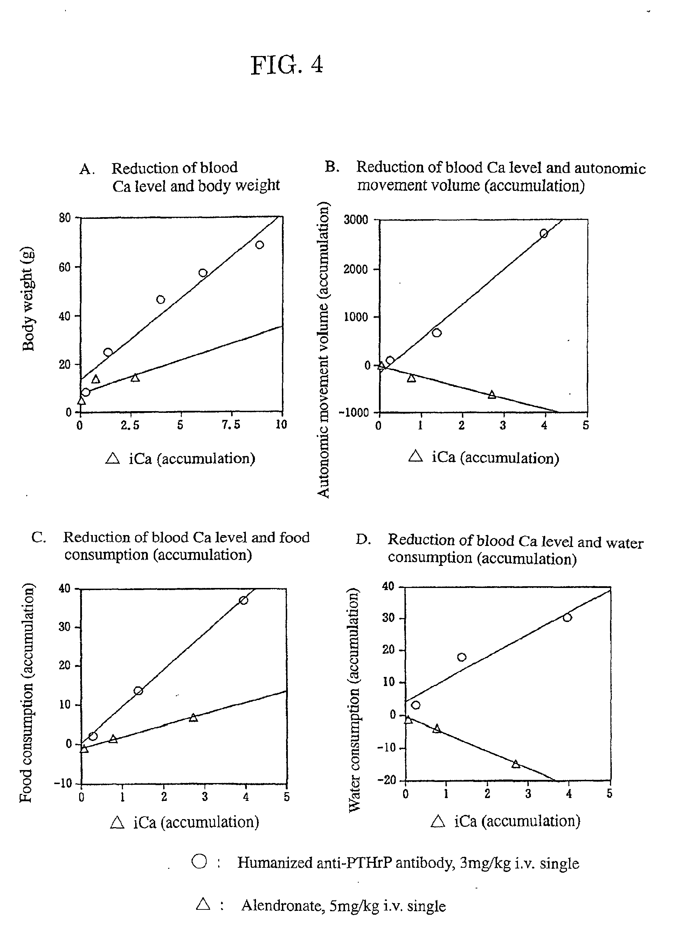

The present inventors had already found that a humanized anti-PTHrP antibody shows not only reduction of blood calcium level but also significant rebound of body weight, increase of amount of autonomic movement and increase of food consumption in human high PTHrP-related hypercalcemia model animals (Japanese Patent Application Laying-Open (kokai) No. 11-92500). There remains some doubt whether the improving effect of QOL parameters such as body weight, amount of autonomic movement and food consumption is provided by improving or corrected to a normal value, blood calcium level, or whether it depends on factors other than stated above. Thus, the present inventors have analyzed the relation between reduction of blood calcium level and increase of body weight, amount of autonomic movement, and food and water consumption. As a brief explanation of the experiment, high PTHrP-related hypercalcemia model animals were prepared by subcutaneously implanting a PTHrP-producing tumor, human large cell lung carcinoma LC-6, to rats. After that, 3mg/kg humanized anti-PTHrP monoclonal antibody were intravenously administered to the thus prepared model animals. As a control group, 5mg/kg alendronate (a bisphosphonate formulation) were intravenously administered (a single administration). Then, blood ionized calcium (iCa) level, body weight, food and water consumption of the animals were measured. From results of parameters obtained from this experiment, an accumulated value (an area calculated from a graph, setting the stalling point of agent administration starting point as 0 level). Regarding body weight, however, the value was directly used. The graph was prepared, setting the accumulated value of blood calcium level reduction at a horizontal axis and the accumulated value of various parameters at a vertical axis. 5mg/kg alendronate were administered as a control agent. The results are shown below. It was found that, in all of the parameters in comparison with reduction of blood calcium level, the gradient of a line obtained regarding a humanized anti-PTHrP antibody differs from that obtained regarding alendronate (Figure 4A, B, C and D). Especially regarding amount of autonomic movement (Figure 4B) and water consumption (Figure 4D), the sign (positive and negative) of the line was reversed. This result suggests that, in respect of reduction of blood calcium level, a humanized anti-PTHrP antibody provides a much higher QOL improving effect than alendronate (a bisphosphonate formulation). Accordingly, it was suggested that PTHrP is not only a causal substance of humoral hypercalcemia of malignancy, but also a cause of malignancy-associated syndrome by means of a mechanism other than calcium.

Moreover, it became clear that an anti-PHTrP antibody has a significantly higher QOL improving effect than the existing therapeutic agents for hypercalcemia.

### [EXAMPLE 4]

### (1) Purpose:

The effect of a humanized anti-PTHrP monoclonal antibody on autonomic movement of high PTHrP-related hypercalcemia model animals was examined.

### (2) Methods:

High PTHrP-related hypercalcemia model animals were prepared by subcutaneously implanting a PTHrP-producing tumor, human large cell lung carcinoma LC-6, to nude rats. After that, PBS (control) or 5mg/kg humanized anti-PTHrP monoclonal antibody were intravenously administered to the thus prepared model animals (a single administration). Amount of autonomic movement of rats was measured by placing each rat in an individual cage and using an amount of autonomic movement counting device, ANIMEX, counting the frequency. A count was performed on the day 1, 3 and 5 after administration for PBS-administered individuals, and on the day 0 (a pre-administration value), 2, 4 and 6 days for antibody-administered individuals. The count period was for 12 hours, from 7:00PM to 7:00AM the following morning.

### (3) Results:

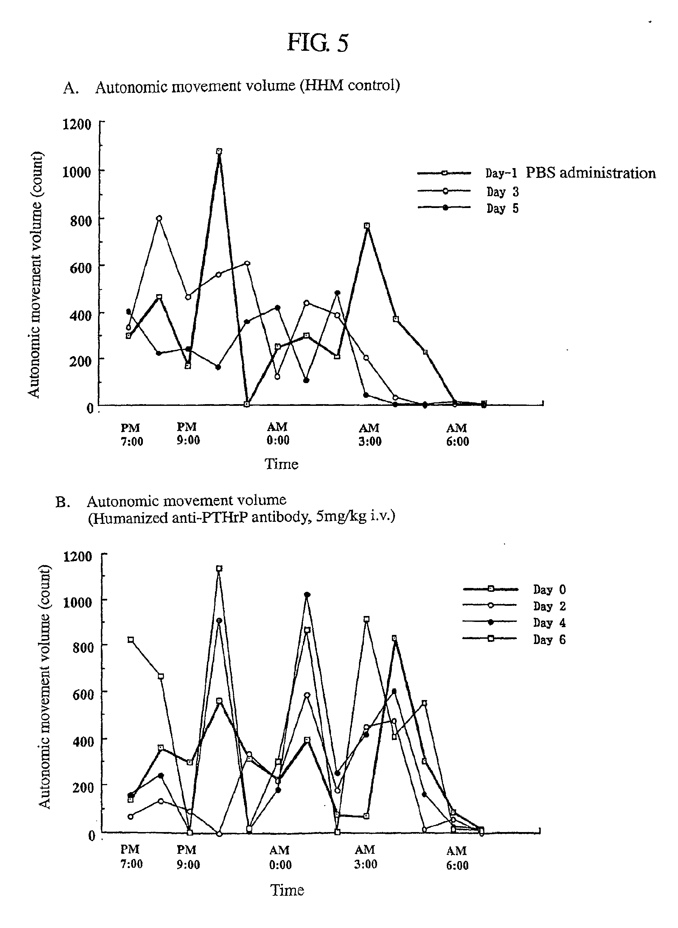

Results were obtained by counting every hour. It is known that autonomic movement patterns of a normal rat has a periodic rhythm. It was found that periodicity of autonomic movement can be observed, but the pattern is irregular in the present high PTHrP-related hypercalcemia models (from results of PBS-administered individuals). In contrast, in antibody-administered individuals, it was found that periodic pattern of the autonomic movement becomes notable in conjunction with increase of the autonomic movement (Figure 5).

### (4) Consideration:

Autonomic movement, in particular, periodicity of the movement etc. is controlled by motor nerve etc. in the central nerve system. In high PTHrP-related hypercalcemia models subjected to the present experiment, not only reduction of amount of movement but also disorder of the periodicity was observed, and effect of high PTHrP-related hypercalcemia on the central nerve system was assumed. From the result that the periodicity of autonomic movement became notable as a result of administration of a humanized monoclonal antibody, it was found that this antibody has an effect of improving the effect of high PTHrP-related hypercalcemia on central nerve system.

### [CONSIDERATION]

### 1. Syndromes associated with malignancy

Examples of syndromes associated with malignancy include digestive system disorders (e.g. diarrhea, vomiturition and nausea), proteometabolism abnormality (e.g. hypoalbuminemia), saccharometabolism abnormality (e.g. reduction of glucose tolerance and reduction of insulin secretion), lipid metabolism abnormality (e.g. hyperlipidemia and reduction of serum lipoprotein lipase ability), anorexia, hematological abnormality (e.g. anemia, thrombosis and DIC syndrome), electrolyte abnormality (e.g. hyponatremia, hypokalemia and hypercalcemia), immunodeficiency (e.g. infection disease), pain. The mechanism of onset of these syndromes associated with malignancy has not been clarified, but many of the symptoms of humoral hypercalcemia of malignancy overlap. As shown in the above Examples, when neutralizing antibody of PTHrP, a causal substance of hypercalcemia, is administered to model animals developing humoral hypercalcemia of malignancy, there can be observed improvements of symptoms such as not only normalization of blood calcium level but also increase in amount of movement, increase of food and water consumption, improvement of polyuria and normalization of vasopressin. As shown in Figure 4, it can hardly be explained that these improving effects are only the secondary effects by reduction (normalization) of blood calcium level, but it can be said that these effects are characteristic and specific functions attributable to PTHrP antagonists such as an anti-PTHrP antibody, irrespective of the effect of normalization of blood calcium level. From the above results, PTHrP is considered to be a causal substance of syndromes associated with malignancy, and various symptoms of the syndromes are considered to be provoked by means of a PTH/PTHrP receptor expressing in various organs. Therefore, it is considered that an active substance blocking a signal to a PTH/PTHrP receptor improves symptoms of syndromes associated with malignancy.

### 2. QOL improving agent

Various symptoms observed in syndromes associated with malignancy, humoral hypercalcemia of malignancy, primary hyperparathyroidism and secondary hyperparathyroidism, that is, symptoms such as digestive system disorders (e.g. diarrhea, vomiturition and nausea), proteometabolism abnormality (e.g. hypoalbuminemia), saccharometabolism abnormality (e.g. reduction of glucose tolerance and reduction of insulin secretion), lipid metabolism abnormality (e.g. hyperlipidemia and reduction of serum lipoprotein lipase ability), anorexia, hematological abnormality (e.g. anemia, thrombosis and DIC syndrome), electrolyte abnormality (e.g. hyponatremia, hypokalemia and hypercalcemia), immunodeficiency (e.g. infection disease), and pain, significantly reduce QOL of a patient. It has been clarified that, among the above diseases, PTH or PTHrP acts as a causal substance in humoral hypercalcemia of malignancy, primary hyperparathyroidism and secondary hyperparathyroidism.

As described in "1. Syndromes associated with malignancy", when neutralizing antibody of PTHrP is administered to model animals developing humoral hypercalcemia of malignancy, there can be observed improvements of symptoms such as not only normalization of blood calcium level but also increase of amount of movement, increase of food and water consumption, improvement of polyuria and normalization of vasopressin. From the above results, PTHrP is considered to be a causal substance of syndromes associated with malignancy, and various symptoms of the syndromes are considered to be provoked by means of a PTH/PTHrP receptor expressing in various organs. Therefore, it is considered that an active substance blocking a signal to a PTH/PTHrP receptor improves the reduction of QOL in diseases such as syndromes associated with malignancy, humoral hypercalcemia of malignancy, primary hyperparathyroidism and secondary hyperparathyroidism.

### 3. Central nerve system

In syndromes associated with malignancy, humoral hypercalcemia of malignancy, primary hyperparathyroidism and secondary hyperparathyroidism, actions towards central nerve such as anorexia, mouth dryness and reduction of amount of movement can be observed. As stated above, improvements of symptoms such as increase of amount of movement, increase of food and water consumption, improvement of polyuria, normalization of vasopressin can be observed by administration of neutralizing antibody of PTHrP. Accordingly, PTHrP is considered as a causal substance of symptoms caused by central nerve, and these symptoms are considered to be provoked by means of a PTH/PTHrP receptor expressing in central nerve system. Therefore, an active substance blocking a signal to a PTH/PTHrP receptor is considered to improve symptoms caused by central nerve. Examples of central nerve system diseases include dyssomnia, neuropathy (e.g. schizophrenia, manic-depressive psychosis, neurosis and psychophysiologic disorder), nervous symptom (e.g. vomiting, nausea, mouth dryness, anorexia and vertigo), brain metabolism abnormality, cerebral circulation abnormality, autonomic imbalance, and endocrine system abnormality with which central nervous system is associated, etc. PTHrP and a PTH/PTHrP receptor express in central nerve system (CNS), but functions thereof have hardly been clarified yet. When localization of PTHrP mRNA in a rat brain was analyzed by in situ hybridization, it was found that the mRNA existed in the hippocampus, the granular cell layer of cerebellum, cerebral cortex and hypothalamus (Weaver et al., Mol Brain Res 28:296-301, 1995; Weir et al., Proc Natl Acad Sci USA 87:108-112, 1990).

Moreover, the distribution of PTH/PTHrP receptor in rat brain matches with the distribution of PTHrP, and so it is assumed that PTHrP acts as a local autocrine/paracrine factor in central nervous system (CNS). An experiment regarding binding of PTH to a cell membrane fraction prepared from each site of rat brain teaches the order of binding strength as hypothalamus, cerebellum and cerebral cortex (Harvey et al., Peptides 14:1187-1191, 1993). Furthermore, it has been reported that arginine vasopressin (AVP) is released by PTHrP (1-34) stimulation of rat supraoptic nucleus (SON) slices, and that there is a possibility that PTHrP involves in homeostasis of in vivo water or electrolyte (Yamamoto et al., Endocrinology 139:383-388, 1998, Yamamoto et al., Endocrinology 138:2066-2072, 1997). Thus, the facts that PTHrP and a PTH/PTHrP receptor are widely distributed in brain and that there are common nervous symptoms and symptoms among the above diseases, suggest involvement of PTH or PTHrP as an onset cause of some of central nervous system diseases. Therefore, an active substance blocking a signal from PTH/PTHrP to the receptor is considered to improve central nervous system diseases.

### 4. Cytokine cascade

As stated above, as reports suggesting the possibility of crosstalk between PTH or PTHrP and cytokine, the following reports are known:
1) The values of IL-6 and TNF-α are high in a patient of primary hyperparathyroidism caused by high value of PTH (Grey A. et al., J Clin Endocrinol Metab 81:3450-5, 1996)
2) When osteoblasts are stimulated by PTH or PTHrP in an in vitro system, expression of IL-6 and LIF is promoted (Pollock JH. et al., J Bone Miner Res 11:754-9, 1996)
3) A series of experiments with synovial cells showed that production of IL-6 is accentuated by stimulation with PTHrP, and that TNF-α and IL-1β promote expression of PTHrP, and it was found that PTHrP is a member of pro-inflammatory cytokine cascade (Funk JL. et al., Endocrinology 138:2665-73, 1997; Funk JL. et al., J Clin Invest 101:1362-71, 1998)
4) In cultured human vascular endothelial cells also, TNF-α and IL-1β promote expression of PTHrP (Biochem Biophys ResCommun 249:339-343, 1998)

Moreover, it is known that not only generation of various cytokines but also that of PTHrP is induced in LPS-induced septicemia models. As shown in Examples, a neutralizing antibody of PTHrP has a life-prolonging effect in LPS-induced septicemia models.

Thus, PTH or PTHrP-cytokine cascade caused by induction of cytokine by PTHrP or induction of PTHrP by cytokine is considered to be involved in onset of pathology. Furthermore, since a life-prolonging effect was observed in LPS-induced septicemia models by blocking a cytokine-production inducing signal by PTHrP in various organs, an active substance blocking a signal from PTH/PTHrP to the receptor can be a therapeutic agent for septicemia, cachexia, inflammation, hemopathy such as hematopoietic system abnormality and leukaemia, calcium metabolism abnormality, and autoimmune disease such as rheumatism.

### [REFERENCE EXAMPLE 1]

### Preparation of hybridomas producing anti-PTHrP (1-34) mouse monoclonal antibody

Hybridomas capable of producing a monoclonal antibody against human PTHrP (1-34) (SEQ ID NO: 75), #23-57-154 and #23-57-137-1, were prepared as follows (see Sato, K. et al., J. Bone Miner. Res. 8, 849-860, 1993). The amino acid sequence of the human PTHrP (1-34) is shown in SEQ ID NO:75.

For use as an immunogen, PTHrP (1-34) (Peninsula) was conjugated with a carrier protein thyroglobulin using carbodiimide (Dojinn). The thycloglobulin-conjugated PTHrP (1-34) was dialyzed to obtain a solution having a protein concentration of 2 µg/ml. The resulting solution was mixed with Freund's adjuvant (Difco) at a mixing ratio of 1:1 to give an emulsion. This emulsion was injected to 16 female BALB/C mice 11 times subcutaneously at the back or intraperitoneally at a dose level of 100 µg/mouse for each injection, thereby immunizing the mice. For the priming immunization, Freund's complete adjuvant was used; while for the boosting immunization, Freund's incomplete adjuvant was used.

Each of the immunized mice was determined for its antibody titer in the serum in the following manner. That is, each of the mice was blood-drawn via its tail vein, and the anti-serum is separated from the blood. The anti-serum was diluted with a RIA buffer and mixed with ¹²⁵I-labeled PTHrP (1-34) to determine binding activity. The mice that were confirmed to have a sufficiently increased titer were injected with PTHrP (1-34) without a carrier protein intraperitoneally at a dose level of 50 µg/mouse for the final immunization.

Three days after the final immunization, the mouse is sacrificed and the spleen was removed therefrom. The spleen cells were subjected to cell fusion with mouse myeloma cell line P3x63Ag8U.1 in accordance with a conventional known method using 50% polyethylene glycol 4000. The fused cells thus prepared were seeded to each well of eighty-five 96-well plates at a density of 2 x 10⁴/well. Hybridomas were screened in HAT medium as follows.

The screening of hybridomas was performed by determining the presence of PTHrP-recognition antibodies in the culture supernatant of the wells in which cell growth had been observed in HAT medium, by solid phase RIA method. The hybridomas were collected from the wells in which binding ability to the PTHrP-recognition antibodies had been confirmed. The hybridomas thus obtained was suspended into RPMI-1640 medium containing 15% FCS supplemented with OPI-supplement (Sigma), followed by unification of the hybridomas by limiting dilution method. Thus, two types of hybridoma clones, #23-57-154 and #23-57-137-1, could be obtained, both which had a high binding ability to PTHrP (1-34).

Hybridoma clone #23-57-137-1 was designated as "mouse-mouse hybridoma #23-57-137-1", and has been deposited under the terms of the Budapest Treaty on August 15, 1996 at the National Institute of Bioscience and Human-technology, Agency of Industrial Science and Technology, Japan (1-3, Higashi 1-chome, Tsukuba-shi, Ibaraki, Japan) under the accession No. FERM BP-5631.

### [REFERENCE EXAMPLE 2]

### Cloning of DNAs encoding V-regions of mouse monoclonal antibody against human PTHrP (1-34)

Cloning of DNAs encoding the V-regions of a mouse monoclonal antibody against human PTHrP (1-34), #23-57-137-1, was performed in the following manner.

### (1) Preparation of mRNA

mRNA from hybridoma #23-57-137-1 was prepared using Quick Prep mRNA Purification Kit (Pharmacia Biotech). That is, cells of hybridoma #23-57-137-1 were fully homogenized with an extraction buffer, and mRNA was isolated and purified therefrom on an oligo(dT)-Cellulose Spun Column in accordance with the instructions included in the kit. The resulting solution was subjected to ethanol precipitation to obtain the mRNA as a precipitate. The mRNA precipitate was dissolved in an elution buffer.

### (2) Production and amplification of cDNA for gene encoding mouse H-chain V-region

### (i) Cloning of cDNA for #23-57-137-1 antibody H-chain V-region

A gene encoding H-chain V-region of the mouse monoclonal antibody against human PTHrP was cloned by 5'-RACE method (Frohman, M. A. et al., Proc. Natl. Acad. Sci. USA, 85, 8998-9002, 1988; Belyavsky, A. et al., Nucleic Acids Res. 17, 2919-2932, 1989). The 5'-RACE method was performed using 5'-Ampli FINDER RACE Kit (CLONETECH) in accordance with the instructions included in the kit. In this method, the primer used for synthesis of cDNA was MHC2 primer (SEQ ID NO: 1) which is capable of hybridizing to mouse H-chain C-region. The above-prepared mRNA (about 2 µg), which was a template for the cDNA synthesis, was mixed with MHC2 primer (10 pmoles). The resulting mixture was reacted with a reverse transcriptase at 52° C for 30 minuets to effect the reverse transcription of the mRNA into cDNA.

The resulting reaction solution was added with 6N NaOH to hydrolyze any RNA remaining therein (at 65° C for 30 min.) and then subjected to ethanol precipitation to isolate and purify the cDNA as a precipitate. The purified cDNA was ligated to Ampli FINDER Anchor (SEQ ID NO: 42) at the 5' end by reacting with T4 RNA ligase at 37° C for 6 hours and additionally at room temperature for 16 hours. As the primers for amplification of the cDNA by PCR method, Anchor primer (SEQ ID NO: 2) and MHC-G1 primer (SEQ ID NO: 3) (S.T. Jones, et al., Biotechnology, 9, 88, 1991) were used.

The PCR solution comprised (per 50µl) 10 mM Tris-HCl (pH 8.3), 50 mM KCl, 0.25 mM dNTPs (dATP, dGTP, dCTP, dTTP), 1.5 mM MgCl₂, 2.5 units of TaKaRa Taq (Takara Shuzo Co., Ltd.), 10 pmoles Anchor primer, and 1 µl of the reaction mixture of the cDNA to which MHC-G1 primer and Ampli FINDER Anchor primer had been ligated, over which mineral oil (50 µl) was layered. The PCR was performed on Thermal Cycler Model 480J (Perkin Elmer) for 30 cycles under the conditions: 94° C for 45 sec.; 60° C for 45 sec.; and 72° C for 2 min.

### (ii) Cloning of cDNA for #23-57-137-1 antibody L-chain V-region

A gene encoding L-chain V-region of the mouse monoclonal antibody against human PTHrP was cloned by 5'-RACE method (Frohman, M. A. et al., Proc. Natl. Acad. Sci. USA, 85, 8998-9002, 1988; Belyavsky, A. et al., Nucleic Acids Res. 17, 2919-2932, 1989). The 5'-RACE method was performed using 5'-Ampli Finder RACE Kit (CLONETECH) in accordance with the instructions included in the kit. In this method, oligo-dT primer was used as the primer for synthesizing cDNA. The above-prepared mRNA (about 2 µg), which was a template for the cDNA synthesis, was mixed with oligo-dT primer. The resulting mixture was reacted with a reverse transcriptase at 52° C for 30 min, to effect the reverse transcription of the mRNA into cDNA. The resulting reaction solution was added with 6N NaOH to hydrolyze any RNA remaining therein (at 65° C for 30 min.). The resulting solution was subjected to ethanol precipitation to isolate and purified the cDNA as a precipitate. The cDNA thus synthesized was ligated to Ampli FINDER Anchor at the 5' end by reacting with T4 RNA ligase at 37° C for 6 hours and additionally at room temperature for 16 hours.

A PCR primer MLC (SEQ ID NO: 4) was designed based on the conserved sequence of mouse L-chain λ chain C-region and then synthesized using 394 DNA/RNA Synthesizer (ABI). The PCR solution comprised (per 100 µl) 10 mM Tris-HCl (pH 8.3), 50 mM KCl, 0.25 mM dNTPs (dATP, dGTP, dCTP, dTTP), 1.5 mM MgCl₂, 2.5 units of AmpliTaq (PERKIN ELMER), 50 pmoles of Anchor primer (SEQ ID NO: 2), and 1 µl of the reaction mixture of the cDNA to which MLC (SEQ ID NO: 4) and Ampli FINDER Anchor were ligated, over which mineral oil (50 µl) was layered. The PCR reaction was performed on Thermal Cycler Model 480J (Perkin Elmer) for 35 cycles under the conditions: 94° C for 45 sec.; 60° C for 45 sec.; and 72° C for 2 min.

### (3) Purification and fragmentation of PCR products

Each of the DNA fragments amplified by PCR method described above was separated by agarose gel electrophoresis on a 3% Nu Sieve GTG agarose (FMC Bio. Products). For each of the H-chain V-region and the L-chain V-region, an agarose gel segment containing a DNA fragment of about 550 bp was excised from the gel. Each of the gel segments was subjected to purification of the DNA fragment of interest using GENECLEAN II Kit (BIO101) in accordance with the instructions included in the kit. The purified DNA was precipitated with ethanol, and the DNA precipitate was dissolved in 20 µl of a solution containing 10 mM Tris-HCl(pH 7.4) and 1 mM EDTA. An aliquot (1 µl) of the DNA solution was digested with a restriction enzyme XmaI (New England Biolabs) at 37° C for 1 hour and further digested with a restriction enzyme EcoRI (Takara Shuzo Co., Ltd.) at 37° C for 1 hour. The digestion solution was extracted with phenol and chloroform and then precipitated with ethanol to collect the DNA.

In this manner, two DNA fragments containing a gene encoding mouse H-chain V-region and a gene encoding mouse L-chain V-region, respectively, were obtained, both which had an EcoRI recognition sequence on the 5' end and an XmaI recognition sequence on the 3' end.

The EcoRI-XmaI DNA fragments containing a gene encoding mouse H-chain V-region and a gene encoding mouse L-chain V-region, respectively, were separately ligeted to pUC19 vector that had been digested with EcoRI and XmaI at 16° C for 1 hour using DNA Ligation Kit ver.2 (Takara Shuzo Co., Ltd.) in accordance with the instructions included in the kit. An aliquot (10 µl) of the ligation mixture was added to 100 µl of a solution containing competent cells of E. coli, JM 109 (Nippon Gene Co., Ltd.). The cell mixture was allowed to stand on ice for 15 min., at 42° C for 1 min. and additionally for 1 min. on ice. The resulting cell mixture was added with 300 µl of SOC medium (Molecular Cloning: A Laboratory Manual, Sambrook, et al., Cold Spring Harbor Laboratory Press, 1989) and then incubated at 37° C for 30 min. The resulting cell solution was plated on LB agar medium or 2xYT agar medium (Molecular Cloning: A Laboratory Manual, Sambrook, et al., Cold Spring Harbor Laboratory Press, 1989) containing either 100 or 50 µg/ml of ampicillin, 0.1 mM of IPTG and 20 µg/ml of X-gal, and then incubated at 37° C overnight. In this manner, E. coli transformants were prepared.

The transformants were cultured at 37° C overnight in 2 ml of LB or 2xYT medium containing either 100 or 50 µg/ml of ampicillin. The cell fraction was applied to Plasmid Extracter PI-100( (Kurabo Industries, Ltd.) or QIAprep Spin Plasmid Kit (QIAGEN) to give a plasmid DNA. The plasmid DNA was sequenced as follows.

### (4) Sequencing of genes encoding mouse antibody V-regions

The nucleotide sequence of the cDNA coding region carried on the plasmid was determined in DNA Sequencer 373A (ABI; Perkin-Elmer) using Dye Terminator Cycle Sequencing Kit (Perkin-Elmer). M13 Primer M4 (Takara Shuzo Co., Ltd.) (SEQ ID NO: 5) and M13 Primer RV (Takara Shuzo Co., Ltd.) (SEQ ID NO: 6) were used as the primers for sequencing, and the nucleotide sequence was confirmed in the both directions.

The plasmid containing a gene encoding mouse H-chain V-region derived from hybridoma #23-57-137-1 was designated as "MBC1H04", and the plasmid containing a gene encoding mouse L-chain V-region derived from hybridoma #23-57-137-1 was designated as "MBC1L24". The nucleotide sequences (including the corresponding amino acids sequences) of the gene encoding the mouse #23-57-137-1 antibody-derived H-chain V-region in plasmid MBC1H04 and the gene encoding the mouse #23-57-137-1 antibody-derived L-chain V-region in plasmid MBC1H24 were shown in SEQ. ID Nos: 57 and 65, respectively. The amino acid sequences of the polypeptides for the H-chain V-region and the L-chain V-region were shown in SEQ. ID NOs: 46 and 45, respectively.

The E. coli strain containing the above plasmid MBC1H04 and the E. coli strain containing the above plasmid MBC1L24 were designated as "Escherichia coli JM109 (MBC1H04)" and "Escherichia coli JM109 (MBC1L24)", respectively. These E. coli strains have been deposited under the terms of the Budapest Treaty at the National Institute of Bioscience and Human-Technology, Agency of Industrial Science and Technology, Japan (1-3, Higashi 1-chome, Tsukuba-shi, Ibaraki, Japan) on August 15, 1996, under the Accession No. FERM BP-5628 for Escherichia coli JM109 (MBC1H04) and FERM BP-5627 for Escherichia coli JM109 (MBC1L24), respectively.

### (5) Determination of CDRs of mouse monoclonal antibody #23-57-137-1 against human PTHrP

The H-chain V-region and the L-chain V-region have general structures similar to each other, each of which has four framework regions (FRs) linked through three hypervariable regions (i.e., complementarity determining regions; CDRs). The amino acid sequences of the FRs are relatively well conserved, while the amino acid sequence of the CDRs have an extremely high variability (Kabat, E.A. et al., "Sequence of Proteins of Immunological Interest", US Dept. Health and Human Services, 1983).

In view of these facts, the homology in amino acid between the V-regions of the mouse monoclonal antibody against human PTHrP was determined with reference to the database of amino acid sequences of antibodies established by Kabat et al. Thus, the CDRs of the V-regions were determined as shown in Table 1.

The amino acid sequences of CDRs 1-3 in the L-chain V-region are shown in SEQ ID Nos: 59 to 61, respectively; and the amino acid sequences of CDRs 1-3 in the H-chain V-region are shown in SEQ ID Nos: 62 to 64, respectively.

**Table 1**

| V-region | SEQ ID NO. | CDR1 | CDR2 | CDR3 |
|---|---|---|---|---|
| H-chain V-region | 57 | 31-35 | 50-66 | 99-107 |
| L-chain V-region | 65 | 23-34 | 50-60 | 93-105 |

### [REFERENCE EXAMPLE 3] Construction of Chimeric Antibody

### (1) Construction of chimeric antibody H-chain

### (i) Construction of H-chain V-region

To ligate to an expression vector carrying a genomic DNA of human H-chain C-region C γ 1, the cloned DNA encoding mouse H-chain V-region was modified by PCR method. A backward primer MBC1-S1 (SEQ ID NO: 7) was designed to hybridize to a DNA sequence encoding the 5' region of the leader sequence of the V-region and to have both a Kozak consensus sequence (Kozak, M. et al., J. Mol. Biol., 196, 947-950, 1987) and a HindIII-recognition sequence. A forward primer MBC1-a (SEQ ID NO: 8) was designed to hybridize to a DNA sequence encoding the 3' region of the J region and to have both a donor splice sequence and a BamHI-recognition sequence. The PCR reaction was performed using TaKaRa Ex Taq (Takara Shuzo Co., Ltd.) and a buffer appended thereto. The PCR solution comprised (per 50µl) 0.07 µg of plasmid MBC1H04 as a template DNA, 50 pmoles of MBC1-a and 50 pmoles of MBC1-S1 as primers, 2.5U of TaKaRa Ex Taq and 0.25 mM dNTPs in the buffer, over which 50µl of mineral oil was layered. The PCR was run for 30 cycles under the conditions: 94° C for 1 min.; 55° C for 1 min.; 72° C for 2 min. The DNA fragments thus amplified by the PCR method were separated by agarose gel electrophoresis on a 3% Nu Sieve GTG Agarose (FMC Bio. Products).

Then, an agarose gel segment containing a DNA fragment of 437 bp was excised, and the DNA fragment was purified therefrom using GENECLEAN II Kit (BIO101) in accordance with the instructions included in the kit. The purified DNA was collected by ethanol precipitation, and then dissolved in 20 µl of a solution containing 10 mM Tris-HCl (pH 7.4) and 1 mM EDTA. An aliquot (1 µl) of the resulting DNA solution was digested with restriction enzymes BamHI and HindIII (Takara Shuzo Co., Ltd.) at 37° C for 1 hour. The digestion solution was extracted with phenol and chloroform and then precipitated with ethanol to collect the DNA of interest.

The obtained HindIII-BamHI DNA fragment, which containing a gene encoding the mouse H-chain V-region, was subcloned into pUC19 vector that had been digested with HindIII and BamHI. The resulting plasmid was sequenced on DNA Sequencer 373A (Perkin-Elmer) using M13 Primer M4 and M13 Primer RV as primers and Dye Terminator Cycle Sequencing Kit (Perkin-Elmer). As a result, a plasmid which carried a gene of correct nucleotide sequence encoding the mouse H-chain V-region derived from hybridoma #23-57-137-1 and had a HindIII-recognition sequence and a Kozak sequence on its 5' region and a BamHI-recognition sequence on its 3' region was obtained, which was designated as "MBC1H/pUC19".

### (ii) Construction of H-chain V-region for preparation of cDNA-type of mouse-human chimeric H-chain

To ligate to cDNA of the human H-chain C-region C γ 1, the DNA encoding the mouse H-chain V-region constructed as described above was modified by PCR method. A backward primer MBC1HVS2 (SEQ ID NO: 9) for the V-region was designed to cause the replacement of the second amino acid (asparagine) of the sequence encoding the front part of the leader sequence of the H-chain V-region by glycine and to have a Kozak consensus sequence (Kozak, M. et al., J. Mol. Biol., 196, 947-950, 1987) and HindIII- and EcoRI-recognition sequences. A forward primer MBC1HVR2 (SEQ ID NO: 10) for the H-chain V-region was designed to hybridize to a DNA sequence encoding the 3' region of the J region, to encoding the 5' region of the C-region and to have ApaI- and SmaI-recognition sequences.

The PCR reaction was performed using TaKaRa Ex Taq (Takara Shuzo Co., Ltd.) and a buffer appended thereto. The PCR solution comprised (per 50 µl) 0.6 µg of plasmid MBC1H/pUC19 as a template DNA, 50 pmoles of MBC1HVS2 and 50 pmoles of MBC1HVR2 as primers, 2.5U of TaKaRa Ex Taq and 0.25 mM of dNTPs in the buffer, over which 50µl of mineral oil was layered. The PCR reaction was run for 30 cycles under the conditions: 94° C for 1 min.; 55° C for 1 min.; 72° C for 1 min. The DNA fragments amplified by the PCR reaction were separated by agarose gel electrophoresis on a 1% Sea Kem GTG Agarose (FMC Bio. Products). Then, an agarose gel segment containing a DNA fragment of 456 bp was excised and the DNA fragment was purified therefrom using GENECLEAN II Kit (BIO101) in accordance with the instructions included in the kit. The purified DNA was precipitated with ethanol and then dissolved in 20 µl of a solution containing 10 mM Tris-HCl (pH 7.4) and 1 mM EDTA.

The resulting DNA solution (1 µg) was digested with restriction enzymes EcoRI and SmaI (Takara Shuzo Co., Ltd.) at 37° C for 1 hour. The digestion solution was extracted with phenol and chloroform and then precipitated with ethanol to collect the DNA. The obtained EcoRI-SmaI DNA fragment, which containing a gene encoding the mouse H-chain V-region, was subcloned into pUC19 vector that had been digested with EcoRI and Smal. The resulting plasmid was sequenced on DNA Sequencer 373A (Perkin-Elmer) using M13 Primer M4 and M13 Primer RV, and Dye Terminator Cycle Sequencing Kit (Perkin-Elmer). As a result, a plasmid which contained a gene of correct nucleotide sequence encoding mouse H-chain V-region derived from hybridoma #23-57-137-1 and had EcoRI- and HindIII-recognition sequences and a Kozak sequence on its 5' region and ApaI- and SmaI-recognition sequences on its 3' region was obtained, which was designated as "MBC1Hv/pUC19".

### (iii) Construction of expression vector for chimeric antibody H-chain

cDNA containing the DNA for human antibody H-chain C-region C γ 1 was prepared as follows. mRNA was prepared from a CHO cell into which both an expression vector DHFR-ΔE-RVh-PM-1-f (see WO 92/19759) encoding the genomic DNAs of humanized PM1 antibody H-chain V-region and human antibody H-chain C-region IgG1 (N. Takahashi et al., Cell 29, 671-679, 1982) and an expression vector RV1-PM1a (see WO 92/19759) encoding the genomic DNAs of humanized PM1 antibody L-chain V-region and human antibody L-chain κ chain C-region had been introduced. Using the mRNA, cDNA containing the humanized PM antibody H-chain V-region and the human antibody C-region C γ1 was cloned by RT-PCR method, and then subcloned into plasmid pUC19 at the HindIII-BamHI site. After sequencing, a plasmid which had the correct nucleotide sequence was obtained, which was designated as "pRVh-PM1f-cDNA".

An expression vector DHFR-ΔE-RVh-PM-1-f in which both a HindIII site located between SV40 promoter and a DHFR gene and an EcoRI site located between EF-1α promoter and a humanized PM1 antibody H-chain V-region gene had been deleted, was prepared for the construction of an expression vector for cDNA containing the humanized PM1 antibody H-chain V-region gene and the human antibody C-region C γ 1 gene.

The plasmid obtained (pRVh-PM1f-cDNA) was digested with BamHI, blunt-ended with Klenow fragment, and further digested with HindIII, thereby obtaining a blunt-ended HindIII-BamHI fragment. The blunt-ended HindIII-BamHI fragment was ligated to the above-mentioned HindIII site- and EcoRI site-deleted expression vector DHFR- ΔE-RVh-PM1-f that had been digested with HindIII and BamHI. Thus, an expression vector RVh-PM1f-cDNA was constructed which contained cDNA encoding the humanized PM1 antibody H-chain V-region and the human antibody C-region C γ 1.

The expression vector RVh-PM1f-cDNA containing the cDNA encoding the humanized PM1 antibody H-chain V-region and the human antibody C-region C γ 1 was digested with ApaI and BamHI, and a DNA fragment containing the H-chain C-region was collected therefrom. The resulting DNA fragment was introduced into the plasmid MBC1Hv/pUC19 that had been digested with ApaI and BamHI. The plasmid thus prepared was designated as "MBC1HcDNA/pUC19". This plasmid contained cDNA encoding the mouse antibody H-chain V-region and the human antibody C-region C γ 1, and had EcoRI- and HindIII-recognition sequences on its 5' region and a BamHI-recognition sequence on its 3' region.

The plasmid MBC1HcDNA/pUC19 was digested with EcoRI and BamHI to give a DNA fragment comprising a nucleotide sequence encoding the chimeric antibody H-chain. The resulting DNA fragment was introduced into an expression vector pCOS1 that had been digested with EcoRI and BamHI, thereby giving an expression vector for the chimeric antibody, which was designated as "MBC1HcDNA/pCOS1". Here, the expression vector pCOS1 was constructed using HEF-PMh-g γ1 (see WO 92/19759) by deleting therefrom an antibody genes by digestion with EcoRI and SmaI, and then ligating it to EcoRI-NotI-BamHI Adaptor (Takara Shuzo Co., Ltd.)

For preparing a plasmid for the expression in a CHO cell, the plasmid MBC1HcDNA/pUC19 was digested with EcoRI and BamHI to obtain a DNA fragment containing a gene for the chimeric antibody H-chain. The DNA fragment was then introduced into an expression plasmid pCHO1 that had been digested with EcoRI and BamHI to give an expression plasmid for the chimeric antibody, which was designated as "MBC1HcDNA/pCHO1". Here, the expression vector pCHO1 was constructed using DHFR-ΔE-rvH-PM1-f (see WO 92/19759) by deleting therefrom an antibody gene by digestion with EcoRI and SmaI, and then ligating it to EcoRI-NotI-BamHI Adaptor (Takara Shuzo Co., Ltd.)

### (2) Construction of human L-chain C-region

### (i) Preparation of cloning vector

To construct pUC19 vector containing a gene for human L-chain C-region, a HindIII site-deleted pUC 19 vector was prepared. pUC19 vector (2 µg) was digested in 20 µl of a reaction solution containing 20 mM Tris-HCl (pH 8.5), 10 mM MgCl₂, 1 mM DTT, 100 mM KCI, 8 U of HindIII (Takara Shuzo Co., Ltd.) at 37° C for 1 hour. The resulting digestion solution was extracted with phenol and chloroform, and then subjected to ethanol precipitation to collect the DNA of interest.

The DNA collected was reacted in 50 µl of a reaction solution containing 50 mM Tris-HCl (pH 7.5), 10 mM MgCl₂, 1 mM DTT, 100 mM NaCI, 0.5 mM dNTPs and 6U of Klenow fragment (GIBCO BRL) at room temperature for 20 min., thereby rendering the terminal ends of the DNA blunt. This reaction mixture was extracted with phenol and chloroform and then subjected to ethanol precipitation to collect the vector DNA.

The vector DNA thus collected was reacted in 10 µl of a reaction solution containing 50 mM Tris-HCl (pH 7.6), 10 mM MgCl₂, 1 mM ATP, 1 mM DTT, 5% (v/v) polyethylene glycol-8000 and 0.5 U of T4 DNA ligase (GIBCO BRL) at 16° C for 2 hours, to cause self-ligation of the vector DNA. The reaction solution (5 µl) was added to 100 µl of a solution containing competent cells of E. coli, JM109 (Nippon Gene Co., Ltd.), and the resulting solution was allowed to stand on ice for 30 min., at 42° C for 1 min., and additionally on ice for 1 min. SOC culture medium (500 µl) was added to the reaction solution and then incubated at 37° C for 1 hour. The resulting solution was plated on 2xYT agar medium (containing 50 µg/ml of ampicillin) on which X-gal and IPTG had been applied (Molecular Cloning: A Laboratory Manual, Sambrook, et al., Cold Spring Harbor Laboratory Press, 1989), and then cultured at 37° C overnight, thereby obtaining a transformant.

The transformant was cultured in 2xYT medium (20 ml) containing ampicillin (50 µg/ml) at 37° C overnight. From the cell fraction of the culture medium, a plasmid DNA was isolated and purified using Plasmid Mini Kit (QIAGEN) in accordance with the instructions included in the kit. The purified plasmid was digested with HindIII. The plasmid that was confirmed to have a HindIII site-deletion was designated as "pUC19 Δ HindIII".

### (ii) Construction of DNA encoding human L-chain λ chain C-region

Human antibody L-chain λ chain C-region is known to have at least four isotypes including Mcg⁺Ke⁺Oz⁻, Mcg⁻Ke⁻Oz⁻, Mcg⁻Ke⁻Oz⁺ and Mcg⁻Ke⁺Oz⁻ (P. Dariavach, et al., Proc. Natl. Acad. Sci. USA, 84, 9074-9078, 1987). A search was made for a human antibody L-chain λ chain C-region homologous to the #23-57-137-1 mouse L-chain λ chain C-region from the EMBL database. As a result, it was found that the isotype Mcg⁺Ke⁺Oz⁻ of the human antibody L-chain λ chain (Accession No. X57819) (P. Dariavach, et al., Proc. Natl. Acad. Sci. USA, 84, 9074-9078, 1987) showed the highest degree of homology to the #23-57-137-1 mouse L-chain λ chain C-region, with a 64.4% homology in terms of amino acid sequence and a 73.4% homology in terms of nucleotide sequence.

Then, a gene encoding the human antibody L-chain λ chain C-region was constructed by PCR method. The primers for the PCR were synthesized using 394 DNA/RNA Synthesizer (ABI). The synthesized primers were as follows: HLAMB1 (SEQ ID NO: 11) and HLAMB3 (SEQ ID NO: 13), both having a sense DNA sequence; and HLAMB2 (SEQ ID NO: 12) and HLAMB4 (SEQ ID NO: 14), both having an antisense DNA sequence; each primer containing a complementary sequence of 20-23 bp on the both terminal ends.

External primers HLAMBS (SEQ ID NO: 15) and HLAMBR (SEQ ID NO: 16) had sequences homologous to the primers HLAMB1 and HLAMB4, respectively. HLAMBS contained EcoRI-, HindIII- and BlnI-recognition sequences, and HLAMBR contained an EcoRI-recognition sequence. In the first-round PCR reaction, the reactions between HLAMB1 and HLAMB2 and between HLAMB3 and HLAMB4 were performed. After the reactions were completed, both of the resulting PCR products were mixed in equivalent quantities, and then assembled in the second-round PCR reaction. The reaction solution was added with the external primers HLAMBS and HLAMBR. This reaction mixture was subjected to the third-round PCR reaction to amplify the full length DNA.

Each PCR reaction was performed using TaKaRa Ex Taq (Takara Shuzo Co., Ltd.) in accordance with the instructions included in the kit. In the first-round PCR reaction, 100 µl of either a reaction solution containing 5 pmoles of HLAMB1, 0.5 pmole of HLAMB2 and 5U of TaKaRa Ex Taq (Takara Shuzo Co., Ltd.) or a reaction solution containing 0.5 pmole of HLAMB3, 5 pmoles of HLAMB4 and 5U of TaKaRa Ex Taq (Takara Shuzo Co., Ltd.) was used, over which 50 µI of mineral oil was layered. The PCR reaction was run for 5 cycles under the conditions: 94° C for 1 min., 60° C for 1 min. and 72° C for 1 min.

In the second-round PCR reaction, a mixture of both the reaction solutions (50 µl each) was used, over which 50 µl of mineral oil was layered. The PCR reaction was run for 3 cycles under the conditions: 94° C for 1 min., 60° C for 1 min. and 72° C for 1 min.

In the third-round PCR reaction, the reaction solution to which the external primers HLAMBS and HLAMBR (50 pmoles each) were added was used. The PCR reaction was run for 30 cycles under the conditions: 94° C for 1 min., 60° C for 1 min. and 72° C for 1 min.

The DNA fragment obtained by the third-round PCR reaction was subjected to electrophoresis on a 3% low-melting agarose gel (NuSieve GTG Agarose, FMC), and separated and purified from the gel using GENECLEAN II Kit (BIO101) in accordance with the instructions included in the kit.

The obtained DNA fragment was digested in a reaction solution (20 µl) containing 50 mM Tris-HCl (pH 7.5), 10 mM MgCl₂, 1 mM DTT, 100 mM NaCl and 8U of EcoRI (Takara Shuzo Co., Ltd.) at 37° C for 1 hour. The digestion solution was extracted with phenol and chloroform, and the DNA was collected therefrom by the ethanol precipitation. The DNA was dissolved in a solution (8 µl) containing 10 mM Tris-HCl (pH 7.4) and 1 mM EDTA.

The above-prepared plasmid pUC19 ΔHindIII (0.8 µg) was digested with EcoRI in the same manner as set forth above. The digestion solution was subjected to phenol/chloroform extraction and then ethanol precipitation, thereby giving a digested plasmid pUC19 ΔHindIII. The digested plasmid was reacted in a reaction solution (50 µl) containing 50 mM Tris-HCl (pH 9.0), 1 mM MgCl₂ and alkaline phosphatase (E. coli C75; Takara Shuzo Co., Ltd.) at 37° C for 30 min. to dephosphorylate (i.e., BAP-treat) the plasmid. The reaction solution was subjected to phenol/chloroform extraction, and the DNA was collected therefrom by ethanol precipitation. The DNA thus obtained was dissolved in a solution (10 µl) containing 10 mM Tris-HCl (pH 7.4) and 1 mM EDTA.

The BAP-treated plasmid pUC19 ΔHindIII (1 µl) was ligated to the above-obtained PCR product (4 µl) using DNA Ligation Kit Ver.2 (Takara Shuzo Co., Ltd.). The resulting plasmid was introduced into a competent cell of E. coli, JM109, to give a transformant. The transformant was cultured overnight in 2xYT medium (2 ml) containing 50 µg/ml of ampicillin. From the cell fraction, the plasmid was isolated using QIAprep Spin Plasmid Kit (QIAGEN).

The obtained plasmid was sequenced for the cloned DNA part. The sequencing was performed on 373A DNA Sequencer (ABI) using M13 Primer M4 and M13 Primer RV (Takara Shuzo Co., Ltd.). As a result, it was found that the cloned DNA had a 12-bp deletion therein. The plasmid was designated as "Cλ Δ/pUC19". Then, for making up for the deleted part, primers HCLMS (SEQ ID NO: 17) and HCLMR (SEQ ID NO: 18) were newly synthesized, and a DNA of correct sequence was reconstructed using these primers by PCR method.

In the first-round PCR reaction, the plasmid C λΔ/pUC19 having the DNA deletion therein was used as a template, and the reaction was performed with each of the primer sets of HLAMBS and HCLMS and HCLMS and HLAMB4. The PCR products were purified separately. In the second-round PCR reaction, the PCR products were assembled together. In the third-round PCR reaction, the reaction product of the second-round PCR reaction was added with external primers HLAMBS and HLAMB4 and amplified to give the full length DNA.

In the first-round PCR reaction, a reaction solution (100 µl) containing 0.1 µg of Cλ Δ/pUC19 as a template, either 50 pmoles of each of the primers HLAMBS and HCLMR or 50 pmoles of each of the primers HCLMS and HLAMB4, and 5U of TaKaRa Ex Taq (Takara Shuzo Co., Ltd.) was used, over which 50 µl of mineral oil was layered. The PCR reaction was run for 30 cycles under the conditions: 94° C for 1 min., 60° C for 1 min. and 72 ° C for 1 min.

The PCR products of the first-round PCR reaction, HLAMBS-HCLMR (236 bp) and HCLMS-HLAMB4 (147 bp), were subjected to electrophoresis separately on a 3% low-melting agarose gel to isolate the DNA fragments. The DNA fragments were collected and purified from the gels using GENECLEAN II Kit (BIO101). In the second-round PCR reaction, 20 µl of a reaction solution containing 40 ng of each of the purified DNA fragments and 1U of TaKaRa Ex Taq (Takara Shuzo Co., Ltd.) was used, over which 25 µl of mineral oil was layered. The PCR reaction was run for 5 cycles under the conditions: 94° C for 1 min., 60° C for 1 min. and 72° C for 1 min.

In the third-round PCR reaction, 100 µl of a reaction solution containing 2 µl of the reaction solution obtained by the second-round PCR reaction, 50 pmoles of each of external primers HLAMBS and HLAMB4 and 5U of TaKaRa Ex Taq (Takara Shuzo Co., Ltd.) was used, over which 50 µl of mineral oil was layered. The PCR reaction was run for 30 cycles under the conditions: 94° C for 1 min., 60° C for 1 min. and 72° C for 1 min., thereby obtaining a DNA fragment of 357 bp (the third PCR product). The DNA fragment was subjected to electrophoresis on a 3% low-melting agarose gel to isolate the DNA fragment. The resulting DNA fragment was collected and purified using GENECLEAN Kit (BIO101).

An aliquot (0.1 µg) of the DNA fragment thus obtained was digested with EcoRI, and then subcloned into plasmid pUC19 ΔHindIII that had been BAP-treated. The resulting plasmid was introduced into a competent cell of E. coli, JM109, to form a transformant. The transformant was cultured overnight in 2 ml of 2xYT medium containing 50 µg/ml of ampicillin. From the cell fraction, the plasmid was isolated and purified using QIAprep Spin Plasmid Kit (QIAGEN).

The purified plasmid was sequenced on 373A DNA Sequencer (ABI) using M13 Primer M4 and M13 Primer RV (Takara Shuzo Co., Ltd.). The plasmid that was confirmed to have the correct nucleotide sequence without any deletion was designated as "Cλ/pUC19".

### (iii) Construction of gene encoding human L-chain κ chain C-region

A DNA fragment encoding the L-chain κ chain C-region was cloned from plasmid HEF-PM1k-gk (WO 92/19759) by PCR method. A forward primer HKAPS (SEQ ID NO: 19) was designed to contain EcoRI-, HindIII and BlnI-recognition sequences, and a backward primer HKAPA (SEQ ID NO: 20) was designed to contain an EcoRI-recognition sequence. These primers were synthesized on 394 DNA/RNA Synthesizer (ABI).

A PCR reaction was performed using 100 µl of a reaction solution containing 0.1µ g of plasmid HEF-PM1k-gk as a template, 50 pmoles of each of primers HKAPS and HKAPA and 5U of TaKaRa Ex Taq (Takara Shuzo Co., Ltd.), over which 50 µl of mineral oil was layered. The PCR reaction was run for 30 cycles under the conditions: 94° C for 1 min., 60 ° C for 1 min. and 72° C for 1 min., thereby giving a PCR product of 360 bp. The DNA fragment was isolated and purified by electrophoresis on a 3% low-melting agarose, and then collected and purified using GENECLEAN II Kit (BIO101).

The thus obtained DNA fragment was digested with EcoRI, and then cloned into plasmid pUC19 (HindIII that had been BAP-treated. The resulting plasmid was introduced into a competent cell of E. coli, JM109, to form a transformant. The transformant was cultured overnight in 2 ml of 2xYT medium containing 50 µg/ml of ampicillin. From the cell fraction, the plasmid was purified using QIAprep Spin Plasmid Kit (QIAGEN).

The purified plasmid was sequenced on 373A DNA Sequencer (ABI) using M13 Primer M4 and M13 Primer RV (Takara Shuzo Co., Ltd.). The plasmid that was confirmed to have the correct nucleotide sequence was designated as "C κ/pUC19".

### (3) Construction of chimeric antibody L-chain expression vector

An expression vector for the chimeric #23-57-137-1 antibody L-chain was constructed. A gene encoding #23-57-137-1 L-chain V-region was ligated to the HindIII-BlnI site (located just in front of the human antibody C-region) of each of the plasmids C λ/pUC19 and C κ/pUC19, thereby obtaining pUC19 vectors that contained the DNAs encoding the chimeric #23-57-137-1 antibody L-chain V-region and either of the L-chain λ chain C-region or the L-chain κ region C-region, respectively. Each of the resulting vectors was then digested with EcoRI to separate the gene for the chimeric antibody L-chain. The gene was subcloned into HEF expression vector.

That is, a DNA fragment encoding #23-57-137-1 antibody L-chain V-region was cloned from plasmid MBC1L24 by PCR method. Primers used in the PCR method were separately synthesized using 394 DNA/RNA Synthesizer (ABI). A backward primer MBCCHL1 (SEQ ID NO: 21) was designed to contain a HindIII-recognition sequence and a Kozak sequence (Kozak, M. et al., J. Mol. Biol. 196, 947-950, 1987), and a forward primer MBCCHL3 (SEQ ID NO: 22) was designed to contain BglII- and RcoRI-recognition sequences.

The PCR reaction was performed using 100µl of a reaction solution containing 10 mM Tris-HCl (pH 8.3), 50 mM KCl, 1.5 mM MgCl₂, 0.2 mM dNTPs, 0.1 µg MBC1L24, 50 pmoles of each of primers MBCCHL1 and MBCCHL3 and 1 µl of AmpliTaq (PERKIN ELMER), over which 50 µl of mineral oil was layered. The PCR reaction was run for 30 cycles under the conditions: 94° C for 45 sec., 60° C for 45 sec. and 72° C for 2 min.

A PCR product of 444 bp was electrophoresed on a 3% low-melting agarose gel, and collected and purified using GENECLEAN II Kit (BIO101). The purified PCR product was dissolved in 20 µl of a solution containing 10 mM Tris-HCl (pH 7.4) and 1 mM EDTA. The PCR product (1 µl) was digested in 20 µl of a reaction solution containing 10 mM Tris-HCl (pH 7.5), 10 mM MgCl₂, 1 mM DTT, 50 mM NaCl, 8U of HindIII (Takara Shuzo Co., Ltd.) and 8U of EcoRI (Takara Shuzo Co., Ltd.) at 37° C for 1 hour. The digestion solution was subjected to phenol/chloroform extraction, and the DNA of interest was collected therefrom by ethanol precipitation. The DNA was dissolved in 8 µl of a solution containing 10 mM Tris-HCl (pH 7.4) and 1 mM EDTA.

In the same manner, plasmid pUC19 (1 µg) was digested with HindIII and EcoRI, and subjected to phenol/chloroform extraction and then ethanol precipitation. The obtained digested plasmid was BAP-treated with alkaline phosphatase (E. coli C75; Takara Shuzo Co., Ltd.). The resulting reaction solution was extracted with phenol and chloroform, and the DNA was collected therefrom by ethanol precipitation. The DNA was dissolved in 10 µl of a solution containing 10 mM Tris-HCl (pH 7.4) and 1 mM EDTA.

The BAP-treated plasmid pUC19(1 µl) was ligated to the above-obtained PCR product (4 µl) using DNA Ligation Kit Ver. 2 (Takara Shuzo Co., Ltd.). The resulting plasmid was introduced into a competent cell of E. coli, JM109 (Nippon Gene Co., Ltd.), in the same manner as set forth above, to form a transformant. The transformant was plated on 2xYT agar medium containing 50 µg/ml of ampicillin and cultured at 37° C overnight. The resulting transformant was then cultured at 37° C overnight in 2 ml of 2xYT medium containing 50 µg/ml of ampicillin. From the cell fraction, the plasmid was purified using QIAprep Spin Plasmid Kit (QIAGEN). After determining the nucleotide sequence, the plasmid that was confirmed to have the correct nucleotide sequence was designated as "CHL/pUC19".

Each of plasmids Cλ/pUC19 and C κ/pUC19 (1 µg each) was digested in 20 µl of a reaction solution containing 20 mM Tris-HCl (pH 8.5), 10 mM MgCl₂, 1 mM DTT, 100 mM KCl, 8U of HindIII (Takara Shuzo Co., Ltd.) and 2U of BlnI (Takara Shuzo Co., Ltd.) at 37° C for 1 hour. The digestion solution was extracted with phenol and chloroform, and the DNA was collected therefrom by ethanol precipitation. The DNA was BAP-treated at 37° C for 30 min. The reaction solution was extracted with phenol and chloroform, and the DNA was collected therefrom by ethanol precipitation. The DNA was dissolved in 10 µl of a solution containing 10 mM Tris-HCI (pH 7.4) and 1 mM EDTA.

The plasmid CHL/pUC19 (8 µg) that contained DNA encoding #23-57-137-1 L-chain V-region was digested with HindIII and BlnI in the same manner as set forth above to give a DNA fragment of 409 bp. The DNA fragment was electrophoresed on a 3% low-melting agarose gel, and then collected and purified from the gel using GENECLEAN II Kit (BIO101). The DNA was dissolved in 10 µl of a solution containing 10 mM Tris-HCI (pH 7.4) and 1 mM EDTA.

The DNA for L-chain V-region DNA (4 µl) was subcloned into 1 µI of each of the BAP-treated plasmids Cλ/pUC19 and C κ/pUC19, and then introduced into a competent cell of E. coli, JM109, to form a transformant. The transformant was cultured overnight in 3 ml of 2xYT medium containing 50 µg/ml of ampicillin. From the cell fraction, the plasmid was isolated and purified using QIAprep Spin Plasmid Kit (QIAGEN). The two plasmids thus prepared were designated as "MBC1L(λ)/pUC19" and "MBC1L(κ)/pUC19", respectively.

Each of plasmids MBC1L(λ)/pUC19 and MBC1L(κ)/pUC19 was digested with EcoRI and then subjected to electrophoresis on a 3% low-melting agarose gel. A DNA fragment of 743 bp was isolated and purified from the gel using GENECLEANII Kit (BIO101), and then dissolved in 10 µl of a solution containing 10 mM Tris-HCl (pH 7.4) and 1 mM EDTA.

An expression vector (plasmid HEF-PM1k-gk) (2.7 µg) was digested with EcoRI and then extracted with phenol and chloroform, and the DNA was collected therefrom by ethanol precipitation. The DNA fragment was BAP-treated, and then subjected to electrophoresis on a 1% low-melting agarose gel. From the gel, a DNA fragment of 6561 bp was isolated and purified therefrom using GENECLEANII Kit (BIO101). The purified DNA fragment was dissolved in 10 µl of a solution containing 10 mM Tris-HCl (pH 7.4) and 1 mM EDTA.

BAP-treated HEF vector (2 µl) was ligated to an EcoRI fragment (3 µl) of each of plasmid MBC1L(λ)/pUC19 and MBC1L(κ)/pUC19. The ligation product was introduced into a competent cell of E. coli, JM 109, to form a transformant. The transformant was cultured in 2 ml of 2xYT medium containing 50 µg/ml of ampicillin. From the cell fraction, the plasmid was purified using QIAprep Spin Plasmid Kit (QIAGEN).

The purified plasmid was digested in 20 µl of a reaction solution containing 20 mM Tris-HCl (pH 8.5), 10 mM MgCl₂, 1 mM DTT, 100 mM KCI, 8U of HindIII (Takara Shuzo Co., Ltd.) and 2 U of PvuI (Takara Shuzo Co., Ltd.) at 37° C for 1 hour. This reaction gave digestion fragments of 5104/2195 bp if the fragment was inserted in the correct orientation, or gave digestion fragments of 4378/2926 bp if the fragment was inserted in the reverse orientation. The plasmid that was confirmed to have the fragment in the correct orientation was designated as "MBC1L(λ)/neo" for plasmid MBC1L(λ)/pUC19 or "MBC1L(κ)/neo" for plasmid MBC1L(κ)/pUC19.

### (4) Transfection of COS-7 cell

To evaluate the antigen-binding activity and the neutralizing activity of the chimeric antibodies, the expression plasmids prepared above were separately expressed transiently in a COS-7 cell.

The transient expression of the chimeric antibodies was performed using each of the combinations of plasmids MBC1HcDNA/pCOS1 and MBC1L (λ)/neo and plasmids MBC1HcDNA/pCOS1 and MBC1L(κ )/neo, by co-tansfecting a COS-7 cell with the plasmids by electroporation using Gene Pulser (Bio Rad). That is, the plasmids (10 µg each) were added to a COS-7 cell suspension (0.8 ml; 1 x 10⁷ cells/ml) in PBS(-). The resulting solution was applied with pulses at an electrostatic capacity of 1,500V and 2 µF to cause electroporation. After 10 min. of recovery period at room temperature, the electroporated cells were suspended in DMEM medium (GIBCO) containing 2% Ultra Low IgG fetal calf serum (GIBCO), and then cultured using a 10-cm culture dish in a CO₂ incubator. After culturing for 72 hours, a culture supernatant was collected and centrifuged to remove cell debris, and was provided for use as a sample for the subsequent ELISA.

In this procedure, the purification of the chimeric antibody from the COS-7 cell culture supernatant was performed using AffiGel Protein A MAPSII Kit (Bio Rad) in accordance with the instructions included in the kit.

### (5) ELISA

### (i) Determination of antibody concentration

An ELISA plate for determining antibody concentration was prepared as follows. Each well of a 96-well ELISA plate (Maxisorp, NUNC) was coated with 100 µl of a coating buffer (0.1 M NaHCO₃, 0.02% NaN₃) supplemented with 1 µg/ml of goat anti-human IgG antibody (TAGO), and then blocked with 200 µl of a dilution buffer [50 mM Tris-HCl, 1 mM MgCl₂, 0.1 M NaCl, 0.05% Tween 20, 0.02% NaN₃, 1% bovine serum albumin (BSA); pH 7.2]. Each well of the plate was added with each of the serial dilutions of the COS-7 cell culture supernatant in which each of the chimeric antibodies had been expressed, or added with each of the serial dilutions of each of the chimeric antibodies per se in a purified form. The plate was incubated at room temperature for 1 hour and washed with PBS-Tween 20. Each well of the plate was then added with 100 µl of a solution of alkaline phosphatase-conjugated goat anti-human IgG antibodies (TAGO). After the plate was incubated at room temperature for 1 hour and washed with PBS-Tween 20, each well was added with 1 mg/ml of a substrate solution ("Sigma 104", p-nitrophenylphosphoric acid, SIGMA). The solution was measured on its absorbance at 405 nm using Microplate Reader (Bio Rad) to determine the antibody concentration. In this determination, Hu IgG1 λ Purified (The Binding Site) was used as the standard substance.

### (ii) Determination of antigen-binding ability

An ELISA plate for the determination of antigen-binding ability was prepared as follows. Each well of a 96-well ELISA plate was coated with 100 µl of a coating buffer supplemented with 1 µg/ml of human PTHrP (1-34) (Peptide Research Institute), and then blocked with 200 µl of a dilution buffer. Each well was added with each of the serial dilutions of the COS-7 cell culture supernatant in which each of the chimeric antibodies had been expressed, or added with each of the serial dilutions of each of the chimeric antibodies per se in a purified form. After the plate was incubated at room temperature and washed with PBS-Tween 20, each well of the plate was added with 100 µl of a solution of alkaline phosphatase-conjugated goat anti-human IgG antibodies (TAGO). After the plate was incubated at room temperature and washed with PBS-Tween 20, each well of the plate was added with 1 mg/ml of a substrate solution ("Sigma 104", p-nitrophenylphosphoric acid, SIGMA). The solution was measured on its absorbance at 405 nm using Microplate Reader (Bio Rad).

As a result, it was found that the chimeric antibodies had an ability to bind to human PTHrP (1-34) and the cloned mouse antibody V-regions had the correct structures (FIG. 5). It was also found that there was no difference in the ability to bind to PTHrP (1-34) between the chimeric antibody with L-chain λ chain C-region and the chimeric antibody with L-chain κ chain C-region. Therefore, the humanized antibody L-chain λ chain was used for construction of the L-chain C-region of the humanized antibody.

### (6) Establishment of CHO cell line capable of stable production of chimeric antibodies

To establish a cell line capable of producing the chimeric antibodies stably, the above-prepared expression plasmids were introduced into CHO cells (DXB11).

For the establishment of a cell line capable of producing the chimeric antibodies stably, either of the following combinations of the expression plasmids for CHO cell was used: MBC1HcDNA/pCHO1 and MBC1L(λ)/neo; and MBC1HcDNA/pCHO1 and MBC1L(κ)/neo. A CHO cell was co-transfected with the plasmids by electroporation using Gene Pulser (Bio Rad) as follows. The expression vectors were separately cleaved with a restriction enzyme PvuI to give linear DNAs. The resulting DNAs were extracted with phenol and chloroform and collected by precipitation with ethanol. The plasmid DNAs thus prepared were subjected to electroporation. That is, each of the plasmid DNAs (10 µg each) was added to 0.8 ml of a cell suspension of CHO cells in PBS(-) (1x10⁷ cells/ml). The resulting solution was applied with pulses at an electrostatic capacity of 1,500V and 25 µF. After 10 min. of recovery period at room temperature, the electroporated cells were suspended in MEM-α medium (GIBCO) containing 10% fetal calf serum (GIBCO). The resulting suspension was cultured using three 96-well plates (Falcon) in a CO₂ incubator. On the day following the culturing being started, the medium was replaced by a selective medium [ribonucleoside- or deoxyribonucleoside-free MEM- α medium (GIBCO) containing 10% fetal calf serum (GIBCO) and 500 mg/ml of GENETICIN (G418Sulfate; GIBCO)]. From the culture medium, cells into which the antibody gene was introduced were selected. The selective medium is replaced by a fresh one. About two weeks after the medium replacement, the cells were observed under a microscope. When a satisfactory cell growth was observed, the amount of the antibodies produced was determined by ELISA as set forth above. Among the cells, those cells which produced a larger amount of antibodies were screened.

Then, the culturing of the established cell line capable of stable production of the antibodies was scaled up in a roller bottle using ribonucleoside- or deoxyribonucleoside-free MEM medium containing 2% Ultra Low IgG fetal calf serum. On day 3 and day 4 of the culturing, the culture supernatant was collected and then filtered on a 0.2-µm filter (Millipore) to remove cell debris therefrom.

Purification of the chimeric antibodies from the CHO cell culture supernatant was performed using POROS Protein A Column (PerSeptive Biosystems) on ConSep LC100 (Millipore) in accordance with the instructions included in the kit. The purified chimeric antibodies were provided for use as samples for the determination of neutralizing activity and for the examination of therapeutic efficacy in hypercalcemic model animals. The concentration and the antigen-binding activity of the purified chimeric antibodies were determined using the same ELISA system as set forth above.

### [REFERENCE EXAMPLE 4] Construction of humanized antibody

### (1) Construction of humanized antibody H-chain

### (i) Construction of humanized H-chain V-region

A humanized #23-57-137-1 antibody H-chain was produced by CDR-grafting technique by means of PCR method. For the production of a humanized #23-57-137-1 antibody H-chain (version "a") having FRs derived from human antibody S31679 (NBRF-PDB; Cuisinier, A. M. et al., Eur. J. Immunol., 23, 110-118, 1993), the following six PCR primers were used: CDR-grafting primers: MBC1HGP1 (SEQ ID NO: 23) and MBC1HGP3 (SEQ ID NO: 24) (both containing a sense DNA sequence) and MBC1HGP2 (SEQ ID NO: 25) and MBC1HGP4 (SEQ ID NO: 26) (both containing an antisense DNA sequence), all of which containing a 15-21 bp complementary sequence on both terminal ends thereof; and external primers: MBC1HVS1 (SEQ ID NO: 27) and MBC1HVR1 (SEQ ID NO: 28) having a homology to the CDR-grafting primers MBC1HGP1 and MBC1HGP4, respectively.

The CDR-grafting primers MBC1HGP1, MBC1HGP2, MBC1HGP3 and MBC1HGP4 were separated on an urea-denatured polyacrylamide gel (Molecular Cloning: A Laboratory Manual, Sambrook, et al., Cold Spring Harbor Laboratory Press, 1989), and extracted therefrom by crush-and-soak method (Molecular Cloning: A Laboratory Manual, Sambrook, et al., Cold Spring Harbor Laboratory Press, 1989) in the following manner.

Each of the CDR-grafting primers (1 nmole) was separated on a 6% denatured polyacrylamide gel to give DNA fragments. From the resulting DNA fragments, a DNA fragment having a desired length was identified on a silica gel thin plate by irradiation of UV ray and then collected therefrom by crush-and-soak method. The resulting DNA was dissolved in 20 µl of a solution containing 10 mM Tris-HCl (pH 7.4) and 1 mM EDTA. The PCR reaction was performed using TaKaRa Ex Taq (Takara Shuzo Co., Ltd.). The PCR reaction solution(100 µl) comprised 1 µl of each of the above-mentioned CDR-grafting primers MBC1HGP1, MBC1HGP2, MBC1HGP3 and MBC1HGP4, 0.25 mM dNTPs and 2.5U of TaKaRa Ex Taq in the buffer. The PCR reaction was run for 5 cycles under the conditions: 94° C for 1 min., 55° C for 1 min. and 72° C for 1 min. The resulting reaction solution was added with the external primers MBC1HVS1 and MBC1HVR1 (50 pmoles each). Using this reaction mixture, the PCR reaction was run for additional 30 cycles under the same conditions. The DNA fragment thus amplified was separated by agarose gel electrophoresis on a 4% Nu Sieve GTG agarose (FMC Bio. Products).

An agarose segment containing a DNA fragment of 421 bp was excised, and the DNA fragment was purified therefrom using GENECLEANII Kit (BIO101) in accordance with the instructions included in the kit. The DNA fragment thus purified was precipitated with ethanol and then dissolved in 20 µl of a solution containing 10 mM Tris-HCl (pH 7.4) and 1 mM EDTA. The resulting PCR reaction mixture was used for subcloning of the DNA fragment into plasmid pUC19 that had been digested with BamHI and HindIII, and subsequently the nucleotide sequence of the resulting plasmid was determined. A plasmid having the correct nucleotide sequence was designated as "hMBCHv/pUC19".

### (ii) Construction of H-chain V-region of Humanized H-chain cDNA

To ligate to cDNA for humanized H-chain C-region Cγ1, the DNA for the humanized H-chain V-region constructed in the above step was modified by PCR method. For the PCR method, a backward primer MBC1HVS2 was designed to hybridize to the sequence encoding the 5' region of the leader sequence for the V-region and to have a Kozak consensus sequence (Kozak et al., J. Mol. Biol. 196, 947-950, 1987) and HindIII- and EcoRI-recognition sequences; and a forward primer MBC1HVR2 was designed to hybridize to both the DNA sequence encoding the 3' region of the J region and the DNA sequence encoding the 5' region of the C-region and to have ApaI- and SmaI-recognition sequences.

The PCR reaction was performed using TaKaRa Ex Taq (Takara Shuzo Co., Ltd.) and a buffer appended thereto. The PCR reaction solution comprised 0.4 µg of hMBCHv/pUC19 as a DNA template, 50 pmoles of each of MBC1HVS2 and MBC1HVR2 as primers, 2.5U of TaKaRa Ex Taq and 0.25 mM dNTPs in the buffer. The PCR reaction was run for 30 cycles under the conditions: 94° C for 1 min., 55° C for 1 min. and 72° C for 1 min. The DNA fragment thus amplified was separated by agarose gel electrophoresis on a 3% Nu Sieve GTG agarose (FMC Bio. Products).

A gel segment containing a DNA fragment of 456 bp was excised, and the DNA fragment was purified therefrom using GENECLEANII Kit (BIO101) in accordance with the instructions included in the kit. The DNA fragment thus purified was precipitated with ethanol and then dissolved in 20 µl of a solution containing 10 mM Tris-HCl (pH 7.4) and 1 mM EDTA. The PCR reaction solution thus obtained was used for subcloning of the DNA fragment into plasmid pUC19 that had been digested with EcoRI and Smal, and then the resulting plasmid was sequenced. As a result, a plasmid was obtained which contained a DNA encoding mouse H-chain V-region derived from hybridoma #23-57-137-1 and also contained EcoRI- and HindIII-recognition sequences and a Kozak sequence on the 5' region and ApaI- and SmaI-recognition sequences on the 3' region, which was designated as "hMBC1Hv/pUC19".

### (2) Construction of expression vector for humanized antibody H-chain

Plasmid RVh-PM1f-cDNA carrying a cDNA sequence for hPM1 antibody H-chain was digested with ApaI and BamHI to give a DNA fragment containing a DNA fragment containing a DNA encoding the H-chain C-region. The DNA fragment was introduced into plasmid hMBC1Hv/pUC19 that had been digested with ApaI and BamHI. The obtained plasmid was designated as "hMBC1HcDNA/pUC19". This plasmid contained both a DNA encoding the humanized #23-57-137-1 antibody H-chain V-region and a DNA encoding the human H-chain C-region C γ 1 and had EcoRI- and HindIII-recognition sequences on the 5' region and a BamHI-recognition sequence on the 3' region. The nucleotide sequence and the corresponding amino acid sequence of the humanized H-chain version "a" carried on the plasmid hMBC1HcDNA/pUC19 are shown in SEQ ID NO: 58 and SEQ ID NO: 56, respectively.

The plasmid hMBC1HcDNA/pUC19 was digested with EcoRI and BamHI to give a DNA fragment containing a DNA encoding the H-chain. The DNA fragment was introduced into expression plasmid pCOS1 that had been digested with EcoRI and BamHI. As a result, an expression plasmid for a humanized antibody was obtained, which was designated as "hMBC1HcDNA/pCOS1".

To produce a plasmid used for expression in a CHO cell, plasmid hMBC1HcDNA/pUC19 was digested with EcoRI and BamHI to give a DNA fragment containing a DNA encoding the H-chain. The DNA fragment was introduced into expression vector pCHO1 that had been digested with EcoRI and BamHI. As a result, an expression plasmid for the humanized antibody was obtained, which was designated as "hMBC1HcDNA/pCHO1".

### (3) Construction of L-chain hybrid V-region

### (i) Preparation of FR1,2/FR3,4 hybrid antibody

A gene for the FR hybrid L-chain having both FRs from a humanized antibody and FRs from a mouse (chimeric) antibody was constructed, and evaluated each region for the humanization. In this step, a hybrid antibody having FR1 and FR2 both derived from a human antibody and FR3 and FR4 both derived from a mouse antibody was prepared by utilizing the AflII restriction site located on CDR2.

Plasmids MBC1L(λ)/neo and hMBC1L(λ)/neo (10 µg each) were separately digested in 100 µl of a reaction solution containing 10 mM Tris-HCl (pH 7.5), 10 mM MgCl₂, 1 mM DTT, 50 mM NaCl, 0.01% (w/v) of BSA and 10 U of AflII (Takara Shuzo Co., Ltd.) at 37° C for 1 hour. The reaction solutions were subjected to electrophoresis on a 2% low-melting agarose gel, thereby giving DNA fragments of 6282 bp (referred to as "c1") and 1022 bp (referred to as "c2") from the plasmid MBC1L(λ)/neo or DNA fragments of 6282 bp (referred to as "h1") and 1022 bp (referred to as "h2") from the plasmid hMBC1L(λ)/neo. These DNA fragments were collected and purified from the gels using GENECLEANII Kit (BIO101).

Each of the c1 and h1 fragments (1 µg each) was BAP-treated. The DNA fragment was extracted with phenol and chloroform, collected by ethanol precipitation, and then dissolved in 10 µl of a solution containing 10 mM Tris-HCl (pH 7.4) and 1 mM EDTA.

The BAP-treated c1 and h1 DNA fragments (1 µl each) were ligated to the h2 and c2 DNA fragments (4 µl each), respectively, (at 4° C overnight). Each of the ligation products was introduced into a competent cell of E. coli, JM109, to form a transformant. The transformant was cultured in 2 ml of 2xYT medium containing 50 µg/ml of ampicillin. From the cell fraction, the plasmid was purified using QIAprep Spin Plasmid Kit (QIAGEN).

The purified plasmid was digested in 20 µl of a reaction solution containing 10 mM Tris-HCl (pH 7.5), 10 mM MgCl₂, 1 mM DTT, and either 2U of ApaLI (Takara Shuzo Co., Ltd.) or 8U of BamHI (Takara Shuzo Co., Ltd.) and HindIII (Takara Shuzo Co., Ltd.) at 37° C for 1 hour. It was expected that if the c1-h2 was ligated correctly, this digestion reaction would give fragments of 5560/1246/498 bp (by the ApaLI digestion) or fragments of 7134/269 bp (by the BamHI/HindIII digestion). Based on this expectation, the desired plasmids were identified.

The expression vector encoding the human FR1,2/mouse FR3,4 hybrid antibody L-chain was designated as "h/mMBC1L(λ)/neo". On the other hand, since a clone for the h1-c1 could not be obtained, recombination on a pUC vector was performed and then the resulting recombinant product was cloned into a HEF vector. In this procedure, plasmid hMBC1Laλ /pUC19, which contained DNA encoding a humanized antibody L-chain V-region without any amino acid replacements, and plasmid hMBC1Ld λ/pUC19, which contained a DNA encoding a humanized antibody L-chain V-region with an amino acid replacement at the 91-position amino acid tyrosine in FR3 (i.e., the 87th amino acid in accordance with The Kabat's prescription) by isoleucine, were used as templates.

Plasmids MBC1L(λ)/pUC19, hMBC1La λ/pUC19 and hMBC1Ld λ/pUC19 (10µl each) were separately digested in 30 µl of a reaction solution containing 10 mM Tris-HCl (pH 7.5), 10 mM MgCl₂, 1 mM DTT, 50 mM NaCl, 0.01% (w/v) of BSA, 16U of HindIII and 4U of AflII at 37° C for 1 hour. The reaction solutions were separately subjected to electrophoresis on a 2% low-melting agarose gel, thereby giving a DNA fragment of 215 bp from plasmid MBC1L(λ)/pUC19 (referred to as "c2"') and a DNA fragment of 3218 bp from each of plasmids hMBC1La λ/pUC19 and hMBC1Ld λ/pUC19 (referred to as "ha1"' and "hd1"', respectively). These DNA fragments were collected and purified using GENECLEANII Kit (BIO101).

Each of the hal' and hdl' fragments was ligated to the c2' fragment and then introduced into a competent cell of E. coli, JM109, to form a transformant. The transformant was cultured in 2 ml of 2xYT medium containing 50 µg/ml of ampicillin. From the cell fraction, the plasmid was purified using QIAprep Spin Plasmid Kit (QIAGEN). The plasmids thus prepared were designated as "m/hMBC1La λ/pUC19" for the hal' fragment-containing plasmid and "m/hMBC1Ld λ/pUC19" for the hdl' fragment-containing plasmid.

Each of the obtained plasmids m/hMBC1La λ/pUC19 and m/hMBC1Ld λ/pUC19 was digested with EcoRI. The DNA fragment of 743 bp was electrophoresed on a 2% low-melting agarose gel, and then collected and purified therefrom using GENECLEANII Kit (BIO101). The resulting DNA fragment was dissolved in 20 µl of a solution containing 10 mM Tris-HCl (pH 7.4) and 1 mM EDTA.

Each of the DNA fragments (4 µl each) was ligated to the above-obtained BAP-treated HEF vector (1 µl). The ligation product was introduced into a competent cell of E. coli, JM109, to form a transformant. The transformant was cultured in 2 ml of 2xYT medium containing 50 µg/ml of ampicillin. From the cell fraction, the plasmid was purified using QIAprep Spin Plasmid Kit (QIAGEN).

Each of the purified plasmids was digested in 20 µl of a reaction solution containing 20 mM Tris-HCl (pH 8.5), 10 mM MgCl₂, 1 mM DTT, 100 mM KCI, 8U of HindIII (Takara Shuzo Co., Ltd.) and 2U of PvuI (Takara Shuzo Co., Ltd.) at 37° C for 1 hour. It was expected that if the DNA fragment was inserted in the plasmid in a correct orientation, this digestion would give digestion fragments of 5104/2195 bp, whereas if the DNA fragment is inserted in the plasmid in the reverse orientation, this digestion would give digestion fragments of 4378/2926 bp. The plasmid DNA was identified based on the expectation. The plasmids thus obtained were expression vectors encoding mouse FR1,2/human FR3,4 hybrid antibody L-chain, which were designated as expression vectors "m/hMBC1La λ/neo" and "m/hMBC1Ld λ/ neo", respectively.

### (ii) Preparation of FR1/FR2 hybrid antibody

An FR1/FR2 hybrid antibody was prepared in the same manner as set forth above utilizing a SnaBI restriction site located on CDR1.

Plasmids MBC1L(λ)/neo and h/mMBC1L(λ)/neo (10 µg each) were separately digested in 20 µl of a reaction solution containing 10 mM Tris-HCl (pH 7.9), 10 mM MgCl₂, 1 mM DTT, 50 mM NaCl, 0.01% (w/v) of BSA and 6U of SnaBI (Takara Shuzo Co., Ltd.) at 37° C for 1 hour. The resulting reaction solutions were further digested in 50 µl of a reaction solution containing 20 mM Tris-HCl (pH 8.5), 10 mM MgCl₂, 1 mM DTT, 100 mM KCl, 0.01% (w/v) of BSA and 6U of PvuI at 37° C for 1 hour.

The resulting reaction solutions were separately subjected to electrophoresis on a 1.5% low-melting agarose gel, thereby giving DNA fragments of 4955 bp (m1) and 2349 bp (m2) from the plasmid MBC1L(λ)/neo and DNA fragments of 4955 bp (hm1) and 2349 bp (hm2) from the plasmid h/mMBC1 L(λ)/neo. These DNA fragments were collected and purified from the gels using GENECLEANII Kit (BIO101). Each of the DNA fragments obtained was dissolved in 40 µl of a solution containing 10 mM Tris-HCl (pH 7.4) and 1 mM EDTA.

The m1 and hm1 fragments (1 µl each) were ligated to the hm2 and m2 fragments (4 µl each), respectively. Each of the resulting ligation products was introduced into a competent cell of E. coli, JM109, to form a transformant. The transformant obtained was cultured in 2 ml of 2xYT medium containing 50 µg/ml of ampicillin. From the cell fraction, the plasmid was purified using QIAprep Spin Plasmid Kit QIAGEN).

Each of the purified plasmids was digested in 20 µl of a reaction solution containing 10 mM Tris-HCl (pH 7.5), 10 mM MgCl₂, 1 mM DTT and either 8U of ApaI (Takara Shuzo Co., Ltd.) or 2U of ApaLI (Takara Shuzo Co., Ltd.) at 37° C for 1 hour.

It was expected that if the fragments were ligated correctly, the digestion reaction would give a fragment of 7304 bp (by the ApaI digestion) or fragments of 5560/1246/498 bp (by the ApaLI digestion) for m1-hm2, and would give fragments of 6538/766 bp (by the ApaI digestion) or fragments of 3535/2025/1246/498 bp (by the ApaLI digestion) for hm1-m2. Based on this expectation, the plasmids were identified. As a result, an expression vector encoding a human FR1/mouse FR2,3,4 hybrid antibody L-chain (designated as "hmmMBC1L(λ)/neo") and an expression vector encoding a mouse FR1/human FR2/mouse FR3,4 hybrid antibody L-chain (designated as "mhmMBC1L(λ)/neo") were obtained.

### (4) Construction of humanized antibody L-chain

A humanized #23-57-137-1 antibody L-chain was prepared by CDR-grafting technique by means of PCR method. For the preparation of a humanized #23-57-137-1 antibody L-chain (version "a") that contained FR1, FR2 and FR3 derived from human antibody HSU03868 (GEN-BANK, Deftos M. et al., Scand. J. Immunol., 39, 95-103, 1994) and FR4 derived from human antibody S25755 (NBRF-PDB), six PCR primers were used.

The six primers were as follows: CDR-grafting primers MBC1LGP1 (SEQ ID NO: 29) and MBC1LGP3 (SEQ ID NO: 30), both having a sense DNA sequence, CDR-grafting primers MBC1LGP2 (SEQ ID NO: 31) and MBC1LGP4 (SEQ ID NO: 32), both having an antisense DNA sequence, all of which had a 15-21 bp complementary sequence on the both terminal ends; and external primers MBC1LVS1 (SEQ ID NO: 33) and MBC1LVR1 (SEQ ID NO: 34) having a homology to the CDR-grafting primers MBC1LGP1 and MBC1LGP4, respectively.

The CDR-grafting primers MBC1LGP1, MBC1LGP2, MBC1LGP3 and MBC1LGP4 were separated on a urea-denatured polyacrylamide gel (Molecular Cloning: A Laboratory Manual, Sambrook et al., Cold Spring Harbor Laboratory Press, 1989) and extracted therefrom by crush-and-soak method (Molecular Cloning: A Laboratory Manual, Sambrook et al., Cold Spring Harbor Laboratory Press, 1989).

Each of the CDR-grafting primers (1 nmole each) was separated on a 6% denatured polyacrylamide gel. The identification of the DNA fragment of a desired length was performed on a silica gel thin plate by irradiation of UV ray. The desired DNA fragment was collected from the gel by crush-and-soak method. The collected DNA fragment was dissolved in 20 µl of a solution containing 10 mM Tris-HCI (pH 7.4) and 1 mM EDTA.

The PCR reaction was performed using TaKaRa Ex Taq (Takara Shuzo Co., Ltd.) and a buffer appended thereto. The PCR reaction solution comprised (per 100 µl) 1 µl of each of the CDR-grafting primers MBC1LGP1, MBC1LGP2, MBC1LGP3 and MBC1LGP4, 0.25 mM dNTPs, 2.5U of TaKaRa Ex Taq in the buffer. The PCR reaction was run for 5 cycles under the conditions: 94° C for 1 min., 55° C for 1 min. and 72° C for 1 min. The resulting reaction mixture was added with 50 pmoles of each of the external primers MBC1LVS and MBC1LVR1. Using this reaction mixture, the PCR reaction was run for additional 30 cycles under the same conditions. The DNA fragment thus amplified was separated by agarose gel electrophoresis on a 3% Nu Sieve GTG agarose (FMC Bio. Products).

An agarose segment containing a DNA fragment of 421 bp was excised, and the DNA fragment was purified therefrom using GENECLEANII Kit (BIO101) in accordance with the instructions included in the kit. The PCR reaction mixture thus obtained was used for subcloning of the DNA fragment into plasmid pUC19 that had been digested with BamHI and HindIII. The resulting plasmid was sequenced. The plasmid thus prepared was designated as "hMBCL/pUC19". In this plasmid, however, the 104-position amino acid (corresponding to the 96th amino acid in accordance with the Kabat's prescription) of CDR4 was replaced by arginine. For the correction of this amino acid to tyrosine, a correction primer MBC1LGP10R (SEQ ID NO: 35) was designed and synthesized. The PCR reaction was performed using TaKaRa Ex Taq (Takara Shuzo Co., Ltd.) and a buffer appended thereto. The PCR reaction solution comprised (per 100 µl) 0.6 µg of the plasmid hMBCL/pUC19 as a template DNA, 50 pmoles of each of the primers MBC1LUS1 and MBC1LGP10R, 2.5U of TaKaRa Ex Taq (Takara Shuzo Co., Ltd.) and 0.25 mM dNTPs in the buffer, over which mineral oil (50 µl) was layered. The PCR reaction was run for 30 cycles under the conditions: 94° C for 1 min., 55° C for 1 min. and 72° C for 1 min. The DNA fragment thus amplified was separated by agarose gel electrophoresis on a 3% Nu Sieve GTG agarose (FMC Bio. Products).

A gel segment containing a DNA fragment of 421 bp was excised, and the DNA fragment was purified therefrom using GENECLEANII Kit (BIO101) in accordance with the instructions included in the kit. The PCR reaction mixture thus prepared was used for subcloning of the DNA fragment into plasmid pUC19 that had been digested with BamHI and HindIII.

The plasmid was sequenced using M13 Primer M4 and M13 Primer RV. As a result, it was confirmed that the plasmid had the correct sequence. The plasmid was then digested with HindIII and BlnI, and a DNA fragment of 416 bp was separated by electrophoresis on a 1% agarose gel. The DNA fragment was purified using GENECLEANII Kit (BIO101) in accordance with the instructions included in the kit, and then introduced into plasmid C λ /pUC19 that had been digested with HindIII and BlnI. The resulting plasmid was designated as "hMBC1La λ/pUC19". This plasmid was digested with EcoRI to give a DNA fragment encoding humanized L-chain. The DNA fragment was introduced into plasmid pCOS 1 so that the initiation codon for the humanized L-chain was located downstream to the EF1 α promoter. The plasmid thus obtained was designated as "hMBC1La λ/pCOS1". The DNA sequence (including the corresponding amino acid sequence) of the humanized L-chain version "a" is shown in SEQ ID NO: 66. The amino acid sequence of the version "a" is also shown in SEQ ID NO: 47.

A humanized L-chain version "b" was prepared using mutagenesis by PCR method. The version "b" was designed such that the 43-position amino acid glycine (corresponding to the 43th amino acid in accordance with the Kabat's prescription) was replaced by proline and the 49-position amino acid lysine (corresponding to the 49th amino acid accordance with the Kabat's prescription) by aspartic acid in the version "a". The PCR reaction was performed using plasmid hMBC 1 La λ/pUC19 as a template and a mutagenic primer MBC1LGP5R (SEQ ID NO: 36) and a primer MBC1LUS1. The DNA fragment obtained was digested with BamHI and HindIII, and the digestion fragment was subcloned into the BamHI-HindIII site of pUC19. After sequencing, the plasmid was digested with HindIII and Af1II, and the resulting digestion fragment was ligated to plasmid hMBC1 La λ /pUC19 that had been digested with HindIII and AflII.

The thus obtained plasmid was designated as "hMBC1Lb λ /pUC19". This plasmid was digested with EcoRI to give a DNA fragment containing a DNA encoding the humanized L-chain. The DNA fragment was introduced into plasmid pCOS I such that the initiation codon for the humanized L-chain was located downstream to the EF1 α promoter. The plasmid thus obtained was designated as "hMBC1Lb λ/pCOS1".

A humanized L-chain version "c" was prepared using mutagenesis by PCR method. The version "c" was designed such that the 84-position amino acid serine (corresponding to the 80th amino acid in accordance with the Kabat's prescription) was replaced by proline. The PCR reaction was performed using plasmid hMBC1La λ /pUC19 as a template and a mutagenic primer MBC1LGP6S (SEQ ID NO: 37) and a primer M13 Primer RV. The DNA fragment obtained was digested with BamHI and HindIII and then subcloned into pUC19 that had been digested with BamHI and HindIII.

After sequencing, the plasmid was digested with BstPI and Aor51HI, and the resulting DNA fragment was ligated to plasmid hMBC1La λ/pUC19 that had been digested with BstPI and Aor51HI. The plasmid thus obtained was designated as "hMBC1Lc λ /pUC19". This plasmid was digested with EcoRI to give a DNA fragment containing a DNA encoding the humanized L-chain. The fragment was introduced into the EcoRI site of plasmid pCOS 1 such that the initiation codon for the humanized L-chain was located downstream to the EF1 α promoter. The plasmid thus obtained was designated as "hMBC1Lc λ/pCOS1".

Humanized L-chain versions "d", "e" and "f" were also prepared using mutagenesis by PCR method. The versions "d", "e" and "f" were. designed such that the 91-position amino acid tyrosine (corresponding to the 87th amino acid in accordance with the Kabat's prescription) was replaced by isoleucine in the versions "a", "b" and "c", respectively. For each of the versions "d", "e" and "f", a PCR reaction was performed using each of plasmid hMBC1La λ/pCOS1 (for version "d"), hMBC1Lb λ /pCOS 1 (for version "e") and hMBC1Lc λ/pCOS1 (for version "f"), respectively, as a template, a mutagenic primer MBC1LGP11R (SEQ ID NO: 38) and a primer M-S1 (SEQ ID NO: 44). The DNA fragment thus obtained was digested with BamHI and HindIII and then subcloned into pUC19 that had been digested with BamHI and HindIII. After sequencing, the plasmid was digested with HindIII and BlnI, and the resulting digestion fragment was ligated to plasmid C λ/pUC19 that had been digested with HindIII and BlnI.

The thus obtained plasmids were respectively designated as "hMBC1Ld λ/pUC19" (for version "d"), "hMBC1Le λ/pUC19" (for version "e") and "hMBC1Lf λ/pUC19" (for version "f"). Each of these plasmids was digested with EcoRI to give a DNA fragment containing a DNA encoding the humanized L-chain. The DNA fragment was introduced into the EcoRI site of plasmid pCOS1 such that the initiation codon for the humanized L-chain was located downstream to the EF1α promoter of the plasmid. The plasmids thus obtained were respectively designated as "hMBC1Ld λ/pCOS1" (for version "d"), "hMBC1Le λ/pCOS1" (for version "e") and "hMBC1Lfλ/pCOS1" (for version "f").

Humanized L-chain versions "g" and "h" were also prepared using mutagenesis by PCR method. The versions "g" and "h" were designed such that the 36-position amino acid histidine (corresponding to the 36th amino acid in accordance with the Kabat's prescription) was replaced by tyrosine in the versions "a" and "d", respectively. The PCR reaction was performed using a mutagenic primer MBC1LGP9R (SEQ ID NO: 39), M13 Primer RV and plasmid hMBC1La λ/pUC19 as a template. An additional PCR was performed using the PCR product thus obtained and M13 Primer M4 as primers and plasmid hMBC1La λ/pUC19 as a template. The DNA fragment obtained was digested with HindIII and BlnI and then subcloned into plasmid Cλ/pUC19 that had been digested with HindIII and BlnI. Using this plasmid as a template, a PCR reaction was performed using primers MBC1LGP13R (SEQ ID NO: 40) and MBC1LVS1. The PCR fragment obtained was digested with ApaI and HindIII and then introduced into either of plasmids hMBC1La λ/pUC19 and hMBC1Ldλ/pUC19 that had been digested with ApaI and HindIII. The plasmids obtained were sequenced. Plasmids that were confirmed to contain the correct sequence were designated as "hMBC1Lg λ/pUC19" (for version "g") and "hMBC1Lh λ/pUC19" (for version "h"). Each of these plasmids was digested with EcoRI to give a DNA fragment containing a DNA encoding the humanized L-chain. The DNA fragment was introduced into the EcoRI site of plasmid pCOS1 such that the initiation codon for the humanized L-chain was located downstream to the EF1 α promoter. The plasmids thus obtained were respectively designated as "hMBC1Lgλ /pCOS1" (for version "g") and "hMBC1Lh λ/pCOS1" (for version "h").

Humanized L-chain versions "i", "j", "k", "l", "m", "n" and "o" were also prepared using mutagenesis by PCR method. The PCR reaction was performed using plasmid hMBC1La λ/pUC19 as a template and a mutagenic primer MBC1LGP14S (SEQ ID NO: 41) and a primer V1RV (λ) (SEQ ID NO: 43). The resulting DNA fragment was digested with ApaI and BlnI and then subcloned into plasmid hMBC1Lg λ/pUC19 that had been digested with ApaI and BlnI. The obtained plasmid was sequenced, and the clone into which the mutation for each version was introduced was selected. The thus obtained plasmid was designated as "hMBC1Lx λ/pUC 19 (x=i, j, k, l, m, n or o)". This plasmid was digested with EcoRI to give a DNA fragment containing a DNA encoding the humanized L-chain. The DNA fragment was introduced into the EcoRI site of plasmid pCOSI such that the initiation codon for the humanized L-chain was located downstream of the EF1 α promoter. The thus obtained plasmid was designated as "hMBC1Lx λ/pCOS1" (x = i,j, k, l, m, n or o). The DNA sequences (including the corresponding amino acid sequences) of the versions "j", "l", "m" and "o" are shown in SEQ ID NOs: 67, 68, 69 and 70, respectively. The amino acid sequences of these versions are also shown in SEQ ID Nos: 48, 49, 50 and 51, respectively.

Humanized L-chain versions "p", "q", "r", "s" and "t" were designed such that the 87-position amino acid (tyrosine) was replaced by isoleucine in the versions "i", "j", "m", "1" and "o", respectively. These versions were prepared utilizing an Aor51MI restriction site on FR3 and replacing that site of each of the versions "i", "j", "m", "l" or "o" by that site of the version "h". That is, an Aor51HI restriction fragment (514 bp) containing CDR3, a part of FR3 and the entire FR4 were removed from an expression plasmid hMBC1Lx λ/pCOS1 (x = i, j, m, l or o). To the removed site, an Aor51HI restriction fragment (514 bp) in the expression plasmid hMBC1Lh λ/pCOS, which containing CDR3 and a part of FR3 and the entire FR4, was ligated, so that the 91-position amino acid tyrosine (corresponding to the 87th amino acid in accordance with the Kabat's prescription) was replaced by isoleucine. The resulting plasmid was sequenced. A clone of each of the versions "i", "j", "m" "l" and "o" in which 91-position amino acid tyrosine (corresponding to the 87th amino acid in accordance with the Kabat's prescription) was replaced by isoleucine was selected. These modified versions respectively corresponding to the versions "i", "j", "m" "1" and "o" were designated as versions "p", "q", "s", "r" and "t", respectively. The obtained plasmid was designated as "hMBC1Lx λ/pCOS1 (x =p, q, s, r or t). The DNA sequences (including the corresponding amino acids) of the versions "q", "r", "s" and "t" are shown in SEQ ID Nos: 71, 72, 73 and 74, respectively. The amino acid sequences of these versions are also shown in SEQ ID Nos: 52, 53, 54 and 55, respectively.

Plasmid hMBC1Lq λ /pCOS1 was digested with HindIII and EcoRI and then subcloned into plasmid pUC19 that had been digested with HindIII and EcoRI. The plasmid thus obtained was designated as "hMBC1Lq λ/pUC19.

The positions of the replaced amino acids in the individual versions of the humanized L-chain are shown in Table 2 below.

**Table 2**

| Versions | 36 | 43 | 45 | 47 | 49 | 80 | 87 |
|---|---|---|---|---|---|---|---|
| a | | | | | | | |
| b | | P | | | D | | |
| c | | | | | | P | |
| d | | | | | | | I |
| e | | P | | | D | | I |
| f | | | | | | P | I |
| g | Y | | | | | | |
| h | Y | | | | | | I |
| i | Y | | K | | | | |
| j | Y | | K | | D | | |
| k | Y | | K | V | | | |
| l | Y | | K | V | D | | |
| m | Y | | | | D | | |
| n | Y | | | V | | | |
| o | Y | | | V | D | | |
| p | Y | | K | | | | I |
| q | Y | | K | | D | | I |
| r | Y | | | | D | | I |
| s | Y | | K | V | D | | I |
| t | Y | | | V | D | | I |
| In Table 2, capital letters represent the following amino acids: Y: tyrosine; P: proline; K: lysine, V: valine; D: aspartic acid; and I: isoleucine. | | | | | | | |

E. coli strains each containing plasmids hMBC1HcDNA/pUC19 and hMBC1Lqλ /pUC19 were designated as "Escherichia coli JM109 (hMBC1HcDNA/pUC19)" and "Escherichia coli JM109 (hMBC1Lq λ/pUC19)", respectively, which have been deposited under the terms of Budapest Treaty at the National Institute of Bioscience and Human-Technology, Agency of Industrial Science and Technology, Japan, (1-3, Higashi 1-chome, Tsukuba-shi, Ibaraki, Japan) on August 15, 1996, under the accession No. FERM BP-5629 for Escherichia coli JM109 (hMBC1HcDNA/pUC19), and FERM BP-5630 for Escherichia coli JM109 (hMBC1Lq λ/pUC19).

### (5) Transfection into COS-7 cell

For the evaluation of the antigen-binding activity and the neutralizing activity of the hybrid antibodies and the humanized #23-57-137-1 antibodies, the above-prepared expression plasmids were expressed transiently in COS-7 cells. For the transient expression of the L-chain hybrid antibodies, each of the following combinations of plasmids were co-transfected into a COS-7 cell by electroporation using Gene Pulser (Bio Rad): hMBC1HcDNA/pCOS1 and h/mMBC1L(λ)/neo; hMBC1HcDNA/pCOS1 and m/hMBC1La λ/neo; hMBC1HcDNA/pCOS1 and m/hMBC1Ld λ/neo; hMBC1HcDNA/pCOS1 and hmmMBC1L(λ)/neo; and hMBC1HcDNA/pCOS1 and mhmMBC1L(λ)/neo. That is, a cell suspension (0.8 ml) of COS-7 cells in PBS(-) (1x10⁷ cells/ml) was added with each combination of the plasmid DNAs (10 µg each). The resulting solution was applied with pulses at an electrostatic capacity of 1,500V and 25 µF. After 10 min. of recovery period at room temperature, the electroporated cells were suspended in DMEM medium containing 2% Ultra Low IgG fetal calf serum (GIBCO), and then cultured using a 10-cm culture dish in a CO₂ incubator. After culturing for 72 hours, a culture supernatant was collected and centrifuged to remove cell debris. The solutions thus prepared were provided for use in the ELISA below.

For the transient expression of the humanized #23-57-137-1 antibodies, plasmids of hMBC1HcDNA/pCOS1 and hMBC1Lx λ/pCOS1 (x = a-t) were co-transfected into a COS-7 cell using Gene Pulser (Bio Rad) in the same manner as described for the above hybrid antibodies. The culture supernatants were prepared and provided for use in the ELISA below.

The purification of the hybrid antibodies and the humanized antibodies from the COS-7 cell culture supernatants was performed using AffiGel Protein A MAPSII Kit (Bio Rad) in accordance with the instructions included in the kit.

### (6) ELISA

### (i) Determination of antibody concentration

An ELISA plate for determining antibody concentration was prepared as follows. Each well of a 96-well ELISA plate (Maxisorp, NUNC) was coated with 100 µl of a coating buffer (0.1 M NaHCO₃, 0.02% NaN₃) containing I µg/ml of goat anti-human IgG antibody (TAGO) and then blocked with 200 µl of a dilution buffer [50 mM Tris-HCl, 1 mM MgCl₂, 0.1 M NaCl, 0.05% Tween 20, 0.02% NaN₃, 1% bovine serum albumin (BSA); pH 7.2]. Each of the wells was added with each of the serial dilutions of the COS cell culture supernatant in which each of the hybrid antibodies and the humanized antibodies was expressed, or added with each of the serial dilutions of each of the hybrid antibodies and humanized antibodies in a purified form. The plate was incubated at room temperature for 1 hour and washed with PBS-Tween 20. Subsequently, each of the wells was added with 100 µl of alkaline phosphatase-conjugated goat anti-human IgG antibody (TAGO). The plate was incubated at room temperature for 1 hour and washed with PBS-Tween 20. Subsequently, each of the wells was added with 1 mg/ml of a substrate solution ("Sigma 104", p-nitrophenylphosphoric acid, SIGMA). The solution in each well was measured on its absorbance at 405 nm using Microplate Reader (Bio Rad) to determine the antibody concentration. In this determination, Hu IgG1λ Purified (The Binding Site) was used as the standard substance.

### (ii) Determination of antigen-binding ability

An ELISA plate for determining antigen-binding ability was prepared as follows. Each well of a 96-well ELISA plate (Maxisorp, NUNC) was coated with 100 µl of a coating buffer containing 1 µg/ml of human PTHrP (1-34) and then blocked with 200 µl of a dilution buffer. Subsequently, each well was added with each of the serial dilutions of the COS-7 cell culture supernatant in which each of the hybrid antibodies and humanized antibodies was expressed, or added with each of the serial dilutions of each of the hybrid antibodies and humanized antibodies in a purified form. The plate was incubated at room temperature and washed with PBS-Tween 20. Subsequently, each well was added with 100 µl of alkaline phosphatase-conjugated goat anti-human IgG antibody (TAGO). The plate was incubated at room temperature and washed with PBS-Tween 20. Subsequently, each well was added with 1 mg/ml of a substrate solution ("Sigma 104", p-nitrophenylphosphoric acid, SIGMA). The solution was measured on its absorbance at 405 nm using Microplate Reader (Bio Rad).

### (7) Confirmation of activities

### (i) Evaluation of humanized H-chain

It was found that an antibody having both a humanized H-chain version "a" and a chimeric L-chain exhibited the same level of PTHrP-binding activity as that of a chimeric antibody. This result suggests that the version "a" achieves the humanization of the H-chain V-region in the degree enough to evaluate the humanization. Therefore, the humanized H-chain version "a" was provided for use as a humanized antibody H-chain in the following experiments.

### (ii) Activity of hybrid antibodies

### (ii-a) FR1,2/FR3,4 hybrid antibody

When the L-chain was h/mMBC1L(λ), no antigen-binding activity was observed. In contrast, when the L-chain was either m/hMBC1La λ or m/hMBC1Ldλ, the same level of antigen-binding activity as that of the chimeric #23-57-137-1 antibody was observed (FIG. 7). These results suggest that FR3 and FR4 have no problem as humanized antibodies but FR1 and FR2 contain amino acid residue(s) that need to be replaced.

### (ii-b) FR1/FR2 hybrid antibody

When the L-chain was mhmMBC1L(λ), no antigen-binding activity was observed. In contrast, when the L-chain was hmmMBC1L(λ), the same level of antigen-binding activity as that of the chimeric #23-57-137-1 antibody was observed (FIG. 8). These results suggest that FR1 has no problem as a humanized antibody but FR2 contains amino acid residue(s) that need to be replaced.

### (iii) Activity of humanized antibodies

The antigen-binding activity of the humanized antibodies having the L-chain versions "a" to "t", respectively, were determined. As a result, it was found that the humanized antibodies having the L-chain versions "j", "l" "m", "o", "q", "r", "s" and "t" exhibited the same levels of PTHrP-binding activity as that of the chimeric antibody.

### (8) Establishment of CHO cell line capable of stable production of antibody

For establishing a cell line capable of stable production of humanized antibodies, each of the above-prepared expression plasmids was introduced into a CHO cell (DXB11).

That is, the establishment of a cell line capable of stable production of a humanized antibody was performed using each of the following combinations of plasmids as expression vectors for a CHO cell; hMBC1HcDNA/pCHO1 and hMBC1Lm λ/pCOS1; hMBC1HcDNA/pCHO1 and hMBC1Lqλ/pCOS1; and hMBC1HcDNA/pCHO1 and hMBC1Lr λ/pCOS1. The plasmids were co-transfected into a CHO cell by electroporation using Gene Pulser (Bio Rad). Subsequently, the expression vectors were separately cleaved with restriction enzyme PvuI to give linear DNA fragments. The resulting DNA fragments were extracted with phenol and chloroform and then precipitated with ethanol. The DNA fragments thus prepared were used in the subsequent electroporation. That is, the plasmid DNA fragments (10 µg each) were added to 0.8 ml of a cell suspension of CHO cells in PBS(-) (1x10⁷ cells/ml). The resulting solution was applied with pulses at an electrostatic capacity of 1,500V and 25 µF. After 10 min. of recovery period at room temperature, the cells thus treated were suspended in MEM- α medium (GIBCO) containing 10% fetal calf serum (GIBCO), and then cultured in a CO₂ incubator using 96-well plates (Falcon). On the day following the culturing being started, the medium was replaced by ribonucleoside- or deoxyribonucleoside-free MEM- α selective medium containing 10% fetal calf serum (GIBCO) and 500 mg/ml of GENETICIN (G418Sulfate; GIBCO). From the culture medium, cells into which the antibody gene was introduced were selected. The culture medium was replaced by a fresh one. About two weeks after the medium replacement, the cells were observed microscopically. When a satisfactory cell growth was observed, the amount of the antibodies produced was determined by conventional ELISA for determination of antibody concentration as set forth above. Among the cells, those cells which produced a larger amount of antibodies were screened.

The culturing of the established cell line capable of stable production of antibodies was scaled up in a roller bottle using a ribonucleoside- or deoxyribonucleoside-free MEM-α medium containing 2% Ultra Low IgG fetal calf serum. On each of day 3 and day 4 of the culturing, the culture supernatant was collected and filtered on a 0.2-µm filter (Millipore) to remove cell debris therefrom. The purification of the humanized antibodies from the culture supernatant of the CHO cells was performed using POROS Protein A Column (PerSeptive Biosystems) on ConSep LC100 (Millipore) in accordance with the appended instructions. The humanized antibodies were provided for use in the determination of neutralizing activity and examination of pharmacological efficacy in hypercalcemic model animals. The concentration and the antigen-binding activity of the purified humanized antibodies were determined by the ELISA system as set forth above.

### [REFERENCE EXAMPLE 5] Determination of neutralizing activity

The determination of neutralizing activity of the mouse antibodies, the chimeric antibodies and the humanized antibodies was performed using rat myeloma cell line ROS17/2.8-5 cells. The ROS17/2.8-5 cells were cultured in Ham'S F-12 medium (GIBCO) containing 10% fetal calf serum (GIBCO) in a CO₂ incubator. The ROS17/2.8-5 cells were seeded into each well of a 96-well plate at a density of 10⁴ cells/100 µl/well and cultured for one day. After the culturing was completed, the culture medium was replaced by Ham'S F-12 medium (GIBCO) containing 4 mM Hydrocortisone and 10% fetal calf serum. After culturing for three to four days, the cultured cells were washed with 260 µl of Ham'S F-12 medium (GIBCO), and then added with 80 µl of Ham's F-12 medium containing 1 mM isobutyl-1-methyl xanthine (IBMX, SIGMA), 10% fetal calf serum and 10 mM HEPES. The resulting mixture was incubated at 37° C for 30 min.

The culture mediums of the mouse antibodies, the chimeric antibodies and the humanized antibodies to be tested for neutralizing activity were previously diluted serially in the following dilution series: [10 µg/ml, 3.3 µg/ml, 1.1 µg/ml and 0.37 µg/ml], [10 µg/ml, 2 µg/ml, 0.5 µg/ml and 0.01 µg/ml] and [10 µg/ml, 5 µg/ml, 1.25 µg/ml, 0.63 µg/ml and 0.31 µg/ml]. Each of the diluted antibody sample solutions was mixed with an equivalent amount of 4 ng/ml of PTHrP (1-34). The resulting mixed solution (80 µl) was added to each well. In each well, the final concentration of each antibody became a quarter of the above-mentioned concentration of the antibody, and accordingly the concentration of PTHrP (1-34) became 1 ng/ml. After the treatment at room temperature for 10 min., the culture supernatant was removed and the residue was washed with PBS three times. Subsequently, cAMP in the cells was extracted with 100 µl of a 0.3% HCl-95% ethanol and then evaporated using a water jet aspirator to remove the HCl-ethanol. The residue was dissolved in 120 µl of EIA buffer appended to cAMP EIA Kit (CAYMAN CHEMICAL'S) to extract the cAMP therefrom. The cAMP was determined using cAMP EIA Kit (CAYMAN CHEMICAL'S) in accordance with the instructions included in the kit. As a result, it was found that, among the humanized antibodies having the same levels of antigen-binding activity as that of the chimeric antibody, those antibodies having L-chain versions "q", "r", "s" and "t" (in which the 91-position tyrosine was replaced by isoleucine) exhibited the similar neutralizing activity to that of the chimeric antibody, and that antibody having a L-chain version "q" exhibited the strongest neutralizing activity.

### Sequence Listing Free Text

SEQ ID NO: 1 Synthesized DNA
SEQ ID NO: 2 Synthesized DNA
SEQ ID NO: 3 Synthesized DNA
SEQ ID NO: 4 Synthesized DNA
SEQ ID NO: 5 Synthesized DNA
SEQ ID NO: 6 Synthesized DNA
SEQ ID NO: 7 Synthesized DNA
SEQ ID NO: 8 Synthesized DNA
SEQ ID NO: 9 Synthesized DNA
SEQ ID NO: 10 Synthesized DNA
SEQ ID NO: 11 Synthesized DNA
SEQ ID NO: 12 Synthesized DNA
SEQ ID NO: 13 Synthesized DNA
SEQ ID NO: 14 Synthesized DNA
SEQ ID NO: 15 Synthesized DNA
SEQ ID NO: 16 Synthesized DNA
SEQ ID NO: 17 Synthesized DNA
SEQ ID NO: 18 Synthesized DNA
SEQ ID NO: 19 Synthesized DNA
SEQ ID NO: 20 Synthesized DNA
SEQ ID NO: 21 Synthesized DNA
SEQ ID NO: 22 Synthesized DNA
SEQ ID NO: 23 Synthesized DNA
SEQ ID NO: 24 Synthesized DNA
SEQ ID NO: 25 Synthesized DNA
SEQ ID NO: 26 Synthesized DNA
SEQ ID NO: 27 Synthesized DNA
SEQ ID NO: 28 Synthesized DNA
SEQ ID NO: 29 Synthesized DNA
SEQ ID NO: 30 Synthesized DNA
SEQ ID NO: 31 Synthesized DNA
SEQ ID NO: 32 Synthesized DNA
SEQ ID NO: 33 Synthesized DNA
SEQ ID NO: 34 Synthesized DNA
SEQ ID NO: 35 Synthesized DNA
SEQ ID NO: 36 Synthesized DNA
SEQ ID NO: 37 Synthesized DNA
SEQ ID NO: 38 Synthesized DNA
SEQ ID NO: 39 Synthesized DNA
SEQ ID NO: 40 Synthesized DNA
SEQ ID NO: 41 Synthesized DNA
SEQ ID NO: 42 Synthesized DNA
SEQ ID NO: 43 Synthesized DNA
SEQ ID NO: 44 Synthesized DNA

All publications, patents and patent applications cited herein are incorporated by reference in their entirety.

### INDUSTRIAL APPLICABILITY

The present invention provides a therapeutic agent for diseases caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor.

SEQUENCE LISTING

<110> CHUGAI SEIYAKU KABUSHIKI KAISHA

<120> Therapeutic agent for diseases caused with PTH or PTHrP

<130> PH-945-PCT

<150> JP 11-189793
<151> 1999-07-02

<160> 75

<170> PatentIn Ver. 2.0

<210> 1
<211> 20
<212> DNA
<213> Artificial Sequence

<220>
<223> Synthetic DNA

<400> 1
aaatagccct tgaccaggca                                                20

<210> 2
<211> 38
<212> DNA

<213> Artificial Sequence

<220>
<223> Synthetic DNA

<400> 2
ctggttcggc ccacctctga aggttccaga atcgatag 38

<210> 3
<211> 28
<212> DNA
<213> Artificial Sequence

<220>
<223> Synthetic DNA

<400> 3
ggatcccggg ccagtggata gacagatg 28

<210> 4
<211> 29
<212> DNA
<213> Artificial Sequence

<220>
<223> Synthetic DNA

<400> 4
ggatcccggg tcagrggaag gtggraaca 29

<210> 5
<211> 17
<212> DNA
<213> Artificial Sequence

<220>
<223> Synthetic DNA

<400> 5
gttttcccag tcacgac                                                    17

<210> 6
<211> 17
<212> DNA
<213> Artificial Sequence

<220>
<223> Synthetic DNA

<400> 6
caggaaacag ctatgac                                                    17

<210> 7
<211> 31
<212> DNA
<213> Artificial Sequence

<220>

<223> Synthetic DNA

<400> 7
gtctaagctt ccaccatgaa acttcgggct c                                    31

<210> 8
<211> 30
<212> DNA
<213> Artificial Sequence

<220>
<223> Synthetic DNA

<400> 8
tgttggatcc ctgcagagac agtgaccaga                                      30

<210> 9
<211> 36
<212> DNA
<213> Artificial Sequence

<220>
<223> Synthetic DNA

<400> 9
gtctgaattc aagcttccac catggggttt gggctg                               36

<210> 10
<211> 41

<212> DNA
<213> Artificial Sequence

<220>
<223> Synthetic DNA

<400> 10
tttcccgggc ccttggtgga ggctgaggag acggtgacca g 41

<210> 11
<211> 109
<212> DNA
<213> Artificial Sequence

<220>
<223> Synthetic DNA

<400> 11
gtctgaattc aagcttagta cttggccagc ccaaggccaa ccccacggtc accctgttcc 60
cgccctcctc tgaggagctc caagccaaca aggccacact agtgtgtct 109

<210> 12
<211> 110
<212> DNA
<213> Artificial Sequence

<220>
<223> Synthetic DNA

<400> 12
ggtttggtgg tctccactcc cgccttgacg gggctgccat ctgccttcca ggccactgtc 60
acagctcccg ggtagaagtc actgatcaga cacactagtg tggccttgtt 110

<210> 13
<211> 98
<212> DNA
<213> Artificial Sequence

<220>
<223> Synthetic DNA

<400> 13
ggagtggaga ccaccaaacc ctccaaacag agcaacaaca agtacgcggc cagcagctac 60
ctgagcctga cgcccgagca gtggaagtcc cacagaag 98

<210> 14
<211> 106
<212> DNA
<213> Artificial Sequence

<220>
<223> Synthetic DNA

<400> 14
tgttgaattc ttactatgaa cattctgtag gggccactgt cttctccacg gtgctccctt 60
catgcgtgac ctggcagctg tagcttctgt gggacttcca ctgctc 106

<210> 15

<211> 43
<212> DNA
<213> Artificial Sequence

<220>
<223> Synthetic DNA

<400> 15
gtctgaattc aagcttagta cttggccagc ccaaggccaa ccc                43

<210> 16
<211> 20
<212> DNA
<213> Artificial Sequence

<220>
<223> Synthetic DNA

<400> 16
tgttgaattc ttactatgaa                                          20

<210> 17
<211> 39
<212> DNA
<213> Artificial Sequence

<220>
<223> Synthetic DNA

<400> 17
caacaagtac gcggccagca gctacctgag cctgacgcc                                39

<210> 18
<211> 39
<212> DNA
<213> Artificial Sequence

<220>
<223> Synthetic DNA

<400> 18
gtagctgctg gccgcgtact tgttgttgct ctgtttgga                                39

<210> 19
<211> 46
<212> DNA
<213> Artificial Sequence

<220>
<223> Synthetic DNA

<400> 19
gtctgaattc aagcttagtc ctaggtcgaa ctgtggctgc accatc                        46

<210> 20
<211> 34
<212> DNA
<213> Artificial Sequence

<220>
<223> Synthetic DNA

<400> 20
tgttgaattc ttactaacac tctcccctgt tgaa                                34

<210> 21
<211> 35
<212> DNA
<213> Artificial Sequence

<220>
<223> Synthetic DNA

<400> 21
gtctaagctt ccaccatggc ctggactcct ctctt                               35

<210> 22
<211> 48
<212> DNA
<213> Artificial Sequence

<220>
<223> Synthetic DNA

<400> 22
tgttgaattc agatctaact acttacctag gacagtgacc ttggtccc                 48

<210> 23
<211> 128
<212> DNA
<213> Artificial Sequence

<220>
<223> Synthetic DNA

<400> 23
gtctaagctt ccaccatggg gtttgggctg agctgggttt tcctcgttgc tcttttaaga 60
ggtgtccagt gtcaggtgca gctggtggag tctgggggag gcgtggtcca gcctgggagg 120
tccctgag 128

<210> 24
<211> 125
<212> DNA
<213> Artificial Sequence

<220>
<223> Synthetic DNA

<400> 24
accattagta gtggtggtag ttacacctac tatccagaca gtgtgaaggg gcgattcacc 60
atctccagag acaattccaa gaacacgctg tatctgcaaa tgaacagcct gagagctgag 120
gacac 125

<210> 25
<211> 132
<212> DNA

<213> Artificial Sequence

<220>
<223> Synthetic DNA

<400> 25
ctaccaccac tactaatggt tgccacccac tccagcccct tgcctggagc ctggcggacc 60
caagacatgc catagctact gaaggtgaat ccagaggctg cacaggagag tctcagggac 120
ctcccaggct gg 132

<210> 26
<211> 110
<212> DNA
<213> Artificial Sequence

<220>
<223> Synthetic DNA

<400> 26
tgttggatcc ctgaggagac ggtgaccagg gttccctggc cccagtaagc aaagtaagtc 60
atagtagtct gtctcgcaca gtaatacaca gccgtgtcct cagctctcag 110

<210> 27
<211> 30
<212> DNA
<213> Artificial Sequence

<220>
<223> Synthetic DNA

<400> 27
gtctaagctt ccaccatggg gtttgggctg                                        30

<210> 28
<211> 30
<212> DNA
<213> Artificial Sequence

<220>
<223> Synthetic DNA

<400> 28
tgttggatcc ctgaggagac ggtgaccagg                                        30

<210> 29
<211> 133
<212> DNA
<213> Artificial Sequence

<220>
<223> Synthetic DNA

<400> 29
acaaagcttc caccatggcc tggactcctc tcttcttctt ctttgttctt cattgctcag 60
gttctttctc ccagcttgtg ctgactcaat cgccctctgc ctctgcctcc ctgggagcct 120
cggtcaagct cac                                                          133

<210> 30

<211> 118

<212> DNA

<213> Artificial Sequence

<220>

<223> Synthetic DNA

<400> 30

agcaagatgg aagccacagc acaggtgatg ggattcctga tcgcttctca ggctccagct 60

ctggggctga gcgctacctc accatctcca gcctccagtc tgaggatgag gctgacta 118

<210> 31

<211> 128

<212> DNA

<213> Artificial Sequence

<220>

<223> Synthetic DNA

<400> 31

ctgtggcttc catcttgctt aagtttcatc aagtaccgag ggcccttctc tggctgctgc 60

tgatgccatt caatggtgta cgtactgtgc tgactactca aggtgcaggt gagcttgacc 120

gaggctcc 128

<210> 32

<211> 114

<212> DNA

<213> Artificial Sequence

<220>
<223> Synthetic DNA

<400> 32
cttggatccg ggctgaccta ggacggtcag tttggtccct ccgccgaaca ccctcacaaa 60
ttgttcctta attgtatcac ccacaccaca gtaatagtca gcctcatcct caga 114

<210> 33
<211> 17
<212> DNA
<213> Artificial Sequence

<220>
<223> Synthetic DNA

<400> 33
acaaagcttc caccatg 17

<210> 34
<211> 19
<212> DNA
<213> Artificial Sequence

<220>
<223> Synthetic DNA

<400> 34
cttggatccg ggctgacct 19

<210> 35

<211> 75

<212> DNA

<213> Artificial Sequence

<220>

<223> Synthetic DNA

<400> 35

cttggatccg ggctgaccta ggacggtcag tttggtccct ccgccgaaca cgtacacaaa 60

ttgttcctta attgt 75

<210> 36

<211> 43

<212> DNA

<213> Artificial Sequence

<220>

<223> Synthetic DNA

<400> 36

aaaggatcct taagatccat caagtaccga gggggcttct ctg 43

<210> 37

<211> 46

<212> DNA

<213> Artificial Sequence

<220>

<223> Synthetic DNA

<400> 37
acaaagctta gcgctacctc accatctcca gcctccagcc tgagga                46

<210> 38
<211> 111
<212> DNA
<213> Artificial Sequence

<220>
<223> Synthetic DNA

<400> 38
cttggatccg ggctgaccta ggacggtcag tttggtccct ccgccgaaca cgtacacaaa 60
ttgttcctta attgtatcac ccacaccaca gatatagtca gcctcatcct c          111

<210> 39
<211> 42
<212> DNA
<213> Artificial Sequence

<220>
<223> Synthetic DNA

<400> 39
cttctctggc tgctgctgat accattcaat ggtgtacgta ct                    42

<210> 40

<211> 26
<212> DNA
<213> Artificial Sequence

<220>
<223> Synthetic DNA

<400> 40
cgagggccct tctctggctg ctgctg                                          26

<210> 41
<211> 35
<212> DNA
<213> Artificial Sequence

<220>
<223> Synthetic DNA

<400> 41
gagaagggcc ctargtacst gatgrawctt aagca                                35

<210> 42
<211> 35
<212> DNA
<213> Artificial Sequence

<220>
<223> Synthetic DNA

<400> 42
cacgaattca ctatcgattc tggaaccttc agagg                                35

<210> 43
<211> 18
<212> DNA
<213> Artificial Sequence

<220>
<223> Synthetic DNA

<400> 43
ggcttggagc tcctcaga                                                   18

<210> 44
<211> 20
<212> DNA
<213> Artificial Sequence

<220>
<223> Synthetic DNA

<400> 44
gacagtggtt caaagttttt                                                 20

<210> 45
<211> 118
<212> PRT
<213> Mus musculus

<400> 45

Gln Leu Val Leu Thr Gln Ser Ser Ser Ala Ser Phe Ser Leu Gly Ala

1               5                   10                  15

Ser Ala Lys Leu Thr Cys Thr Leu Ser Ser Gln His Ser Thr Tyr Thr

            20                  25                  30

Ile Glu Trp Tyr Gln Gln Gln Pro Leu Lys Pro Pro Lys Tyr Val Met

        35                  40                  45

Asp Leu Lys Gln Asp Gly Ser His Ser Thr Gly Asp Gly Ile Pro Asp

    50                  55                  60

Arg Phe Ser Gly Ser Ser Ser Gly Ala Asp Arg Tyr Leu Ser Ile Ser

65                  70                  75                  80

Asn Ile Gln Pro Glu Asp Glu Ala Met Tyr Ile Cys Gly Val Gly Asp

                85                  90                  95

Thr Ile Lys Glu Gln Phe Val Tyr Val Phe Gly Gly Gly Thr Lys Val

            100                 105                 110

Thr Val Leu Gly Gln Pro

        115

<210> 46

<211> 118

<212> PRT

<213> Mus musculus

<400> 46

Glu Val Gln Leu Val Glu Ser Gly Gly Asp Leu Val Lys Pro Gly Gly

1               5                   10                  15

Ser Leu Lys Leu Ser Cys Ala Ala Ser Gly Phe Thr Phe Ser Ser Tyr

            20                  25                  30

Gly Met Ser Trp Ile Arg Gln Thr Pro Asp Lys Arg Leu Glu Trp Val
        35                  40                  45
Ala Thr Ile Ser Ser Gly Gly Ser Tyr Thr Tyr Tyr Pro Asp Ser Val
    50                  55                  60
Lys Gly Arg Phe Thr Ile Ser Arg Asp Asn Ala Lys Asn Thr Leu Tyr
65                  70                  75                  80
Leu Gln Met Ser Ser Leu Lys Ser Glu Asp Thr Ala Met Phe Tyr Cys
                85                  90                  95
Ala Arg Gln Thr Thr Met Thr Tyr Phe Ala Tyr Trp Gly Gln Gly Thr
            100                 105                 110
Leu Val Thr Val Ser Ala
        115

<210> 47
<211> 116
<212> PRT
<213> Homo sapiens

<400> 47
Gln Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp His Gln Gln Gln Pro Glu Lys Gly Pro Arg Tyr Leu Met
        35                  40                  45
Lys Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80

Ser Leu Gln Ser Glu Asp Glu Ala Asp Tyr Tyr Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly
        115

<210> 48
<211> 118
<212> PRT
<213> Homo sapiens

<400> 48
Gln Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Lys Tyr Leu Met
        35                  40                  45
Asp Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly Gln Pro

        115

<210> 49
<211> 118
<212> PRT
<213> Homo sapiens

<400> 49
Gln Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Lys Tyr Val Met
        35                  40                  45
Asp Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly Gln Pro
        115

<210> 50
<211> 118
<212> PRT
<213> Homo sapiens

<400> 50

Gln Leu Val Leu Thr Gln Ser Pro Ser Ala Ser Ala Ser Leu Gly Ala
1               5                   10                  15

Ser Val Lys Leu Thr Cys Thr Leu Ser Ser Gln His Ser Thr Tyr Thr
            20                  25                  30

Ile Glu Trp Tyr Gln Gln Gln Pro Glu Lys Gly Pro Arg Tyr Leu Met
        35                  40                  45

Asp Leu Lys Gln Asp Gly Ser His Ser Thr Gly Asp Gly Ile Pro Asp
    50                  55                  60

Arg Phe Ser Gly Ser Ser Ser Gly Ala Glu Arg Tyr Leu Thr Ile Ser
65                  70                  75                  80

Ser Leu Gln Ser Glu Asp Glu Ala Asp Tyr Tyr Cys Gly Val Gly Asp
                85                  90                  95

Thr Ile Lys Glu Gln Phe Val Tyr Val Phe Gly Gly Gly Thr Lys Leu
            100                 105                 110

Thr Val Leu Gly Gln Pro
        115

<210> 51
<211> 118
<212> PRT
<213> Homo sapiens

<400> 51
Gln Leu Val Leu Thr Gln Ser Pro Ser Ala Ser Ala Ser Leu Gly Ala
1               5                   10                  15

Ser Val Lys Leu Thr Cys Thr Leu Ser Ser Gln His Ser Thr Tyr Thr
            20                  25                  30

Ile Glu Trp Tyr Gln Gln Gln Pro Glu Lys Gly Pro Arg Tyr Val Met
        35                  40                  45
Asp Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly Gln Pro
        115

<210> 52
<211> 118
<212> PRT
<213> Homo sapiens

<400> 52
Gln Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Lys Tyr Leu Met
        35                  40                  45
Asp Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80

Ser Leu Gln Ser Glu Asp Glu Ala Asp Tyr Ile Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly Gln Pro
        115

<210> 53
<211> 118
<212> PRT
<213> Homo sapiens

<400> 53
Gln Leu Val Leu Thr Gln Ser Pro Ser Ala Ser Ala Ser Leu Gly Ala
  1               5                  10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Arg Tyr Leu Met
        35                  40                  45
Asp Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
 65                  70                  75                  80
Ser Leu Gln Ser Glu Asp Glu Ala Asp Tyr Ile Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly Gln Pro
        115

<210> 54
<211> 118
<212> PRT
<213> Homo sapiens

<400> 54
Gln Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Lys Tyr Val Met
        35                  40                  45
Asp Leu Lys Gln Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Ile Cys Gly Val Gly Asp
                85                  90                  95
Thr Ile Lys Glu Gln Phe Val Tyr Val Phe Gly Gly Gly Thr Lys Leu
            100                 105                 110
Thr Val Leu Gly Gln Pro
        115

<210> 55
<211> 118
<212> PRT
<213> Homo sapiens

<400> 55

Gln Leu Val Leu Thr Gln Ser Pro Ser Ala Ser Ala Ser Leu Gly Ala

1               5                   10                  15

Ser Val Lys Leu Thr Cys Thr Leu Ser Ser Gln His Ser Thr Tyr Thr

            20                  25                  30

Ile Glu Trp Tyr Gln Gln Gln Pro Glu Lys Gly Pro Arg Tyr Val Met

        35                  40                  45

Asp Leu Lys Gln Asp Gly Ser His Ser Thr Gly Asp Gly Ile Pro Asp

    50                  55                  60

Arg Phe Ser Gly Ser Ser Ser Gly Ala Glu Arg Tyr Leu Thr Ile Ser

65                  70                  75                  80

Ser Leu Gln Ser Glu Asp Glu Ala Asp Tyr Ile Cys Gly Val Gly Asp

                85                  90                  95

Thr Ile Lys Glu Gln Phe Val Tyr Val Phe Gly Gly Gly Thr Lys Leu

            100                 105                 110

Thr Val Leu Gly Gln Pro

        115

<210> 56

<211> 118

<212> PRT

<213> Homo sapiens

<400> 56

Gln Val Gln Leu Val Glu Ser Gly Gly Gly Val Val Gln Pro Gly Arg

1               5                   10                  15

Ser Leu Arg Leu Ser Cys Ala Ala Ser Gly Phe Thr Phe Ser Ser Tyr

            20                  25                  30

Gly Met Ser Trp Val Arg Gln Ala Pro Gly Lys Gly Leu Glu Trp Val

        35                  40                  45
Ala Thr Ile Ser Ser Gly Gly Ser Tyr Thr Tyr Tyr Pro Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Gln Thr Thr Met Thr Tyr Phe Ala Tyr Trp Gly Gln Gly Thr
            100                 105                 110
Leu Val Thr Val Ser Ser
        115

<210> 57
<211> 411
<212> DNA
<213> Mus musculus

<220>
<221> CDS
<222> (1)..(411)

<220>
<221> mat_peptide
<222> (58)..(411)

<400> 57
atg aac ttc ggg ctc agc ttg att ttc ctt gcc ctc att tta aaa ggt 48
Met Asn Phe Gly Leu Ser Leu Ile Phe Leu Ala Leu Ile Leu Lys Gly
-15 -10 -5

gtc cag tgt gag gtg caa ctg gtg gag tct ggg gga gac tta gtg aag    96
Val Gln Cys Glu Val Gln Leu Val Glu Ser Gly Gly Asp Leu Val Lys
        -1   1                   5                   10
cct gga ggg tcc ctg aaa ctc tcc tgt gca gcc tct gga ttc act ttc    144
Pro Gly Gly Ser Leu Lys Leu Ser Cys Ala Ala Ser Gly Phe Thr Phe
     15                  20                  25
agt agc tat ggc atg tct tgg att cgc cag act cca gac aag agg ctg    192
Ser Ser Tyr Gly Met Ser Trp Ile Arg Gln Thr Pro Asp Lys Arg Leu
 30                  35                  40                  45
gag tgg gtc gca acc att agt agt ggt ggt agt tac acc tac tat cca    240
Glu Trp Val Ala Thr Ile Ser Ser Gly Gly Ser Tyr Thr Tyr Tyr Pro
                 50                  55                  60
gac agt gtg aag ggg cga ttc acc atc tcc aga gac aat gcc aag aac    288
Asp Ser Val Lys Gly Arg Phe Thr Ile Ser Arg Asp Asn Ala Lys Asn
             65                  70                  75
acc cta tac ctg caa atg agc agt ctg aag tct gag gac aca gcc atg    336
Thr Leu Tyr Leu Gln Met Ser Ser Leu Lys Ser Glu Asp Thr Ala Met
         80                  85                  90
ttt tac tgt gca aga cag act act atg act tac ttt gct tac tgg ggc    384
Phe Tyr Cys Ala Arg Gln Thr Thr Met Thr Tyr Phe Ala Tyr Trp Gly
     95                 100                 105
caa ggg act ctg gtc act gtc tct gca                                411
Gln Gly Thr Leu Val Thr Val Ser Ala
110                 115

<210> 58
<211> 411
<212> DNA
<213> Homo sapiens

<220>
<221> CDS
<222> (1)..(411)

<220>
<221> mat_peptide
<222> (58)..(411)

<400> 58
atg ggg ttt ggg ctg agc tgg gtt ttc ctc gtt gct ctt tta aga ggt 48
Met Gly Phe Gly Leu Ser Trp Val Phe Leu Val Ala Leu Leu Arg Gly
-15 -10 -5
gtc cag tgt cag gtg cag ctg gtg gag tct ggg gga ggc gtg gtc cag 96
Val Gln Cys Gln Val Gln Leu Val Glu Ser Gly Gly Gly Val Val Gln
-1 1 5 10
cct ggg agg tcc ctg aga ctc tcc tgt gca gcc tct gga ttc acc ttc 144
Pro Gly Arg Ser Leu Arg Leu Ser Cys Ala Ala Ser Gly Phe Thr Phe
15 20 25
agt agc tat ggc atg tct tgg gtc cgc cag gct cca ggc aag ggg ctg 192
Ser Ser Tyr Gly Met Ser Trp Val Arg Gln Ala Pro Gly Lys Gly Leu
30 35 40 45
gag tgg gtg gca acc att agt agt ggt ggt agt tac acc tac tat cca 240
Glu Trp Val Ala Thr Ile Ser Ser Gly Gly Ser Tyr Thr Tyr Tyr Pro
50 55 60
gac agt gtg aag ggg cga ttc acc atc tcc aga gac aat tcc aag aac 288
Asp Ser Val Lys Gly Arg Phe Thr Ile Ser Arg Asp Asn Ser Lys Asn
65 70 75
acg ctg tat ctg caa atg aac agc ctg aga gct gag gac acg gct gtg 336

Thr Leu Tyr Leu Gln Met Asn Ser Leu Arg Ala Glu Asp Thr Ala Val
80 85 90
tat tac tgt gcg aga cag act act atg act tac ttt gct tac tgg ggc 384
Tyr Tyr Cys Ala Arg Gln Thr Thr Met Thr Tyr Phe Ala Tyr Trp Gly
95 100 105
cag gga acc ctg gtc acc gtc tcc tca 411
Gln Gly Thr Leu Val Thr Val Ser Ser
110 115

<210> 59
<211> 11
<212> PRT
<213> Homo sapiens

<400> 59
Lys Ala Ser Gln Asp Val Asn Thr Ala Val Ala
1               5                   10

<210> 60
<211> 7
<212> PRT
<213> Homo sapiens

<400> 60
Ser Ala Ser Asn Arg Tyr Thr
1               5

<210> 61
<211> 9

<212> PRT
<213> Homo sapiens

<400> 61
Gln Gln His Tyr Ser Thr Pro Phe Thr
1               5

<210> 62
<211> 5
<212> PRT
<213> Homo sapiens

<400> 62
Pro Tyr Trp Met Gln
1               5

<210> 63
<211> 16
<212> PRT
<213> Homo sapiens

<400> 63
Ser Ile Phe Gly Asp Gly Asp Thr Arg Tyr Ser Gln Lys Phe Lys Gly
1               5                   10                  15

<210> 64
<211> 11
<212> PRT
<213> Homo sapiens

<400> 64

Gly Leu Arg Arg Gly Gly Tyr Tyr Phe Asp Tyr
1               5                   10

<210> 65
<211> 411
<212> DNA
<213> Mus musculus

<220>
<221> CDS
<222> (1)..(411)

<220>
<221> mat_peptide
<222> (58)..(411)

<400> 65
atg gcc tgg act cct ctc ttc ttc ttc ttt gtt ctt cat tgc tca ggt     48
Met Ala Trp Thr Pro Leu Phe Phe Phe Phe Val Leu His Cys Ser Gly
                -15                 -10                 -5
tct ttc tcc caa ctt gtg ctc act cag tca tct tca gcc tct ttc tcc     96
Ser Phe Ser Gln Leu Val Leu Thr Gln Ser Ser Ser Ala Ser Phe Ser
        -1  1               5                   10
ctg gga gcc tca gca aaa ctc acg tgc acc ttg agt agt cag cac agt     144
Leu Gly Ala Ser Ala Lys Leu Thr Cys Thr Leu Ser Ser Gln His Ser
    15                  20                  25
acg tac acc att gaa tgg tat cag caa cag cca ctc aag cct cct aag     192

Thr Tyr Thr Ile Glu Trp Tyr Gln Gln Gln Pro Leu Lys Pro Pro Lys
30                  35                  40                  45
tat gtg atg gat ctt aag caa gat gga agc cac agc aca ggt gat ggg    240
Tyr Val Met Asp Leu Lys Gln Asp Gly Ser His Ser Thr Gly Asp Gly
                50                  55                  60
att cct gat cgc ttc tct gga tcc agc tct ggt gct gat cgc tac ctt    288
Ile Pro Asp Arg Phe Ser Gly Ser Ser Ser Gly Ala Asp Arg Tyr Leu
            65                  70                  75
agc att tcc aac atc cag cca gaa gat gaa gca atg tac atc tgt ggt    336
Ser Ile Ser Asn Ile Gln Pro Glu Asp Glu Ala Met Tyr Ile Cys Gly
        80                  85                  90
gtg ggt gat aca att aag gaa caa ttt gtg tat gtt ttc ggc ggt ggg    384
Val Gly Asp Thr Ile Lys Glu Gln Phe Val Tyr Val Phe Gly Gly Gly
    95                  100                 105
acc aag gtc act gtc cta ggt cag ccc                                411
Thr Lys Val Thr Val Leu Gly Gln Pro
110                 115

<210> 66
<211> 411
<212> DNA
<213> Homo sapiens

<220>
<221> CDS
<222> (1)..(411)

<220>
<221> mat_peptide

<222> (58)..(411)

<400> 66
atg gcc tgg act cct ctc ttc ttc ttc ttt gtt ctt cat tgc tca ggt    48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc    96
Ser Phe Ser Gln Leu Val Leu Thr Gln Ser Pro Ser Ala Ser Ala Ser
            -1  1               5                   10
ctg gga gcc tcg gtc aag ctc acc tgc acc ttg agt agt cag cac agt    144
Leu Gly Ala Ser Val Lys Leu Thr Cys Thr Leu Ser Ser Gln His Ser
    15                  20                  25
acg tac acc att gaa tgg cat cag cag cag cca gag aag ggc cct cgg    192
Thr Tyr Thr Ile Glu Trp His Gln Gln Gln Pro Glu Lys Gly Pro Arg
30                  35                  40                  45
tac ttg atg aaa ctt aag caa gat gga agc cac agc aca ggt gat ggg    240
Tyr Leu Met Lys Leu Lys Gln Asp Gly Ser His Ser Thr Gly Asp Gly
                50                  55                  60
att cct gat cgc ttc tca ggc tcc agc tct ggg gct gag cgc tac ctc    288
Ile Pro Asp Arg Phe Ser Gly Ser Ser Ser Gly Ala Glu Arg Tyr Leu
            65                  70                  75
acc atc tcc agc ctc cag tct gag gat gag gct gac tat tac tgt ggt    336
Thr Ile Ser Ser Leu Gln Ser Glu Asp Glu Ala Asp Tyr Tyr Cys Gly
        80                  85                  90
gtg ggt gat aca att aag gaa caa ttt gtg tac gtg ttc ggc gga ggg    384
Val Gly Asp Thr Ile Lys Glu Gln Phe Val Tyr Val Phe Gly Gly Gly
    95                  100                 105
acc aaa ctg acc gtc cta ggt cag ccc                                411
Thr Lys Leu Thr Val Leu Gly Gln Pro

110                 115

<210> 67
<211> 411
<212> DNA
<213> Homo sapiens

<220>
<221> CDS
<222> (1)..(411)

<220>
<221> mat_peptide
<222> (58)..(411)

<400> 67
atg gcc tgg act cct ctc ttc ttc ttc ttt gtt ctt cat tgc tca ggt     48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc     96
Ser Phe Ser Gln Leu Val Leu Thr Gln Ser Pro Ser Ala Ser Ala Ser
        -1  1               5                   10
ctg gga gcc tcg gtc aag ctc acc tgc acc ttg agt agt cag cac agt     144
Leu Gly Ala Ser Val Lys Leu Thr Cys Thr Leu Ser Ser Gln His Ser
    15                  20                  25
acg tac acc att gaa tgg tat cag cag cag cca gag aag ggc cct aag     192
Thr Tyr Thr Ile Glu Trp Tyr Gln Gln Gln Pro Glu Lys Gly Pro Lys
30                  35                  40                  45
tac ctg atg gat ctt aag caa gat gga agc cac agc aca ggt gat ggg     240

Tyr Leu Met Asp Leu Lys Gln Asp Gly Ser His Ser Thr Gly Asp Gly
50 55 60
att cct gat cgc ttc tca ggc tcc agc tct ggg gct gag cgc tac ctc 288
Ile Pro Asp Arg Phe Ser Gly Ser Ser Ser Gly Ala Glu Arg Tyr Leu
65 70 75
acc atc tcc agc ctc cag tct gag gat gag gct gac tat tac tgt ggt 336
Thr Ile Ser Ser Leu Gln Ser Glu Asp Glu Ala Asp Tyr Tyr Cys Gly
80 85 90
gtg ggt gat aca att aag gaa caa ttt gtg tac gtg ttc ggc gga ggg 384
Val Gly Asp Thr Ile Lys Glu Gln Phe Val Tyr Val Phe Gly Gly Gly
95 100 105
acc aaa ctg acc gtc cta ggc cag ccc 411
Thr Lys Leu Thr Val Leu Gly Gln Pro
110 115

<210> 68
<211> 411
<212> DNA
<213> Homo sapiens

<220>
<221> CDS
<222> (1)..(411)

<220>
<221> mat_peptide
<222> (58)..(411)

<400> 68

atg gcc tgg act cct ctc ttc ttc ttc ttt gtt ctt cat tgc tca ggt 48
Met Ala Trp Thr Pro Leu Phe Phe Phe Phe Val Leu His Cys Ser Gly
-15 -10 -5
tct ttc tcc cag ctt gtg ctg act caa tcg ccc tct gcc tct gcc tcc 96
Ser Phe Ser Gln Leu Val Leu Thr Gln Ser Pro Ser Ala Ser Ala Ser
-1 1 5 10
ctg gga gcc tcg gtc aag ctc acc tgc acc ttg agt agt cag cac agt 144
Leu Gly Ala Ser Val Lys Leu Thr Cys Thr Leu Ser Ser Gln His Ser
15 20 25
acg tac acc att gaa tgg tat cag cag cag cca gag aag ggc cct aag 192
Thr Tyr Thr Ile Glu Trp Tyr Gln Gln Gln Pro Glu Lys Gly Pro Lys
30 35 40 45
tac gtg atg gat ctt aag caa gat gga agc cac agc aca ggt gat ggg 240
Tyr Val Met Asp Leu Lys Gln Asp Gly Ser His Ser Thr Gly Asp Gly
50 55 60
att cct gat cgc ttc tca ggc tcc agc tct ggg gct gag cgc tac ctc 288
Ile Pro Asp Arg Phe Ser Gly Ser Ser Ser Gly Ala Glu Arg Tyr Leu
65 70 75
acc atc tcc agc ctc cag tct gag gat gag gct gac tat tac tgt ggt 336
Thr Ile Ser Ser Leu Gln Ser Glu Asp Glu Ala Asp Tyr Tyr Cys Gly
80 85 90
gtg ggt gat aca att aag gaa caa ttt gtg tac gtg ttc ggc gga ggg 384
Val Gly Asp Thr Ile Lys Glu Gln Phe Val Tyr Val Phe Gly Gly Gly
95 100 105
acc aaa ctg acc gtc cta ggc cag ccc 411
Thr Lys Leu Thr Val Leu Gly Gln Pro
110 115

<210> 69

<211> 411
<212> DNA
<213> Homo sapiens

<220>
<221> CDS
<222> (1).. (411)

<220>
<221> mat_peptide
<222> (58).. (411)

<400> 69
atg gcc tgg act cct ctc ttc ttc ttc ttt gtt ctt cat tgc tca ggt    48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc    96
Ser Phe Ser Gln Leu Val Leu Thr Gln Ser Pro Ser Ala Ser Ala Ser
        -1  1               5                   10
ctg gga gcc tcg gtc aag ctc acc tgc acc ttg agt agt cag cac agt    144
Leu Gly Ala Ser Val Lys Leu Thr Cys Thr Leu Ser Ser Gln His Ser
    15                  20                  25
acg tac acc att gaa tgg tat cag cag cag cca gag aag ggc cct agg    192
Thr Tyr Thr Ile Glu Trp Tyr Gln Gln Gln Pro Glu Lys Gly Pro Arg
30                  35                  40                  45
tac ctg atg gat ctt aag caa gat gga agc cac agc aca ggt gat ggg    240
Tyr Leu Met Asp Leu Lys Gln Asp Gly Ser His Ser Thr Gly Asp Gly
                50                  55                  60
att cct gat cgc ttc tca ggc tcc agc tct ggg gct gag cgc tac ctc    288

Ile Pro Asp Arg Phe Ser Gly Ser Ser Ser Gly Ala Glu Arg Tyr Leu
65 70 75
acc atc tcc agc ctc cag tct gag gat gag gct gac tat tac tgt ggt 336
Thr Ile Ser Ser Leu Gln Ser Glu Asp Glu Ala Asp Tyr Tyr Cys Gly
80 85 90
gtg ggt gat aca att aag gaa caa ttt gtg tac gtg ttc ggc gga ggg 384
Val Gly Asp Thr Ile Lys Glu Gln Phe Val Tyr Val Phe Gly Gly Gly
95 100 105
acc aaa ctg acc gtc cta ggc cag ccc 411
Thr Lys Leu Thr Val Leu Gly Gln Pro
110 115

<210> 70
<211> 411
<212> DNA
<213> Homo sapiens

<220>
<221> CDS
<222> (1)..(411)

<220>
<221> mat_peptide
<222> (58)..(411)

<400> 70
atg gcc tgg act cct ctc ttc ttc ttc ttt gtt ctt cat tgc tca ggt 48
Met Ala Trp Thr Pro Leu Phe Phe Phe Phe Val Leu His Cys Ser Gly
-15 -10 -5

tct ttc tcc cag ctt gtg ctg act caa tcg ccc tct gcc tct gcc tcc 96
Ser Phe Ser Gln Leu Val Leu Thr Gln Ser Pro Ser Ala Ser Ala Ser
-1 1 5 10
ctg gga gcc tcg gtc aag ctc acc tgc acc ttg agt agt cag cac agt 144
Leu Gly Ala Ser Val Lys Leu Thr Cys Thr Leu Ser Ser Gln His Ser
15 20 25
acg tac acc att gaa tgg tat cag cag cag cca gag aag ggc cct agg 192
Thr Tyr Thr Ile Glu Trp Tyr Gln Gln Gln Pro Glu Lys Gly Pro Arg
30 35 40 45
tac gtg atg gat ctt aag caa gat gga agc cac agc aca ggt gat ggg 240
Tyr Val Met Asp Leu Lys Gln Asp Gly Ser His Ser Thr Gly Asp Gly
50 55 60
att cct gat cgc ttc tca ggc tcc agc tct ggg gct gag cgc tac ctc 288
Ile Pro Asp Arg Phe Ser Gly Ser Ser Ser Gly Ala Glu Arg Tyr Leu
65 70 75
acc atc tcc agc ctc cag tct gag gat gag gct gac tat tac tgt ggt 336
Thr Ile Ser Ser Leu Gln Ser Glu Asp Glu Ala Asp Tyr Tyr Cys Gly
80 85 90
gtg ggt gat aca att aag gaa caa ttt gtg tac gtg ttc ggc gga ggg 384
Val Gly Asp Thr Ile Lys Glu Gln Phe Val Tyr Val Phe Gly Gly Gly
95 100 105
acc aaa ctg acc gtc cta ggc cag ccc 411
Thr Lys Leu Thr Val Leu Gly Gln Pro
110 115

<210> 71
<211> 411
<212> DNA
<213> Homo sapiens

<220>
<221> CDS
<222> (1)..(411)

<220>
<221> mat_peptide
<222> (58)..(411)

<400> 71
atg gcc tgg act cct ctc ttc ttc ttc ttt gtt ctt cat tgc tca ggt     48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc     96
Ser Phe Ser Gln Leu Val Leu Thr Gln Ser Pro Ser Ala Ser Ala Ser
        -1  1               5                   10
ctg gga gcc tcg gtc aag ctc acc tgc acc ttg agt agt cag cac agt     144
Leu Gly Ala Ser Val Lys Leu Thr Cys Thr Leu Ser Ser Gln His Ser
    15                  20                  25
acg tac acc att gaa tgg tat cag cag cag cca gag aag ggc cct aag     192
Thr Tyr Thr Ile Glu Trp Tyr Gln Gln Gln Pro Glu Lys Gly Pro Lys
30                  35                  40                  45
tac ctg atg gat ctt aag caa gat gga agc cac agc aca ggt gat ggg     240
Tyr Leu Met Asp Leu Lys Gln Asp Gly Ser His Ser Thr Gly Asp Gly
                50                  55                  60
att cct gat cgc ttc tca ggc tcc agc tct ggg gct gag cgc tac ctc     288
Ile Pro Asp Arg Phe Ser Gly Ser Ser Ser Gly Ala Glu Arg Tyr Leu
            65                  70                  75
acc atc tcc agc ctc cag tct gag gat gag gct gac tat atc tgt ggt     336

Thr Ile Ser Ser Leu Gln Ser Glu Asp Glu Ala Asp Tyr Ile Cys Gly
        80                  85                  90
gtg ggt gat aca att aag gaa caa ttt gtg tac gtg ttc ggc gga ggg    384
Val Gly Asp Thr Ile Lys Glu Gln Phe Val Tyr Val Phe Gly Gly Gly
    95                  100                 105
acc aaa ctg acc gtc cta ggc cag ccc                                411
Thr Lys Leu Thr Val Leu Gly Gln Pro
110                 115

<210> 72
<211> 411
<212> DNA
<213> Homo sapiens

<220>
<221> CDS
<222> (1)..(411)

<220>
<221> mat_peptide
<222> (58)..(411)

<400> 72
atg gcc tgg act cct ctc ttc ttc ttc ttt gtt ctt cat tgc tca ggt    48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc    96
Ser Phe Ser Gln Leu Val Leu Thr Gln Ser Pro Ser Ala Ser Ala Ser
        -1  1               5                   10

ctg gga gcc tcg gtc aag ctc acc tgc acc ttg agt agt cag cac agt     144
Leu Gly Ala Ser Val Lys Leu Thr Cys Thr Leu Ser Ser Gln His Ser
    15                  20                  25
acg tac acc att gaa tgg tat cag cag cag cca gag aag ggc cct agg     192
Thr Tyr Thr Ile Glu Trp Tyr Gln Gln Gln Pro Glu Lys Gly Pro Arg
30                  35                  40                  45
tac ctg atg gat ctt aag caa gat gga agc cac agc aca ggt gat ggg     240
Tyr Leu Met Asp Leu Lys Gln Asp Gly Ser His Ser Thr Gly Asp Gly
                50                  55                  60
att cct gat cgc ttc tca ggc tcc agc tct ggg gct gag cgc tac ctc     288
Ile Pro Asp Arg Phe Ser Gly Ser Ser Ser Gly Ala Glu Arg Tyr Leu
            65                  70                  75
acc atc tcc agc ctc cag tct gag gat gag gct gac tat atc tgt ggt     336
Thr Ile Ser Ser Leu Gln Ser Glu Asp Glu Ala Asp Tyr Ile Cys Gly
        80                  85                  90
gtg ggt gat aca att aag gaa caa ttt gtg tac gtg ttc ggc gga ggg     384
Val Gly Asp Thr Ile Lys Glu Gln Phe Val Tyr Val Phe Gly Gly Gly
    95                  100                 105
acc aaa ctg acc gtc cta ggc cag ccc                                 411
Thr Lys Leu Thr Val Leu Gly Gln Pro
110                 115

<210> 73
<211> 411
<212> DNA
<213> Homo sapiens

<220>
<221> CDS

<222> (1)..(411)

<220>
<221> mat_peptide
<222> (58)..(411)

<400> 73
atg gcc tgg act cct ctc ttc ttc ttc ttt gtt ctt cat tgc tca ggt    48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc    96
Ser Phe Ser Gln Leu Val Leu Thr Gln Ser Pro Ser Ala Ser Ala Ser
        -1  1               5                   10
ctg gga gcc tcg gtc aag ctc acc tgc acc ttg agt agt cag cac agt    144
Leu Gly Ala Ser Val Lys Leu Thr Cys Thr Leu Ser Ser Gln His Ser
    15                  20                  25
acg tac acc att gaa tgg tat cag cag cag cca gag aag ggc cct aag    192
Thr Tyr Thr Ile Glu Trp Tyr Gln Gln Gln Pro Glu Lys Gly Pro Lys
30                  35                  40                  45
tac gtg atg gat ctt aag caa gat gga agc cac agc aca ggt gat ggg    240
Tyr Val Met Asp Leu Lys Gln Asp Gly Ser His Ser Thr Gly Asp Gly
                50                  55                  60
att cct gat cgc ttc tca ggc tcc agc tct ggg gct gag cgc tac ctc    288
Ile Pro Asp Arg Phe Ser Gly Ser Ser Ser Gly Ala Glu Arg Tyr Leu
            65                  70                  75
acc atc tcc agc ctc cag tct gag gat gag gct gac tat atc tgt ggt    336
Thr Ile Ser Ser Leu Gln Ser Glu Asp Glu Ala Asp Tyr Ile Cys Gly
        80                  85                  90
gtg ggt gat aca att aag gaa caa ttt gtg tac gtg ttc ggc gga ggg    384

Val Gly Asp Thr Ile Lys Glu Gln Phe Val Tyr Val Phe Gly Gly Gly
    95                  100                 105
acc aaa ctg acc gtc cta ggc cag ccc                                 411
Thr Lys Leu Thr Val Leu Gly Gln Pro
110                 115

<210> 74
<211> 411
<212> DNA
<213> Homo sapiens

<220>
<221> CDS
<222> (1)..(411)

<220>
<221> mat_peptide
<222> (58)..(411)

<400> 74
atg gcc tgg act cct ctc ttc ttc ttc ttt gtt ctt cat tgc tca ggt    48
Met Ala Trp Thr Pro Leu Phe Phe Phe Phe Val Leu His Cys Ser Gly
                -15                 -10                 -5
tct ttc tcc cag ctt gtg ctg act caa tcg ccc tct gcc tct gcc tcc    96
Ser Phe Ser Gln Leu Val Leu Thr Gln Ser Pro Ser Ala Ser Ala Ser
        -1  1               5                   10
ctg gga gcc tcg gtc aag ctc acc tgc acc ttg agt agt cag cac agt    144
Leu Gly Ala Ser Val Lys Leu Thr Cys Thr Leu Ser Ser Gln His Ser
    15                  20                  25

acg tac acc att gaa tgg tat cag cag cag cca gag aag ggc cct agg    192
Thr Tyr Thr Ile Glu Trp Tyr Gln Gln Gln Pro Glu Lys Gly Pro Arg
 30                  35                  40                  45
tac gtg atg gat ctt aag caa gat gga agc cac agc aca ggt gat ggg    240
Tyr Val Met Asp Leu Lys Gln Asp Gly Ser His Ser Thr Gly Asp Gly
                50                  55                  60
att cct gat cgc ttc tca ggc tcc agc tct ggg gct gag cgc tac ctc    288
Ile Pro Asp Arg Phe Ser Gly Ser Ser Ser Gly Ala Glu Arg Tyr Leu
            65                  70                  75
acc atc tcc agc ctc cag tct gag gat gag gct gac tat atc tgt ggt    336
Thr Ile Ser Ser Leu Gln Ser Glu Asp Glu Ala Asp Tyr Ile Cys Gly
        80                  85                  90
gtg ggt gat aca att aag gaa caa ttt gtg tac gtg ttc ggc gga ggg    384
Val Gly Asp Thr Ile Lys Glu Gln Phe Val Tyr Val Phe Gly Gly Gly
    95                  100                 105
acc aaa ctg acc gtc cta ggc cag ccc                                411
Thr Lys Leu Thr Val Leu Gly Gln Pro
110                 115

<210> 75
<211> 34
<212> PRT
<213> Homo sapiens

<400> 75
Ala Val Ser Glu His Gln Leu Leu His Asp Lys Gly Lys Ser Ile Gln
 1               5                   10                  15
Asp Leu Arg Arg Arg Phe Phe Leu His His Leu Ile Ala Glu Ile His
            20                  25                  30
Thr Ala

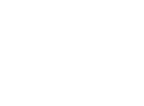

## Claims

1. A therapeutic agent for a disease caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor.

2. The therapeutic agent according to claim 1, wherein the disease caused by PTH or PTHrP is primarily a disease other than hypercalcemia.

3. A QOL improving agent for alleviating a symptom of a disease caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor.

4. A therapeutic agent for a syndrome associated with malignancy caused by PTHrP, which comprises, as an active ingredient, an agonist or antagonist binding to a PTH receptor or PTHrP receptor, or a substance binding to a ligand of the receptor to promote or inhibit binding between the ligand and the receptor.

5. The therapeutic agent according to claim 4, wherein the syndrome associated with malignancy is selected from the group consisting of digestive system disorders, proteometabolism abnormality, saccharometabolism abnormality, lipid metabolism abnormality, anorexia, hematological abnormality, electrolyte abnormality, immunodeficiency and pain.

6. The therapeutic agent according to claim 1 or 2, wherein the disease is a secondary hyperparathyroidism or primary hyperparathyroidism caused by PTH.

7. An alleviation agent for a central nervous system disease caused by PTH or PTHrP, which comprises, as an active ingredient, an agonist or antagonist binding to a PTH receptor or PTHrP receptor, or a substance binding to a ligand of the receptor to promote or inhibit binding between the ligand and the receptor.

8. The alleviation agent according to claim 7, wherein the central nervous system disease is selected from the group consisting of dyssomnia, neuropathy, nervous symptom, brain metabolism abnormality, cerebral circulation abnormality, autonomic imbalance, and endocrine system abnormality with which central nervous system is associated.

9. An alleviation agent for a disease caused by PTH or PTHrP-cytokine cascade, which comprises, as an active ingredient, an agonist or antagonist binding to a PTH receptor or PTHrP receptor, or a substance binding to a ligand of the receptor to promote or inhibit binding between the ligand and the receptor.

10. The alleviation agent according to claim 9, wherein the cytokine is selected from the group consisting of IL-1, IL-2, IL-3, IL-4, IL-5, IL-6, IL-7, IL-8, IL-9, IL-10, IL-11, IL-12, IL-13, IL-15, G-CSF, GM-CSF, M-CSF, EPO, LIF, TPO, EGF, TGF- α, TGF- β, FGF, IGF, HGF, VEGF, NGF, activin, inhibin, a BMP family, TNF and IFN.

11. The alleviation agent according to claim 9 or 10, wherein the disease caused by PTH or PTHrP-cytokine cascade is selected from the group consisting of septicemia, cachexia, inflammation, hemopathy, calcium metabolism abnormality, and autoimmune disease.

12. A central nervous system regulator, which comprises, as an active ingredient, an agonist or antagonist binding to a PTH receptor or PTHrP receptor, or a substance binding to a ligand of the receptor to promote or inhibit binding between the ligand and the receptor.

13. A cytokine network regulator, which comprises, as an active ingredient, an agonist or antagonist binding to a PTH receptor or PTHrP receptor, or a substance binding to a ligand of the receptor to promote or inhibit binding between the ligand and the receptor.

14. The agent according to any one of claims 1 to 13, wherein the PTH receptor or PTHrP receptor is a PTH/PTHrP type I receptor.

15. The agent according to any one of claims 1 to 14, wherein the substance binding to a ligand of PTH receptor or PTHrP receptor to inhibit binding between the ligand and the receptor is selected from the group consisting of an anti-PTHrP antibody and anti-PTH antibody.

16. The agent according to claim 15, wherein the substance binding to a ligand of PTH receptor or PTHrP receptor to inhibit binding between the ligand and the receptor is an anti-PTHrP antibody.

17. The agent according to claim 16, wherein the anti-PTHrP antibody is a humanized anti-PTHrP antibody.
